# FASCICULE DE BREVET EUROPEEN

(11) **EP 1 156 049 B1**
(45) Date de publication et mention de la délivrance du brevet: **01.06.2005**
(21) Numéro de dépôt: 01119949.4
(22) Date de dépôt: 30.01.1996
(51) Int. Cl.: C07D 413/06, C07D 401/06, C07D 413/12, C07D 265/10, C07D 265/30, C07D 265/32, C07D 453/02, C07D 309/12, C07D 263/38, C07D 405/12, C07C 219/22, C07C 255/36, C07C 233/18, C07C 255/42, C07C 271/22, C07C 215/30, A61K 31/445

(54) **Composés hétérocycliques substitués, procédé pour leur préparation et compositions pharmaceutiques les contenant**
Substituierte heterocyclische Verbindungen, Verfahren zu ihrer Herstellung und diese Verbindungen enthaltende pharmazeutische Zusammensetzungen
Substituted heterocyclic compounds, their process of preparation and pharmaceutical compositions containing them

(30) Priorité: 30.01.1995 FR 9501016; 04.07.1995 FR 9508046; 03.11.1995 FR 9513005
(43) Date de publication de la demande: 21.11.2001
(62) Demande divisionnaire de: 96902305.0
(73) Titulaire: Sanofi-Aventis, 75013 Paris (FR)
(72) Inventeur: Emonds-Alt, Xavier, 34980 Combaillaux (FR); Grossriether, Isabelle, 75014 Paris (FR); Gueule, Patrick, 34820 Teyran (FR); Proietto, Vincenzo, 34680 Saint Georges d'Orques (FR); Van Broeck, Didier, 34570 Murviel les Montpellier (FR)
(74) Mandataire: Gillard, Marie-Louise

(56) Documents cités:
- EP-A- 0 428 434
- EP-A- 0 436 334
- EP-A- 0 474 561
- EP-A- 0 512 901
- EP-A- 0 515 240
- EP-A- 0 559 538
- EP-A- 0 591 040

## Description

La présente invention a pour objet de nouveaux composés hétérocycliques substitués, un procédé pour leur préparation et les compositions pharmaceutiques en contenant en tant que principe actif.

Plus particulièrement, la présente invention concerne une nouvelle classe de composés hétérocycliques substitués à usage thérapeutique, dans les phénomènes pathologiques qui impliquent le système des tachykinines comme par exemple de manière non limitative et exclusive : la douleur (D. Regoli et al., Life Sciences, 1987, 40, 109-117), l'allergie et l'inflammation (J.E. Morlay et al., Life Sciences, 1987, 41, 527-544), l'insuffisance circulatoire (J. Losay et al., 1977, Substance P, Von Euler, I.S. and Pernow ed., 287-293, Raven Press, New York), les troubles gastro- intestinaux (D. Regoli et al., Trends Pharmacol. Sci., 1985, 6, 481-484), les troubles respiratoires (J. Mizrahi et al., Pharmacology, 1982, 25, 39-50) les troubles neurologiques, les troubles neuropsychiatriques (C.A. Maggi et al., J. Autonomie. Pharmacol., 1993, 13, 23-93).

Dans les années récentes de nombreux travaux de recherche ont été effectués sur les tachykinines et leurs récepteurs. Les tachykinines sont distribuées à la fois dans le système nerveux central et dans le système nerveux périphérique. Les récepteurs aux tachykinines ont été reconnus et sont classés en trois types : NK₁, NK₂, NK₃. La substance P (SP) est le ligand endogène des récepteurs NK₁, la neurokinine A (NK_{A}) celui des récepteurs NK₂ et la neurokinine B (NK_{B}), celui des récepteurs NK₃.

Les récepteurs NK₁, NK₂, NK₃ ont été mis en évidence chez différentes espèces. Une revue de C.A. Maggi et al. fait le point sur les récepteurs aux tachykinines et leurs antagonistes et expose les études pharmacologiques et les applications en thérapeutique humaine (J. Autonomic Pharmacol., 1993, 13, 23-93).

Parmi les antagonistes spécifiques du récepteur NK₁ on peut citer les composés non peptidiques suivants : CP-96345 (J. Med. Chem., 1992, 35, 2591-2600), RP-68651 (Proc. Natl. Acad. Sci. USA, 1991, 88, 10208-10212), SR 140333 (Curr. J. Pharmacol., 1993, 250, 403-413).

Pour le récepteur NK₂, un antagoniste sélectif non peptidique, le SR 48968 a été décrit en détail (Life Sci., 1992, 50, PL101-PL106).

En ce qui concerne le récepteur NK₃, certains composés non peptidiques, ont été décrits, comme ayant une affinité pour le récepteur NK₃ du cerveau de rat et de cobaye (FASEB J., 1993, 7 (4), A710-4104) ; un antagoniste peptidique [Trp⁷, βAla⁸]NK_{A}, faiblement spécifique du récepteur NK₃ du rat a également été décrit (J. Autonomic. Pharmacol., 1993, 13, 23-93).

La demande de brevet EP-A-336230 décrit des dérivés peptidiques antagonistes de la substance P et de la neurokinine A utiles pour le traitement et la prévention de l'asthme.

Les demandes de brevet internationales WO 90/05525, WO 90/05729, WO 91/09844, WO 91/18899 et européennes EP-A-0436334, EP-A-0429466 et EP-A-0430771 décrivent des antagonistes de la Substance P.

Les demandes de brevet européennes EP-A-0474561, EP-A-512901, EP-A-515240, EP-A-559538 et EP-A-591040 concernent également des antagonistes des récepteurs des neurokinines.

On a maintenant trouvé des nouveaux composés hétérocycliques substitués qui sont des antagonistes des récepteurs des neurokinines.

Ainsi selon un de ses aspects, la présente invention a pour objet des composés de formule : 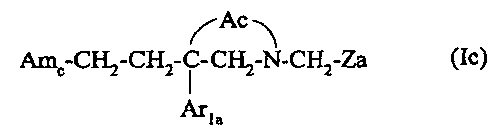 dans laquelle :
- Ac représente un radical bivalent choisi parmi : -O-CH₂-CO- ; -CH₂-O-CO- ou -O-CO-;
- Am_{c} représente un groupe Am₁ₐ de formule: 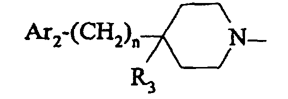
- n est 0 ou 1 ;
- Ar₂ représente un pyridyle ; un phényle non substitué ou substitué une ou plusieurs fois par un substituant choisi parmi : un atome d'halogène, un hydroxy, un (C₁-C₄)alcoxy, un (C₁-C₄)alkyle, un trifluorométhyle, un nitro, un méthylènedioxy, lesdits substituants étant identiques ou différents ; un thiényle ; un pyrimidyle ; un imidazolyle non substitué ou substitué par un (C₁-C₄)alkyle ;
- R₃ représente un groupe choisi parmi :
   (1) hydrogène ;
   (2) (C₁-C₇)alkyle ;
   (3) formyle ;
   (4) (C₁-C₇)alkylcarbonyle ;
   (5) cyano ;
   (6) -(CH₂)_{q}-OH ;
   (7) -(CH₂)_{q}-O-(C₁-C₇)alkyle ;
   (8) -(CH₂)_{q}-OCHO ;
   (9) -(CH₂)_{q}-OCOR₁₇ ;
   (10) -(CH₂)_{q}-OCONH-(C₁-C₇)alkyle ;
   (11) -NR₄R₅ ;
   (12) -(CH₂)_{q}-NR₆C(=W₁)R₇ ;
   (13) -(CH₂)_{q}-NR₆COOR₈ ;
   (14) -(CH₂)_{q}-NR₆SO₂R₉ ;
   (15) -(CH₂)_{q}-NR₆C(=W₁)NR₁₀R₁₁ ;
   (16) -CH₂-NR₁₂R₁₃ ;
   (17) -CH₂-CH₂-NR₁₂R₁₃ ;
   (18) -COOH ;
   (19) (C₁-C₇)alcoxycarbonyle ;
   (20) -C(=W₁)NR₁₀R₁₁ ;
   (21) -CH₂-COOH ;
   (22) (C₁-C₇)alcoxycarbonylméthyle ;
   (23) -CH₂-C(=W₁)NR₁₀R₁₁ ;
   (24) -O-CH₂CH₂-OR₁₈ ;
   (25) -NR₆COCOR₁₉ ;
   (26) -CO-NR₂₀-NR₂₁R₂₂ ;
   (27) 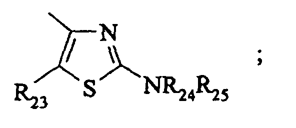
   (28) 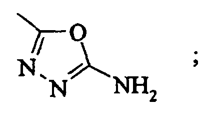
- ou R₃ constitue une double liaison entre l'atome de carbone auquel il est lié et l'atome de carbone voisin du cycle pipéridine ;
- q est 0, 1 ou 2 ;
- W ₁ représente un atome d'oxygène ou un atome de soufre ;
- R₄ et R₅ représentent chacun indépendamment un hydrogène ou un (C₁-C₇)alkyle ; R₅ peut de plus représenter un (C₃-C₇)cycloalkylméthyle, un benzyle ou un phényle ; ou R₄ et R₅ ensemble avec l'atome d'azote auquel ils sont liés constituent un hétérocycle choisi parmi l'azétidine, la pyrrolidine, la pipéridine, la morpholine, la thiomorpholine, la perhydroazépine ou la pipérazine non substituée ou substituée en position 4 par un (C₁-C₄)alkyle ;
- R₆ représente un hydrogène ou un (C₁-C₇)alkyle ;
- R₇ représente un hydrogène ; un (C₁-C₇)alkyle ; un vinyle ; un phényle ; un benzyle ; un pyridyle ; un (C₃-C₇)cycloalkyle non substitué ou substitué par un ou plusieurs méthyles ; un furyle ; un thiényle ; un pyrrolyle ; un imidazolyle ;
- ou R₆ et R₇ ensemble représentent un groupe -(CH₂)ₚ- ;
- p est 3 ou 4 ;
- R₈ représente un (C₁-C₇)alkyle ou un phényle ;
- R₉ représente un (C₁-C₇)alkyle ; un amino libre ou substitué par un ou deux (C₁-C₇)alkyles ; un phényle non substitué ou substitué une ou plusieurs fois par un substituant choisi parmi : un atome d'halogène, un (C₁-C₇)alkyle, un trifluorométhyle, un hydroxy, un (C₁-C₇)alcoxy, un carboxy, un (C₁-C₇)alcoxycarbonyle, un (C₁-C₇)alkylcarbonyloxy, un cyano, un nitro, un amino libre ou substitué par un ou deux (C₁-C₇)alkyles, lesdits substituants étant identiques ou différents ;
- R₁₀ et R₁₁ représentent chacun indépendamment un hydrogène ou un (C₁-C₇)alkyle ; R₁₁ peut de plus représenter un (C₃-C₇)cycloalkyle, un (C₃-C₇)cycloalkylméthyle, un hydroxy, un (C₁-C₄)alcoxy, un benzyle ou un phényle ; ou R₁₀ et R₁₁ ensemble avec l'atome d'azote auquel ils sont liés constituent un hétérocycle choisi parmi l'azétidine, la pyrrolidine, la pipéridine, la morpholine, la thiomorpholine ou la perhydroazépine ;
- R₁₂ et R₁₃ représentent chacun indépendamment un hydrogène ou un (C₁-C₇)alkyle ; R₁₃ peut de plus représenter un (C₃-C₇)cycloalkylméthyle ou un benzyle ;
- R₁₇ représente un (C₁-C₇)alkyle ; un (C₃-C₇)cycloalkyle non substitué ou substitué par un ou plusieurs méthyles ; un phényle ; un pyridyle ;
- R₁₈ représente un hydrogène ; un (C₁-C₇)alkyle ; un formyle ; un (C₁-C₇)alkylcarbonyle ;
- R₁₉ représente un (C₁-C₄)alcoxy ;
- R₂₀ représente un hydrogène ou un (C₁-C₇)alkyle ;
- R₂₁ et R₂₂ représentent chacun indépendamment un hydrogène ou un (C₁-C₇)alkyle ;
- ou bien R₂₁ et R₂₂ ensemble avec l'atome d'azote auquel ils sont liés constituent un hétérocycle choisi parmi la pyrrolidine, la pipéridine ou la morpholine ;
- R₂₃ représente un hydrogène ou un (C₁-C₇)alkyle ;
- R₂₄ et R₂₅ représentent chacun indépendamment un hydrogène ou un (C₁-C₇)alkyle ; R₂₅ peut de plus représenter un formyle ou un (C₁-C₇)alkylcarbonyle ;
- Ar₁ₐ représente un phényle non substitué ou substitué une ou plusieurs fois par un substituant choisi parmi : un atome d'halogène, un hydroxy, un (C₁-C₄)alcoxy, un (C₁-C₄)alkyle, un trifluorométhyle, lesdits substituants étant identiques ou différents ;
- Za représente un phényle non substitué ou substitué une ou plusieurs fois par un substituant choisi parmi : un atome d'halogène, un trifluorométhyle, un (C₁-C₁₀)alkyle, un (C₁-C₁₀)alcoxy, un hydroxy, lesdits substituants étant identiques ou différents ;
et leurs sels avec des acides minéraux ou organiques.

Parmi ces composés, ceux de formule : 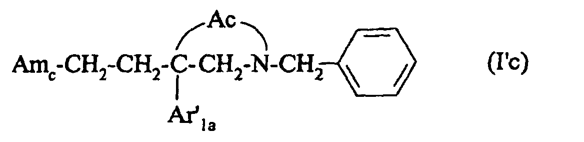 dans laquelle :
- Ac, Am_{c} sont tels que définis précédemment ;
- Ar'₁ₐ représente un 3,4-dichlorophényle ou un 3,4-difluorophényle ;
et leurs sels avec des acides minéraux ou organiques, sont particulièrement préférés.

Les composés de formule (Ic) selon l'invention comprennent aussi bien les racémiques que les isoméres optiquement purs.

Le groupe phényle de Za peut être mono substitué ou disubstitué notamment en position 2,4 mais aussi par exemple en position 2,3 ou 4,5 ou 3,4 ou 3,5 ; il peut aussi être trisubstitué, notamment en position 2,4,6 mais aussi par exemple en 2,3,4 ou 2,3,5 ou 2,4,5 ou 3,4,5 ; tétrasubstitué, par exemple en 2,3,4,5 ; ou pentasubstitué.

Outre les sels d'ammonium quaternaire, on peut former des sels des composés de formule (Ic). Ces sels comprennent aussi bien ceux avec des acides minéraux et organiques qui permettent une séparation ou une cristallisation convenable des composés de formule (Ic), tels que l'acide picrique ou l'acide oxalique ou un acide optiquement actif, par exemple un acide mandélique ou camphosulfonique, que ceux qui forment des sels pharmaceutiquement acceptables tels que le chlorhydrate, le bromhydrate, le sulfate, l'hydrogénosulfate, le dihydrogénophosphate, le méthanesulfonate, le méthylsulfate, le maléate, le fumarate, le 2-naphtalènesulfonate, le benzènesulfonate, le gluconate, le citrate, l'iséthionate, le *p*-toluènesulfonate.

Dans la présente description les groupes alkyles ou les groupes alcoxy sont droits ou ramifiés ; par atome d'halogène on entend un atome de chlore, de brome, de fluor ou d'iode.

Dans les substituants du groupe Za, par (C₁-C₁₀)alkyle on entend par exemple un méthyle, un éthyle, un n-propyle, un isopropyle, un n-butyle, un isobutyle, un sec-butyle, un tert-butyle, un pentyle ou n-pentyle, un hexyle ou n-hexyle, un heptyle ou un n-heptyle, un octyle ou n-octyle, un nonyle ou n-nonyle, un décyle ou n-décyle ; par (C₁-C₁₀)alcoxy on entend par exemple un méthoxy, un éthoxy, un n-propoxy, un isopropoxy, un n-butoxy, un isobutoxy, un *sec*-butoxy, un *tert*-butoxy, un pentyloxy, un hexyloxy, un heptyloxy, un nonyloxy ou un décyloxy.

De manière avantageuse, le radical Za représente un phényle non substitué ou substitué une ou plusieurs fois par un atome d'halogène, plus particulièrement un atome de chlore, de fluor ou d'iode, un trifluorométhyle, un (C₁-C₄)alkyle, un hydroxy, un (C₁-C₄)alcoxy.

Parmi les composés de formules (Ic) et (I'c) ci-dessus, on préfère tout particulièrement ceux dans lesquels :
- Ar₂ représente un pyridyle ; un phényle non substitué ou substitué une ou plusieurs fois par un substituant choisi parmi : un atome d'halogène, un hydroxy, un (C₁-C₄)alcoxy, un (C₁-C₄)alkyle, un trifluorométhyle, lesdits substituants étant identiques ou différents ;
- R₃ représente un hydrogène, un (C₁-C₇)alkyle ; un formyle ; un (C₁-C₇)alkylcarbonyle ; un cyano ; un groupe -(CH₂)_{q}-OH ; un groupe (C₁-C₇)alkyl-O-(CH₂)_{q}- ; un groupe HCOO-(CH₂)_{q}- ; un groupe (C₁-C₇)alkyl-COO-(CH₂)_{q}- ; un groupe (C₁-C₇)alkyl-NHCOO-(CH₂)_{q}- ; un groupe -NR₄R₅ ; un groupe -(CH₂)_{q}-NR₆COR₇ ; un groupe -(CH₂)_{q}-NR₆COOR₈ ; un groupe -(CH₂)_{q}-NR₆SO₂R₉ ; un groupe -(CH₂)_{q}-NR₆CONR₁₀R₁₁ ; un groupe -CH₂-NR₁₂R₁₃ ; un groupe -CH₂-CH₂-NR₁₂R₁₃ ; un carboxy ; un (C₁-C₇)alcoxycarbonyle ; un groupe -CONR₁₀R₁₁ ; un carboxyméthyle ; un (C₁-C₇)alcoxycarbonylméthyle ; un groupe -CH₂-CONR₁₀R₁₁ ; un 2-aminothiazol-4-yle dans lequel l'amino est libre ou substitué par un ou deux (C₁-C₇)alkyles ;
- ou R₃ constitue une double liaison entre l'atome de carbone auquel il est lié et l'atome de carbone voisin du cycle pipéridine ;
- q est 0, 1 ou 2 ;
et leurs sels avec des acides minéraux ou organiques.

Le 5-(3,4-difluorophényl)-5-[2-[4-phényl-1-azoniabicyclo[2.2.2]oct-1-yl]éthyl]-3-[3,5-bis(trifluorométhyl)benzyl]oxazolidin-2-one, avec un anion pharmaceutiquement acceptable, sous forme optiquement pure, préférentiellement sous forme de l'isomère (+), est tout particulièrement préféré.

Selon un autre de ses aspects, la présente invention concerne un procédé pour la préparation des composés de formule (Ic) et de leurs sels, caractérisé en ce que :
1) on traite un composé de formule : 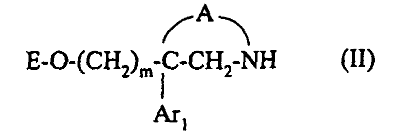 dans laquelle Ar₁ est Ar₁ₐ et A est Ac, Ar₁ₐ et Ac étant tels que définis précédemment pour un composé de formule (Ic), m est 2 et E représente l'hydrogène ou un groupe O-protecteur avec un dérivé halogéné de formule :

   Hal-CH₂-Z (IV)

   dans laquelle Zest Za, Za étant tel que défini précédemment pour (Ic), et Hal représente un halogène, préférentiellement le brome ou le chlore pour obtenir un composé de formule : 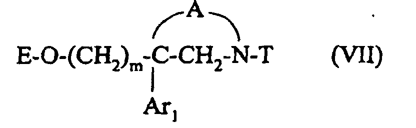 dans laquelle E, m, Ar₁ et A sont tels que définis ci-dessus et T est -CH₂-Za, Za étant tel que défini précédemment pour le composé de formule (Ia) ;
2) on élimine éventuellement le groupe O-protecteur par action d'un acide ou d'une base, pour obtenir l'alcool de formule : 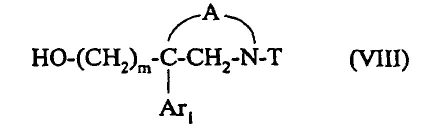
3) on traite l'alcool (VIII) avec un composé de formule :

   Y-SO₂-Cl (IX)

   dans laquelle Y représente un groupe méthyle, phényle, tolyle, trifluorométhyle, pour obtenir un composé de formule : 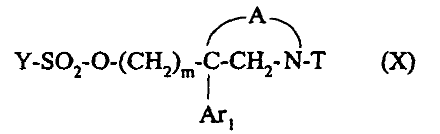
4) on fait réagir le composé (X) avec une amine secondaire cyclique de formule : 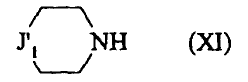 dans laquelle J'₁ représente un groupe : 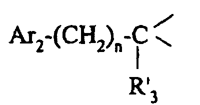 dans lequel Ar₂ et n sont tels que définis pour (Ic) et R'₃ représente soit R₃ tel que défini pour (Ic), soit un précurseur de R₃, étant entendu que lorsque R'₃ représente un hydroxyle ou un amino, ces groupes peuvent être protégés ; et
5) après déprotection éventuelle du groupe hydroxy ou du groupe amino représenté par R'₃ ou transformation éventuelle de R'₃ en R₃, on transforme éventuellement le produit ainsi obtenu en un de ses sels ;

Selon une variante du procédé,
1') on oxyde un composé de formule (VIII) tel que défini précédemment pour obtenir un composé de formule : 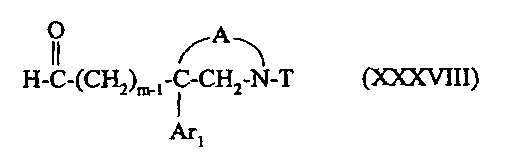 dans laquelle m, Ar₁, A et T sont tels que définis ci-dessus,
2') on fait réagir le composé de formule (XXXVIII) avec un composé de formule (XI) tel que défini précédemment en présence d'un acide, puis on réduit le sel d'iminium formé intermédiairement au moyen d'un agent réducteur ; et
3') après déprotection éventuelle des groupes hydroxyles ou des groupes aminés ou transformation éventuelle de R'₃ en R₃, on transforme éventuellement le produit ainsi obtenu en l'un de ses sels.

Les composés de formule : 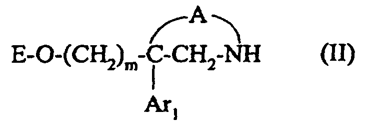 dans laquelle m, E, A et Ar₁, sont tels que définis ci-dessus,
sous forme énantiomériquement pure ou sous forme racémique sont nouveaux et font partie de l'invention.

Les composés de formule : 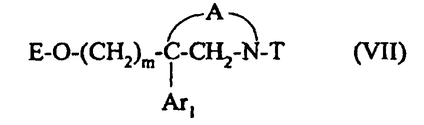 dans laquelle m, E, A et Ar₁, sont tels que définis ci-dessus,
sous forme énantiomériquement pure ou sous forme racémique sont nouveaux et font partie de l'invention.

Les composés de formule (II) et (VII) dans lesquels E représente l'hydrogène sont particulièrement préférés.

Les composés de formule : 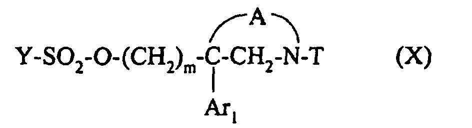 dans laquelle Y, m, E, A et Ar₁, sont tels que définis ci-dessus,
sous forme énantiomériquement pure ou sous forme racémique sont nouveaux et font partie de l'invention.

Les composés de formule : 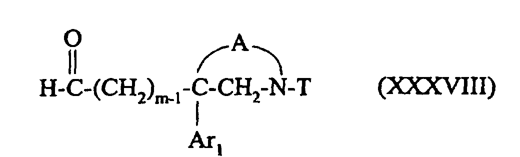 dans laquelle m, E, A et Ar₁, sont tels que définis ci-dessus,
sous forme énantiomériquement pure ou sous forme racémique sont nouveaux et font partie de l'invention.

Dans les formules (II), (VII), (X) et (XXXVIII), m et les groupes E, A, Ar₁ T et Y ont les définitions données ci-dessus.

Ainsi, selon un autre de ses aspects, la présente invention a pour objet des composés de formule : 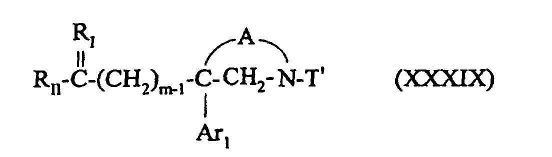 dans laquelle :
- m, Ar₁, A sont tels que définis ci-dessus ;
- R_{I} représente deux atomes d'hydrogène et R_{II} représente :
   - soit un groupe -O-E dans lequel E représente un atome d'hydrogène ou un groupe O-protecteur,
   - soit un groupe -O-SO₂-Y dans lequel Y représente un groupe méthyle, phényle, tolyle ou trifluorométhyle ;
- ou bien R_{I} représente un atome d'oxygène et R_{II} représente un atome d'hydrogène;
- T' représente T tel que défini ci-dessus, T' peut de plus représenter l'hydrogène lorsque à la fois R_{I} représente 2 atomes d'hydrogène et R_{II} représente un groupe -O-E ;
sous forme énantiomèriquement pure ou sous forme de racémique.

Ainsi, lorsque E représente un groupe O-protecteur, celui-ci est choisi parmi les groupes O-protecteurs classiques bien connus de l'homme de l'art, tels que, par exemple, le tétrahydropyran-2-yle, le benzoyle ou un (C₁-C₄)alkylcarbonyle.

Les groupes O-protecteurs éventuellement utilisés pour obtenir un composé de formule (I) dans laquelle R₃ représente un hydroxy sont les groupes O-protecteurs classiques bien connus de l'homme de l'art tels que définis ci-dessus pour E.

Les groupes N-protecteurs éventuellement utilisés pour obtenir un composé de formule (I) dans laquelle R₃ représente un amino sont les groupes N-protecteurs classiques bien connus de l'homme de l'art tels que, par exemple, le groupe trityle, méthoxytrityle, *tert*-butoxycarbonyle ou benzyloxycarbonyle.

De façon particulière, lorsqu'on utilise comme groupe O-protecteur un groupe acétyle, le composé de formule (Ic) obtenu représente le produit final dans lequel R₃ représente un acétoxy ou lorsqu'on utilise comme groupe N-protecteur un groupe tert-butoxycarbonyle, le composé de formule (Ic) obtenu représente le produit final dans lequel R₃ représente un *tert*-butoxycarbonylamino.

La réaction de l"étape 1) s'effectue dans un solvant inerte tel que le tétrahydrofurane, le N,N-diméthylformamide ou le diméthylsulfoxyde en présence d'une base telle que le *tert*-butylate de potassium, l'hydrure de sodium ou le diisopropylamidure de lithium et à une température comprise entre 0°C et 80°C.

Le composé de formule (VII) ainsi obtenu est éventuellement déprotégé à l'étape 2) selon les méthodes connues de l'homme de l'art. Par exemple, lorsque E représente un groupe tétrahydropyran-2-yle, la déprotection s'effectue par hydrolyse acide en utilisant l'acide chlorhydrique dans un solvant tel que l'éther, le méthanol ou le mélange de ces solvants, ou en utilisant le *p*-toluènesulfonate de pyridinium dans un solvant tel que le méthanol ou encore, en utilisant une résine Amberlyst® dans un solvant tel que le méthanol. La réaction s'effectue à une température comprise entre la température ambiante et la température de reflux du solvant. Lorsque E représente un groupe benzoyle ou un groupe (C₁-C₄)alkylcarbonyle, la déprotection s'effectue par hydrolyse en milieu alcalin en utilisant par exemple un hydroxyde de métal alcalin tel que l'hydroxyde de sodium, l'hydroxyde de potassium ou l'hydroxyde de lithium, dans un solvant inerte tel que l'eau, le méthanol, l'éthanol, le dioxane ou un mélange de ces solvants, à une température comprise entre 0°C et la température de reflux du solvant.

A l'étape 3) la réaction de l'alcool de formule (VIII) avec un chlorure de sulfonyle de formule (IX) s'effectue en présence d'une base telle que la triéthylamine, la pyridine, la N,N-diisopropyléthylamine ou la N-méthylmorpholine, dans un solvant inerte tel que le dichlorométhane, le benzène ou le toluène, à une température comprise entre -20°C et la température de reflux du solvant.

Le composé de formule (X) ainsi obtenu est mis en réaction à l'étape 4), avec un composé de formule (XI), selon des modes opératoires différents.

Lorsqu'on fait réagir un composé de formule (X) avec un composé de formule (XI), la réaction s'effectue dans un solvant inerte tel que le N,N-diméthylformamide, l'acétonitrile, le chlorure de méthylène, le toluène ou l'isopropanol et en présence ou en l'absence d'une base. Lorsqu'on utilise une base, celle-ci est choisie parmi les bases organiques telles que la triéthylamine, la N,N-diisopropyléthylamine ou la N-méthylmorpholine ou parmi les carbonates ou bicarbonates de métal alcalin tels que le carbonate de potassium, le carbonate de sodium ou le bicarbonate de sodium. En l'absence de base, la réaction s'effectue en utilisant un excès du composé de formule (XI) et en présence d'un iodure de métal alcalin tel que l'iodure de potassium ou l'iodure de sodium. Lorsque dans le composé de formule (X), -A- représente le radical bivalent -O-CO- ou -CH₂-O-CO-, la réaction s'effectue à une température comprise entre la température ambiante et 80°C. Lorsque dans le composé de formule (X) -A- représente le radical bivalent -O-CH₂-CO-, la réaction s'effectue à une température comprise entre la température ambiante et 100°C.

Selon la variante du procédé, à l'étape 1') on soumet un alcool de formule (VIII) à une oxydation pour obtenir un aldéhyde de formule (XXXVIII). La réaction d'oxydation s'effectue en utilisant par exemple le chlorure d'oxalyle, le diméthylsulfoxyde et la triéthylamine dans un solvant tel que de dichlorométhane et à une température comprise entre -78°C et la température ambiante.

Puis à l'étape 2'), on fait réagir le composé de formule (XI) avec un aldéhyde de formule (XXXVIII) en présence d'un acide tel que l'acide acétique, dans un solvant alcoolique tel que le méthanol, pour former *in-situ* une imine intermédiaire qui est réduite chimiquement en utilisant par exemple le cyanoborohydrure de sodium ou catalytiquement en utilisant l'hydrogène et un catalyseur tel que le palladium sur charbon ou le nickel de Raney®.

On obtient finalement après déprotection éventuelle des groupes hydroxyles ou des groupes aminés, ou transformation éventuelle de R'₃ en R₃, les composés de formule (Ic) selon l'invention.

Les produits de formule (Ic) ainsi obtenus sont isolés sous forme de base libre ou de sel, selon les techniques classiques.

Lorsque le composé de formule (Ic) est obtenu sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane, avec une solution de l'acide choisi dans le même solvant, on obtient le sel correspondant qui est isolé selon les techniques classiques.

Ainsi, on prépare par exemple le chlorhydrate, le bromhydrate, le sulfate, l'hydrogénosulfate, le dihydrogénophosphate, le méthanesulfonate, l'oxalate, le maléate, le fumarate, le naphtalène-2-sulfonate, le benzènesulfonate.

A la fin de la réaction, les composés de formule (Ic) peuvent être isolés sous forme d'un de leurs sels, par exemple le chlorhydrate, ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les composés de formule (II) se préparent selon différents modes opératoires.

Les composés de formule (II) dans laquelle -A- représente le radical bivalent -CH₂-O-CO- et E représente l'hydrogène ou un groupe O-protecteur se préparent selon le SCHEMA 1 ci-après dans lequel m et Ar₁ sont tels que définis pour un composé de formule (I) et Pr désigne un groupe O-protecteur tel que défini ci-dessus pour E.

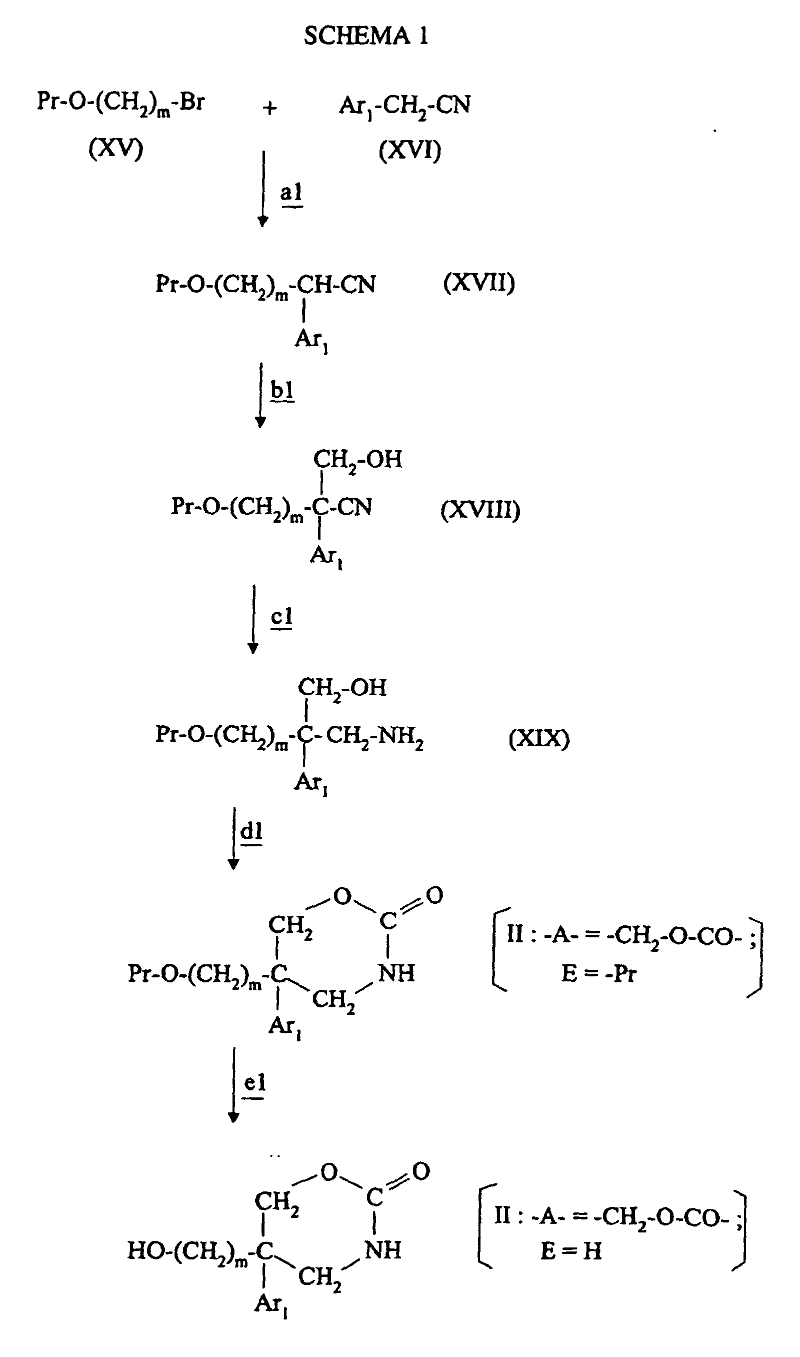

A l'étape a1 du SCHEMA 1, on fait réagir un composé de formule (XV) avec un composé de formule (XVI) selon la méthode décrite dans les demandes de brevet EP-A-0428434 et EP-A-0474561.

Le composé (XVII) ainsi obtenu est mis en réaction à l'étape b1 avec une solution aqueuse de formaldéhyde, en présence d'une base telle que le 1,8-diazabicyclo[5.4.0]undec-7-ène, dans un solvant tel que le 1,2-diméthoxyéthane, et à une température comprise entre la température ambiante et la température de reflux du solvant.

Le dérivé nitrile de formule (XVIII) est réduit à l'étape c1 pour obtenir l'amine primaire de formule (XIX). Cette réduction peut s'effectuer au moyen d'hydrogène, en présence d'un catalyseur tel que le nickel de Raney®, l'oxyde de platine ou le palladium sur charbon, dans un solvant inerte tel qu'un alcool, l'éthanol par exemple, seul ou en mélange avec de l'ammoniaque, ou au moyen d'un agent réducteur tel que l'hydrure d'aluminium lithium, l'hydrure de diisobutylaluminium, le borane dans le THF, dans un solvant tel que le toluène, l'hexane, l'éther de pétrole, le xylène ou le tétrahydrofurane. La réaction s'effectue à une température comprise entre 0°C et 70°C.

Le composé (XIX) est mis en réaction à l'étape d1 avec un dérivé réactif de l'acide carbonique tel que le phosgène en solution dans le toluène, le 1,1'-carbonyldiimidazole, en présence d'une base telle que la triéthylamine, la N,N-diisopropyléthylamine ou la N-méthylmorpholine, dans un solvant chloré tel que le dichlorométhane, le 1,2-dichloroéthane ou un éther tel que le tétrahydrofurane, et à une température comprise entre -70°C et la température ambiante pour obtenir un composé de formule (II) attendue dans laquelle E représente un groupe O-protecteur.

Par hydrolyse, selon les méthodes décrites précédemment, on élimine le groupe O-protecteur (étape e1) et obtient le composé de formule (II) dans laquelle E représente l'hydrogène.

Les composés de formule (II) dans laquelle -A- représente le radical bivalent -O-CH₂-CO- et E représente l'hydrogène ou un groupe O-protecteur se préparent selon le SCHEMA 2 ci-après dans lequel m et Ar₁ sont tels que définis ci-dessus. Pr₁ et Pr₂ représentent le groupe O-protecteur Pr tel que défini ci-dessus pour E, plus particulièrement Pr₁ représente un groupe O-protecteur hydrolysable en milieu acide, Pr₂ représente un groupe O-protecteur hydrolysable en milieu basique.

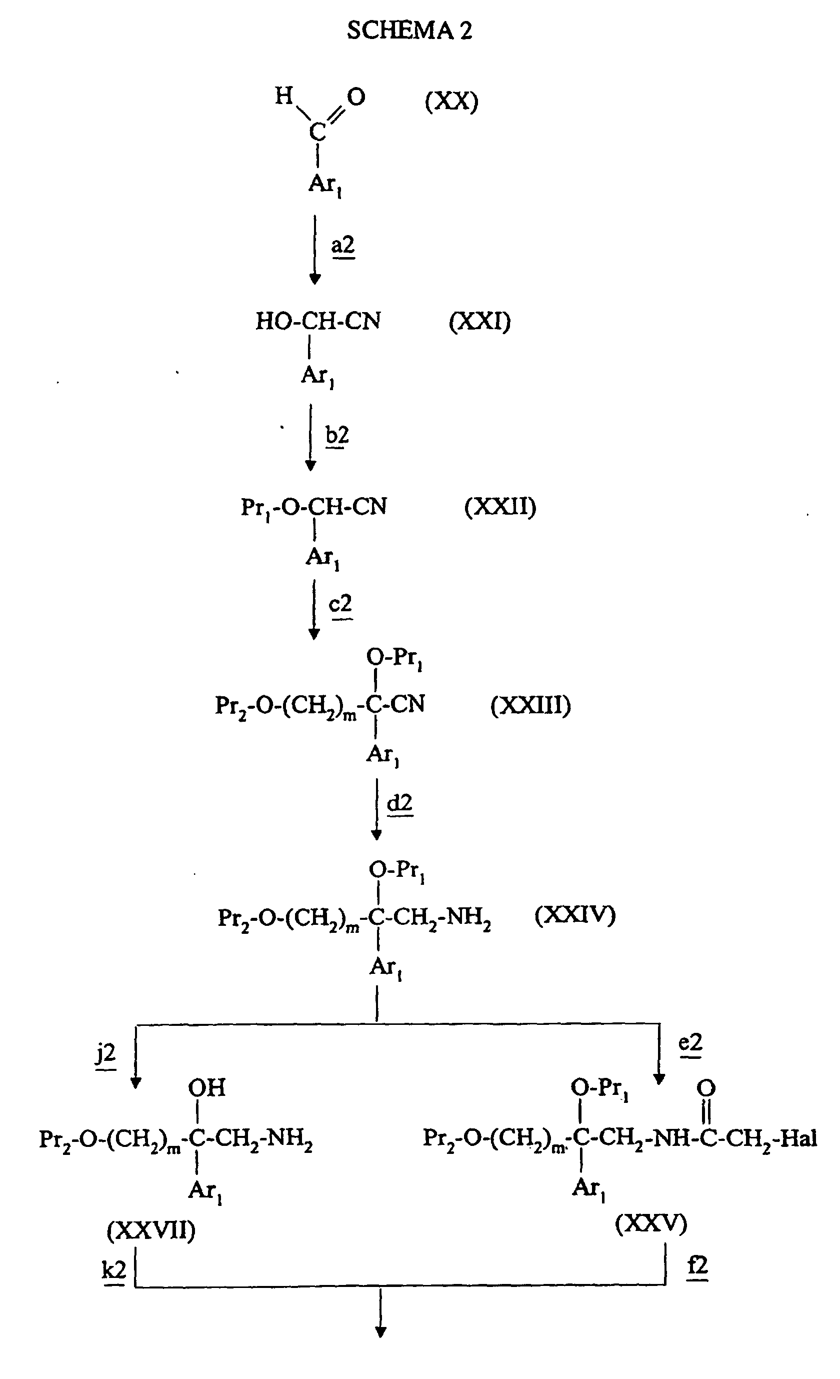

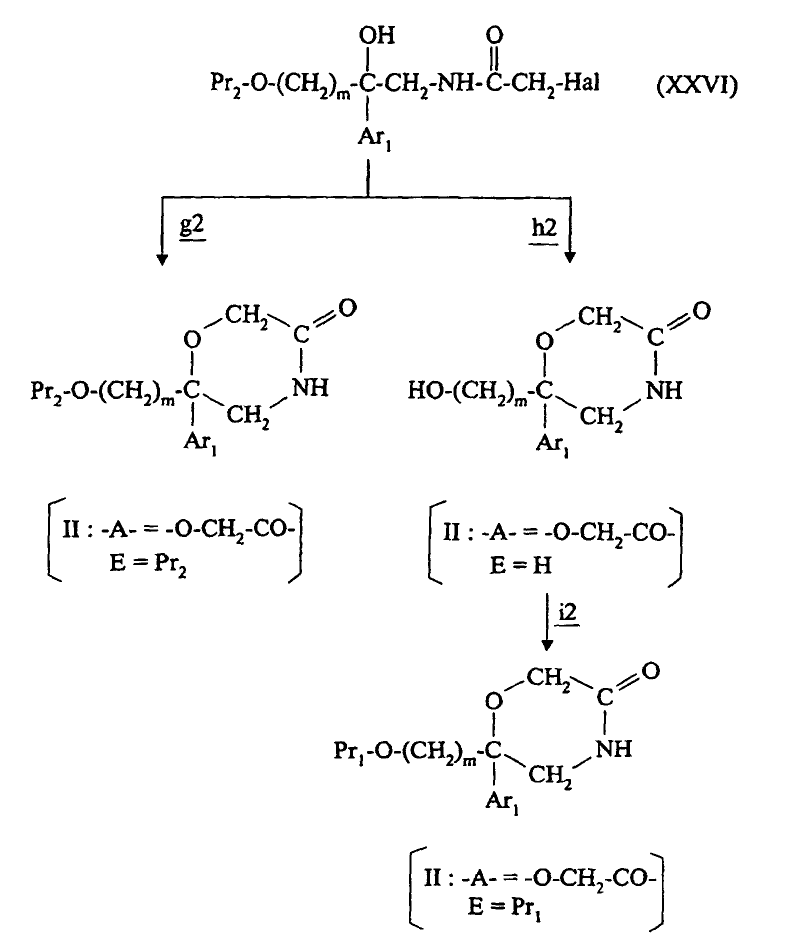

A l'étape a2 du SCHEMA 2, la synthèse d'une cyanhydrine de formule (XXI) à partir d'un aldéhyde de formule (XX) s'effectue selon les méthodes bien connues de l'homme de l'art telle que par exemple celle décrite dans Organic Syntheses ; Wiley, New-York, 1932 ; Collect. vol. 1, p. 336, ou par adaptation de cette méthode en utilisant l'action du métabisulfite de sodium et du cyanure de potassium en solution aqueuse.

A l'étape b2, le groupe hydroxy du composé de formule (XXI) est protégé selon les méthodes connues de l'homme de l'art.

Le composé de formule (XXII) ainsi obtenu est traité à l'étape c2 par une base forte telle que le diisopropylamidure de lithium, le *tert*-butylate de potassium ou l'hydrure de sodium pour fournir un carbanion qui est mis en réaction avec un composé de formule Hal-(CH₂)ₘ-O-Pr₂, dans laquelle Hal représente un halogène de préférence le brome ou le chlore, pour obtenir le composé de formule (XXIII). La réaction s'effectue dans un solvant inerte tel qu'un éther (tétrahydrofurane, éther diéthylique, 1,2-diméthoxyéthane par exemple) ou un amide (N,N-diméthylformamide par exemple) ou un hydrocarbure aromatique (toluène, xylène par exemple) à une température comprise entre -70°C et +60°C.

Le dérivé nitrile de formule (XXIII) est réduit à l'étape d2 selon les méthodes précédemment décrites, pour obtenir l'amine primaire de formule (XXIV).

A l'étape e2, le composé de formule (XXIV) est mis en réaction, avec un composé de formule Hal-CO-CH₂-Hal dans laquelle Hal représente un halogène, de préférence le chlore ou le brome, en présence d'une base telle qu'une amine tertiaire (triéthylamine, N-méthylmorpholine, pyridine par exemple) pour obtenir un composé de formule (XXV). La réaction s'effectue dans un solvant inerte tel qu'un solvant chloré (dichlorométhane, dichloroéthane, chloroforme par exemple), un éther (tétrahydrofurane, dioxane par exemple) ou un amide (N,N-diméthylformamide par exemple) à une température comprise entre -70°C et la température ambiante.

On élimine à l'étape f2 le groupe O-protecteur Pr₁ du composé de formule (XXV), par hydrolyse acide selon les méthodes précédemment décrites.

Alternativement, on élimine à l'étape j2 le groupe O-protecteur Pr₁ du composé de formule (XXIV) par hydrolyse acide, puis on met en réaction, à l'étape k2, le composé (XXVII) ainsi obtenu avec un composé de formule Hal-CO-CH₂-Hal selon les méthodes décrites ci-dessus à l'étape e2.

Le composé de formule (XXVI) ainsi obtenu est cyclisé en présence d'une base pour obtenir le composé de formule (II) attendue. Lorsqu'on veut obtenir un composé de formule (II) dans laquelle E représente un groupe protecteur Pr₂, on utilise une base telle qu'un carbonate de métal alcalin (le carbonate de potassium par exemple) ou un hydrure de métal alcalin (l'hydrure de sodium par exemple) ou le *tert*-butylate de potassium, dans un solvant inerte tel qu'un hydrocarbure aromatique (xylène, toluène par exemple) ou un amide (N,N-diméthylformamide par exemple) ou un éther (tétrahydrofurane par exemple), à une température comprise entre -30°C et la température de reflux du solvant (étape g2). Lorsqu'on veut obtenir un composé de formule (II) dans laquelle E représente l'hydrogène, on utilise une base telle qu'un hydroxyde de métal alcalin (l'hydroxyde de sodium, l'hydroxyde de potassium par exemple) en solution aqueuse concentrée dans un solvant tel qu'un alcanol (le propan-2-ol par exemple) ou un amide (le N,N-diméthylformamide par exemple) ou un mélange de ces solvants à une température comprise entre la température ambiante et la température de reflux du solvant (étape h2).

Eventuellement, on prépare à l'étape i2 un composé de formule (II) dans laquelle E représente un groupe O-protecteur Pr₁ selon les méthodes connues de l'homme de l'art.

Les composés de formule (II) dans laquelle -A- représente le radical bivalent -O-CO- et E représente l'hydrogène ou un groupe O-protecteur, se préparent selon le SCHEMA 3 ci-après dans lequel m et Ar₁ sont tels que définis ci-dessus et Pr₁ et Pr₂ sont tels que définis dans le SCHEMA 2 ci-dessus.

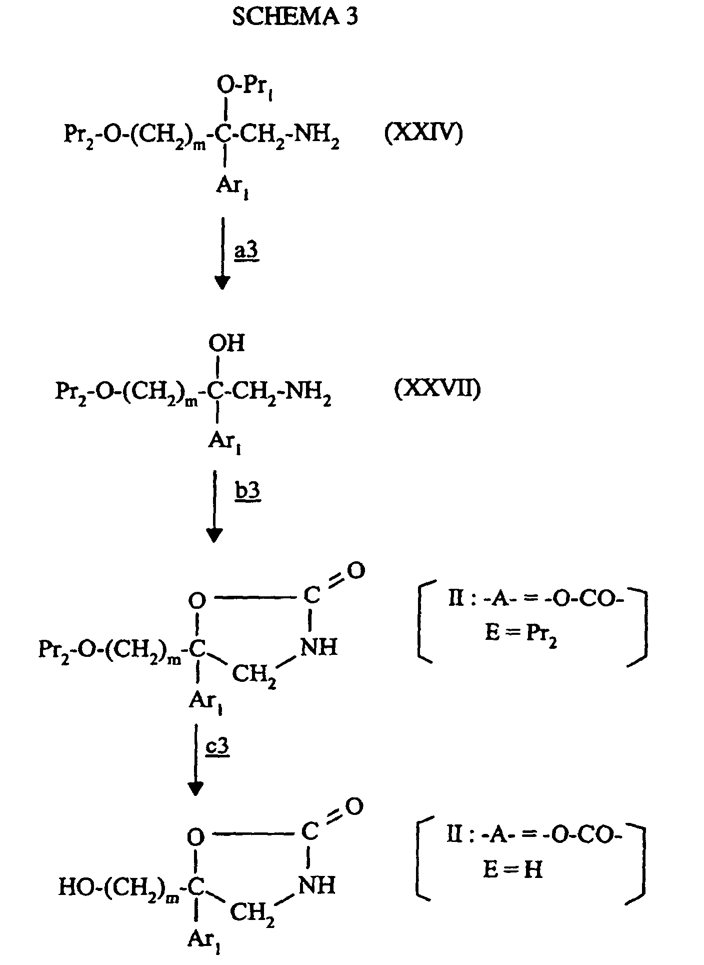

A l'étape a3 du SCHEMA 3, on élimine le groupe O-protecteur Pr₁ du composé de formule (XXIV), obtenu à l'étape d2 du SCHEMA 2, par hydrolyse acide selon les méthodes précédemment décrites.

Le composé de formule (XXVII) ainsi obtenu est mis en réaction à l'étape b3 avec un dérivé réactif de l'acide carbonique tel que le 1,1'-carbonyldiimidazole, le phosgène dans le toluène, le chloroformiate de *p*-nitrophényle, et en présence d'une base telle que la triéthylamine, la N,N-diisopropyléthylamine, la N-méthylmorpholine, pour obtenir un composé de formule (II) attendue dans laquelle E représente un groupe O-protecteur. La réaction s'effectue dans un solvant inerte tel qu'un solvant chloré (le 1,2-dichloroéthane, le dichlorométhane par exemple) ou un éther tel que le tétrahydrofurane ou un amide tel que le N,N-diméthylformamide ou un solvant aromatique tel que le toluène à une température comprise entre -60°C et la température ambiante.

Par hydrolyse basique, selon les méthodes décrites précédemment, on élimine le groupe O-protecteur Pr₂ (étape c3) pour obtenir le composé de formule (II) dans laquelle E représente l'hydrogène.

Les composés de formule (XVIII), (XIX), (XXIII), (XXIV), (XXVII), ainsi que les composés de formule (XXXIV) définis ultérieurement, constituent les intermédiaires clés pour la préparation des composés de formule (Ic).

Ainsi, selon un autre de ses aspects, la présente invention a pour objet des composés de formule : 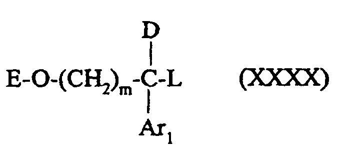 dans laquelle :
- m est 2 et Ar₁ est Ar₁ₐ, Ar₁ₐ étant tel que défini pour un composé de formule (Ic) ;
- E représente un hydrogène ou un groupe O-protecteur ;
- L représente un groupe cyano ou un groupe aminométhyle ;
- D représente un groupe choisi parmi : -CH₂-OH,
sous forme énantiomériquement pure ou sous forme racémique lorsque L est un groupe aminométhyle.

Les pipéridines de formule (XI) sont connues ou préparées par des méthodes connues, telles que celles décrites dans EP-A-0428434, EP-A-0474561, EP-A-0512901 et EP-A-0515240.

On peut également préparer les pipéridines de formule (XI) par des méthodes bien connues de l'homme de l'art, telles que celles décrites dans les publications suivantes ;
J. Heterocyclic. Chem., 1986, 23, 73-75
J. Chem. Soc., 1950, 1469
J. Chem. Soc., 1945, 917
J. Pharmaceutical. Sci., 1972, 61, 1316-1317
J. Org. Chem. 1957, 22, 1484-1489
Chem. Ber., 1975, 108, 3475-3482.

Les composés de formule (XI) sont généralement préparés sous forme protégée sur l'azote de la pipéridine ; après une étape de déprotection, on obtient les composés de formule (XI) eux-mêmes.

Plus particulièrement, pour préparer par exemple un composé de formule (XI), dans laquelle J'₁ représente un groupe 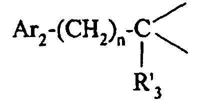 dans lequel Ar₂ représente un radical pyrid-2-yle, n est zéro et R'₃ représente un hydroxyle, on fait réagir la 2-bromopyridine sur la 1-benzylpipérid-4-one en présence d'une base telle que le butyllithium. Après élimination du groupe N-protecteur, on obtient la 4-hydroxy-4-(pyrid-2-yl)pipéridine attendue.

Pour préparer un composé de formule (XI) dans laquelle R'₃ représente un groupe -NR₄R₅ dans lequel R₄ et R₅ représentent chacun l'hydrogène, on effectue une hydrolyse en milieu acide d'un composé de formule (XI) dans laquelle R'₃ représente un groupe acétamido.

On prépare un composé de formule (XI) dans laquelle R'₃ représente un groupe -NR₄R₅ dans lequel R₄ et R₅ ensemble avec l'atome d'azote auquel ils sont liés constituent un hétérocycle, par application ou adaptation de la réaction de Bruylants (Bull. Soc. Chim. Belges, 1924, 33, 467 et Tetrahedron Letters, 1988, 29 (52), 6827-6830).

Pour préparer un composé de formule (XI) dans laquelle R'₃ représente un groupe -CH₂-NR₁₂R₁₃ dans lequel R₁₂ et R₁₃ représentent chacun l'hydrogène, on effectue la réduction d'un composé de formule (XI) dans laquelle R'₃ représente un cyano. Cette réduction s'effectue selon les méthodes bien connues de l'homme de l'art.

On prépare un composé de formule (XI) dans laquelle R'₃ représente un groupe -CH₂-CH₂-NR₁₂R₁₃ dans lequel R₁₂ et R₁₃ représentent chacun un hydrogène, à partir d'un composé de formule (XI) dans laquelle R'₃ représente un groupe -CH₂-CH₂-OH, par application ou adaptation de la méthode décrite dans J. Med. Chem., 1989, 32, 391-396.

Pour préparer un composé de formule (XI) dans laquelle R'₃ représente un groupe -NR₄R₅ dans lequel R₄ représente un hydrogène et R₅ représente un (C₁-C₇)alkyle, ou respectivement un (C₃-C₇)cycloalkylméthyle ou un benzyle on peut effectuer une réduction d'un composé de formule (XI) dans laquelle R'₃ représente un groupe -(CH₂)_{q}-NR₆COR₇ dans lequel q est zéro, R₆ représente l'hydrogène et R₇ représente un hydrogène ou un (C₁-C₆)alkyle, ou respectivement un (C₃-C₇)cycloalkyle ou un phényle. La réaction s'effectue au moyen d'un agent réducteur tel que l'hydrure d'aluminium et de lithium dans un solvant tel que le tétrahydrofurane à la température de reflux du solvant.

Par une réaction identique on peut préparer les composés de formule (XI) dans laquelle R'₃ représente un groupe -NR₄R₅ dans lequel R₄ représente un (C₁-C₇)alkyle et R₅ représente un (C₁-C₇)alkyle, ou respectivement un (C₃-C₇)cycloalkylméthyle ou un benzyle à partir d'un composé de formule (XI) dans laquelle R'₃ représente un groupe -(CH₂)_{q}-NR₆COR₇ dans lequel q est zéro, R₆ représente un (C₁-C₇)alkyle et R₇ représente un hydrogène ou un (C₁-C₆)alkyle, ou respectivement un (C₃-C₇)cycloalkyle ou un phényle.

De même on peut préparer les composés de formule (XI) dans laquelle R'₃ représente un groupe -CH₂-NR₁₂R₁₃ ou respectivement -CH₂CH₂NR₁₂R₁₃ dans lesquels R₁₂ représente un hydrogène ou un (C₁-C₇)alkyle et R₁₃ représente un (C₁-C₇)alkyle, un (C₃-C₇)cycloalkylméthyle ou un benzyle à partir d'un composé de formule (XI) dans laquelle R'₃ représente un groupe -(CH₂)_{q}-NR₆COR₇ dans lequel q est respectivement 1 ou 2, R₆ représente un hydrogène ou un (C₁-C₇)alkyle et R₇ représente un hydrogène, un (C₁-C₆)alkyle, un (C₃-C₇)cycloalkyle ou un phényle.

On prépare un composé de formule (XI) dans laquelle R'₃ représente un groupe -(CH₂)_{q}-NR₆COR₇ dans lequel R₆ et R₇ ensemble représentent un groupe -(CH₂)₃- ou -(CH₂)₄- par application ou adaptation de la méthode décrite dans J. Med. Chem., 1985, 28, 46-50.

Les composés de formule (XI) dans laquelle R'₃ représente un groupe -(CH₂)_{q}-NR₆C(=W₁)R₇ dans lequel W₁ représente un atome d'oxygène, q est 0, 1 ou 2, R₆ représente un hydrogène ou un (C₁-C₇)alkyle et R₇ représente un hydrogène ou respectivement, un (C₁-C₇)alkyle, un phényle, un benzyle, un pyridyle, un (C₃-C₇)cycloalkyle éventuellement substitué, un furyle, un thiényle, un pyrrolyle ou un imidazolyle, s'obtiennent par action, de l'acide formique dans l'anhydride acétique ou respectivement de l'anhydride approprié (R₇CO)₂O ou d'un chlorure d'acide approprié R₇COCl en présence d'une base telle que la triéthylamine, sur un composé de formule (XI) dans laquelle R'₃ représente un groupe -NHR₆, -CH₂-NHR₆ ou -CH₂-CH₂-NHR₆.

De la même façon, il est clair pour l'homme du métier que par action du chlorure d'acryloyle, on peut préparer les composés de formule (XI) dans laquelle R'₃ représente le groupe -(CH₂)_{q}-NR₆C(=W₁)R₇ dans lequel q, R₆ et W₁ sont tels que définis ci-dessus et R₇ est un groupe vinyle.

De même, par action d'un chloroformiate de formule ClCOOR₈ en présence d'une base telle que la triéthylamine, sur un composé de formule (XI) dans laquelle R'₃ représente un groupe -NHR₆, -CH₂-NHR₆ ou -CH₂-CH₂-NHR₆, on prépare les composés de formule (XI) dans laquelle R'₃ représente un groupe -(CH₂)_{q}-NR₆COOR₈.

Par action d'un chlorure de sulfonyle de formule ClSO₂R₉, en présence d'une base telle que la triéthylamine, sur un composé de formule (XI) dans laquelle R'₃ représente un groupe -NHR₆, -CH₂-NHR₆ ou -CH₂-CH₂-NHR₆, on prépare les composés de formule (XI) dans laquelle R'₃ représente un groupe -(CH₂)_{q}-NR₆SO₂R₉.

Par action d'un isocyanate de formule R₁₁ N=C=O, en présence d'une base telle que la triéthylamine, sur un composé de formule (XI) dans laquelle R'₃ représente un groupe -NHR₆, -CH₂-NHR₆ ou -CH₂-CH₂-NHR₆, on prépare les composés de formule (XI) dans laquelle R'₃ représente un groupe -(CH₂)_{q}-NR₆C(=W₁)NR₁₀R₁₁ dans lequel R₁₀ représente un hydrogène et W₁ représente un atome d'oxygène.

Par action d'un chlorure de carbamoyle de formule ClCONR₁₀R₁₁, en présence d'une base telle que la triéthylamine, sur un composé de formule (XI) dans laquelle R'₃ représente un groupe -NHR₆, -CH₂-NHR₆ ou -CH₂-CH₂-NHR₆, on prépare les composés de formule (XI) dans laquelle R'₃ représente un groupe -(CH₂)_{q}-NR₆C(=W₁)NR₁₀R₁₁ dans lequel R₁₀ représente un (C₁-C₇)alkyle et W₁ représente un atome d'oxygène.

On peut également obtenir un composé de formule (XI) dans laquelle R'₃ représente un groupe -(CH₂)_{q}-NR₆CONR₁₀R₁₁ par action d'un composé HNR₁₀R₁₁ avec un composé de formule (XI) dans laquelle R'₃ représente un groupe -(CH₂)_{q}-NR₆COOR₈ dans lequel R₈ représente un phényle.

On peut également préparer un composé de formule (XI) dans laquelle R'₃ représente un groupe -(CH₂)_{q}-NR₆COOR₈ dans lequel q = 0 et R₆ représente l'hydrogène par action d'un composé R₈OH avec un composé de formule (XI) dans laquelle R'₃ représente un groupe isocyanato, -N = C = O.

On peut aussi préparer un composé de formule (XI) dans laquelle R'₃ représente un groupe -(CH₂)_{q}-NR₆CONR₁₀R₁₁ dans lequel q = 0 et R₆ représente l'hydrogène par action d'un composé NHR₁₀R₁₁ avec un composé de formule (XI) dans laquelle R'₃ représente un groupe isocyanato.

On prépare un composé de formule (XI) dans laquelle R'₃ représente un groupe isocyanato à partir d'un composé de formule (XI) dans laquelle R'₃ représente un carboxy selon la méthode décrite dans Organic Synthesis, 51, 48-52.

On obtient un composé de formule (XI) dans laquelle R'₃ représente un groupe -(CH₂)_{q}-NR₆C(=W₁)R₇ dans lequel W₁ représente un atome de soufre à partir d'un composé de formule (XI) correspondant, protégé sur l'azote de la pipéridine, et dans lequel W₁ représente un atome d'oxygène par réaction avec du pentasulfure de phosphore ou avec le réactif de Lawesson, le 2,4-bis(4-méthoxyphényl)-1,3-dithia-2,4-diphosphétane-2,4-disulfure, suivi de la déprotection de l'azote de la pipéridine.

Par réaction d'un composé de formule (XI), protégé sur l'azote de la pipéridine, dans laquelle R'₃ représente un groupe -(CH₂)_{q}-NR₆CONR₁₀R₁₁ avec du pentasulfure de phosphore ou avec le réactif de Lawesson, on prépare un composé de formule (XI) dans laquelle R'₃ représente un groupe -(CH₂)_{q}-NR₆C(=W₁)NR₁₀R₁₁ dans lequel W₁ est un atome de soufre.

On prépare un composé de formule (XI) dans laquelle R'₃ représente un groupe -(CH₂)_{q}-OH dans lequel q est un ou respectivement deux, par réduction d'un composé de formule (XI) dans laquelle R'₃ représente un méthoxycarbonyle ou respectivement un méthoxycarbonylméthyle selon la méthode décrite dans Chem. Ber., 1975, 108, 3475-3482.

Par action d'un chlorure d'acide R₁₇COCl sur les composés de formule (XI) dans laquelle R'₃ représente un groupe -(CH₂)_{q}-OH et en présence d'une base telle que la triéthylamine, on obtient les composés de formule (XI) dans laquelle R'₃ représente un groupe -(CH₂)_{q}-OCOR₁₇ ; par action de l'acide formique on obtient les composés de formule (XI) dans laquelle R'₃ représente un groupe HCOO-(CH₂)_{q}-.

Par action d'un chlorure de carbamoyle (C₁-C₇)alkyl-NHCOCl sur les composés de formule (XI) dans laquelle R'₃ représente un groupe -(CH₂)_{q}-OH, on obtient les composés de formule (XI) dans laquelle R'₃ représente un groupe (C₁-C₇)alkyl-NHCOO-(CH₂)_{q}-.

On prépare les mêmes composés par action d'un isocyanate (C₁-C₇)alkyl-N=C=O sur les composés de formule (XI) dans laquelle R'₃ représente un groupe -(CH₂)_{q}-OH.

On peut préparer un composé de formule (XI) dans laquelle R'₃ représente un carboxy par hydrolyse d'un composé de formule (XI) dans laquelle R'₃ représente un cyano selon les méthodes connues de l'homme de l'art.

On peut préparer un composé de formule (XI) dans laquelle R'₃ représente un carboxyméthyle selon la méthode décrite dans Chem. Ber., 1975, 108, 3475-3482.

On peut préparer un composé de formule (XI) dans laquelle R'₃ représente un (C₁-C₇)alcoxycarbonyle ou respectivement un (C₁-C₇)alcoxycarbonylméthyle à partir d'un composé de formule (XI) dans laquelle R'₃ représente un carboxy ou respectivement un carboxyméthyle, par réaction d'estérification selon les méthodes bien connues de l'homme de l'art.

Pour préparer un composé de formule (XI) dans laquelle J' ₁ représente un groupe 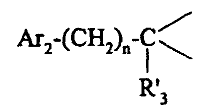 dans lequel Ar₂ représente un radical phényle éventuellement substitué, n est un et R'₃ représente un (C₁-C₇)alcoxycarbonyle, on fait réagir un 4-(C₁-C₇)alcoxycarbonylpipéridine protégé avec un halogénure de benzyle éventuellement substitué en présence d'une base telle que l'hydrure de sodium, le *tert*-butylate de potassium ou le diisopropylamidure de sodium dans un solvant tel que le tétrahydrofurane, le N,N-diméthylformamide ou le diméthylsulfoxyde, à une température comprise entre -78°C et la température ambiante. Après une étape de déprotection, on obtient le composé de formule (XI) attendue.

Pour préparer un composé de formule (XI) dans laquelle R'₃ représente un groupe -CONR₁₀R₁₁ ou respectivement un groupe -CH₂CONR₁₀R₁₁, on fait réagir un composé de formule (XI) dans laquelle R'₃ représente un carboxy ou respectivement un carboxyméthyle avec un composé de formule HNR₁₀R₁₁ selon les méthodes bien connues de l'homme de l'art.

Selon les méthodes précédemment citées, on prépare un composé de formule (XI) dans laquelle R'₃ représente un groupe -C(=W₁)NR₁₀R₁₁ ou un groupe -CH₂-C(=W₁)NR₁₀R₁₁ dans lesquels W représente un atome de soufre à partir d'un composé de formule (XI) correspondante dans laquelle W₁ représente un atome d'oxygène.

Pour préparer un composé de formule (XI) dans laquelle R'₃ représente un groupe 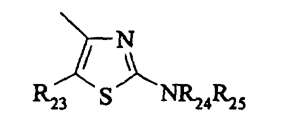 dans lequel R₂₄ et R₂₅ représentent chacun indépendamment un hydrogène ou un (C₁-C₇)alkyle, on fait réagir un composé de formule (XI) dans laquelle R'₃ représente un groupe : 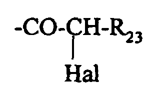 dans lequel Hal représente un atome d'halogène de préférence le brome, avec une thiourée dans laquelle un des groupes amino est libre ou substitué par un ou deux (C₁-C₇)alkyles.

On prépare un composé de formule (XI) dans laquelle R'₃ représente un groupe : 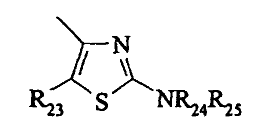 dans lequel R₂₅ représente un formyle ou respectivement un (C₁-C₇)alkylcarbonyle par réaction de l'acide formique dans l'anhydride acétique ou respectivement d'un chlorure d'acide (C₁-C₇)alkyl-COCl en présence d'une base telle que la triéthylamine, sur le composé de formule (XI) ci-dessus protégé sur l'azote de la pipéridine et dans laquelle R₂₅ représente l'hydrogène. Après une étape de déprotection on obtient le composé attendu.

Le composé de formule (XI) dans laquelle R'₃ représente un groupe : 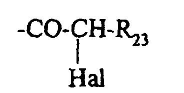 dans lequel Hal représente un atome de brome s'obtient par bromation selon les méthodes classiques d'un composé de formule (XI) dans laquelle R'₃ représente un groupe -CO-CH₂-R₂₃.

On peut préparer un composé de formule (XI) dans laquelle R'₃ représente un groupe -O-CH₂-CH₂-OR₁₈ dans lequel R₁₈ représente l'hydrogène par réaction d'un composé de formule (XI) dans laquelle R'₃ représente un benzoyloxy avec l'éthylèneglycol en présence d'un acide tel que l'acide sulfurique.

Par une réaction identique et en utilisant un 2-(C₁-C₇)alcoxyéthanol, on prépare les composés de formule (XI) dans laquelle R'₃ représente un groupe -O-CH₂CH₂-OR₁₈ dans lequel R₁₈ représente un (C₁-C₇)alkyle.

Par action de l'acide formique sur un composé de formule (XI) dans laquelle R'₃ représente un groupe -O-CH₂CH₂-OH on prépare les composés de formule (XI) dans laquelle R'₃ représente un groupe -O-CH₂CH₂-OR₁₈ dans lequel R₁₈ représente un formyle. Par action d'un chlorure d'acide en C₂-C₈ et en présence d'une base telle que la triéthylamine, on prépare les composés de formule (XI) dans laquelle R'₃ représente un groupe -O-CH₂CH₂-OR₁₈ dans lequel R₁₈ représente un (C₁-C₇)alkylcarbonyle.

On prépare un composé de formule (XI) dans laquelle R'₃ représente un groupe -NR₆COCOR₁₉ dans lequel R₁₉ représente un (C₁-C₄)alcoxy par réaction d'un composé de formule Cl-COCOR₁₉ avec un composé de formule (XI) dans laquelle R'₃ représente un groupe -NHR₆.

On prépare un composé de formule (XI) dans laquelle R'₃ représente un groupe -CO-NR₂₀-NR₂₁R₂₂ par réaction d'un composé HNR₂₀-NR₂₁R₂₂ avec un composé de formule (XI) dans laquelle R'₃ représente un chloroformyle.

On peut préparer un composé de formule (XI) dans laquelle R'₃ représente un groupe : 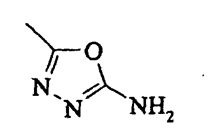 par réaction d'un composé de formule (XI) protégé dans laquelle R'₃ représente un groupe carbazoyle (-CONH-NH₂) avec le bromure de cyanogène selon la méthode décrite dans J. Org. Chem., 1961, 26, 88-95. Le composé de formule (XI) dans laquelle R'₃ représente un groupe carbazoyle s'obtient par réaction de l'hydrazine avec un composé de formule (XI) dans laquelle R'₃ représente un chloroformyle, lui-même obtenu par réaction du chlorure de thionyle avec un composé de formule (XI) dans laquelle R'₃ représente un carboxy.

Les énantiomères des composés selon l'invention, de formule : 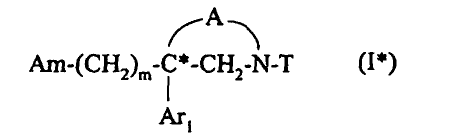 dans laquelle :
- "*" signifie que l'atome de carbone ainsi marqué a la configuration absolue (+) ou (-) déterminée ;
- Am est Amc tel que défini pour les composés de formule (Ic) ;
- m est 2 ;
- Ar₁ est Ar₁ₐ tel que défini pour les composés (Ic) ;
- A est Ac tel que défini pour les composés (Ic) ;
- T est -CH₂-Za, Za étant tel que défini pour les composés (Ic) ;
ainsi que leurs sels éventuels avec des acides minéraux ou organiques ;
sont des composés nouveaux qui font partie de l'invention.

La résolution des mélanges racémiques des composés de formule (Ic) permet d'isoler les énantiomères de formule (I*).

Il est cependant préférable d'effectuer le dédoublement des mélanges racémiques à partir d'un composé de formule (II) ou d'un composé intermédiaire utile pour la préparation d'un composé de formule (II).

Ainsi, lorsqu'on veut préparer les énantiomères (I*) des composés de formule (I) dans laquelle -A- représente le radical bivalent -CH₂-O-CO-, on effectue le dédoublement du mélange racémique d'un composé intermédiaire de formule : 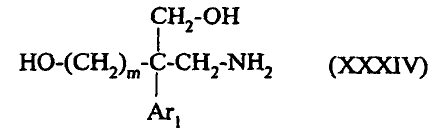 dans laquelle m et Ar₁ sont tels que définis pour un composé de formule (I), obtenu par élimination du groupe O-protecteur Pr, selon les méthodes précédemment décrites, d'un composé de formule (XIX).

Lorsqu'on veut préparer les énantiomères (I*) des composés de formule (I) dans laquelle -A- représente le radical bivalent -O-CO- ou -O-CH₂-CO ou -O-CH₂-CH₂- ou -O-CH₂-, on effectue le dédoublement du mélange racémique d'un composé intermédiaire de formule : 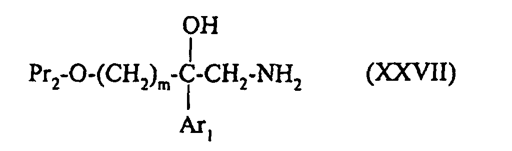 dans laquelle m et Ar₁ sont tels que définis pour un composé de formule (I) et Pr₂ est tel que défini précédemment dans le SCHEMA 2 ci-dessus.
dans laquelle m, Ar₁ et R₁ sont tels que définis pour un composé de formule (I).

Les composés de formule (Ic) ci-dessus comprennent également ceux dans lesquels un ou plusieurs atomes d'hydrogène, de carbone ou d'iode ont été remplacés par leur isotope radioactif par exemple le tritium, le carbone-14 ou l'iode-125. De tels composés marqués sont utiles dans des travaux de recherche, de métabolisme ou de pharmacocinétique, dans des essais biochimiques en tant que ligands de récepteurs.

L'affinité des composés pour les récepteurs aux tachykinines a été évaluée in vitro par plusieurs essais biochimiques utilisant des radioligands :
1) La liaison de [¹²⁵I] BH-SP (Substance P marquée à l'iode-125 à l'aide du réactif de Bolton-Hunter) aux récepteurs NK₁ des cellules lymphoblastiques humaines.
2) La liaison [¹²⁵I] His-NK_{A} aux récepteurs NK₂ du duodénum ou de la vessie de rat.
3) La liaison [¹²⁵I] His [MePhe⁷] NK_{B} aux récepteurs NK₃ du cortex cérébral de rat, du cortex cérébral de cobaye et du cortex cérébral de gerbille ainsi qu'aux récepteurs clones NK₃ humains exprimés par des cellules CHO (Buell et al., FEBS Letters, 1992, 299, 90-95).

Les essais ont été effectués selon X. Emonds-Alt et al. (Eur. J. Pharmacol., 1993, 250, 403-413).

Les composés selon l'invention présentent généralement une affinité pour les récepteurs aux tachykinines cités ci-dessus, avec une constante d'inhibition Ki inférieure à 10⁻⁸M.

Les composés de la présente invention sont notamment des principes actifs de compositions pharmaceutiques, dont la toxicité est compatible avec leur utilisation en tant que médicaments.

Les composés de la présente invention sont généralement administrés en unités de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (Ic) ou un de ses sels pharmaceutiquement acceptables.

Les composés de formule (Ic) ci-dessus et leurs sels pharmaceutiquement acceptables peuvent être utilisés à des doses journalières de 0,01. à 100 mg par kilo de poids corporel du mammifère à traiter, de préférence à des doses journalières de 0,1 à 50 mg/kg. Chez l'être humain, la dose peut varier de préférence de 0,5 à 4000 mg par jour, plus particulièrement de 2,5 à 1000 mg selon l'âge du sujet à traiter ou le type de traitement : prophylactique ou curatif.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif seul ou associé à un excipient, sous forme de poudre.

Le principe actif peut être formulé également sous forme de microcapsules, éventuellement avec un ou plusieurs supports ou additifs.

Dans chaque unité de dosage le principe actif de formule (Ic) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Les compositions susdites peuvent également renfermer d'autres produits actifs tels que, par exemple, des bronchodilatateurs, des antitussifs, des antihistaminiques, des antiinflammatoires, des antiémétiques, des agents de chimiothérapie.

Selon un autre de ses aspects, la présente invention concerne l'utilisation des produits de formule (Ic) pour la préparation de médicaments destinés à traiter des troubles physiologiques associés à un excès de tachykinines et toutes les pathologies neurokinine-dépendantes du système respiratoire, gastro-intestinal, urinaire, immunitaire, cardiovasculaire et du système nerveux central ainsi que la douleur et la migraine.

Par exemple et de manière non limitative :
- douleurs aigües et chroniques liées par exemple à la migraine, aux douleurs du cancéreux et de l'angineux, aux processus inflammatoires chroniques tels que l'ostéoarthrite et l'arthrite rhumatoïde,
- les inflammations telles que les inflammations neurogéniques, les maladies inflammatoires chroniques, par exemple, les maladies respiratoires chroniques obstructives, l'asthme, les allergies, les rhinites, les toux, les bronchites, l'hypersensibilité par exemple aux pollens et aux acariens, les arthrites rhumatoïdes, les fibrosites, les ostéoarthrites, les psoriasis, les colites ulcératives, la maladie de Crohn, l'inflammation des intestins (colon irritable), la prostatite, la vessie neurologique, l'incontinence, la cystite, l'urétrite, la néphrite, les maladies ophtalmiques telle que la conjonctivite, la vitréorétinopathie, les maladies cutanées telles que les dermatites de contact, les dermatites atopiques, l'urticaire, l'eczéma, le prurit, les brûlures, notamment les coups de soleil,
- les maladies du système immunitaire liées à la suppression ou à la stimulation des fonctions des cellules immunes par exemple l'arthrite rhumatoïde, le psoriasis, la maladie de Crohn, le diabète, le lupus, les réactions de rejet après transplantation,
- les cancers du poumon à petite cellule, les maladies de demyélination telles que la sclérose multiple ou la sclérose latérale amyotrophique,
- les maladies du système nerveux central du type neuropsychiatrique ou neurologique telles que l'anxiété, les troubles de la vigilance, de l'humeur, la dépression, la psychose, la schizophrénie, la manie, la démence, l'épilepsie, la maladie de Parkinson, la maladie d'Alzheimer, la drogue-dépendance, l'alcoolisme, le syndrôme de Down et la chorée d'Huntington ainsi que les maladies neurodégénératives, les désordres somatiques liés au stress,
- les maladies du système gastro-intestinal telles que nausées, vomissements de toutes origines, colon irritable, ulcères gastriques et duodénaux, ulcères oesophagiques, diarrhées, hypersécrétions,
- les maladies du système cardiovasculaire telles que l'hypertension, les aspects vasculaires de la migraine, les oedèmes, la thrombose, l'angine de poitrine, les spasmes vasculaires, les maladies circulatoires dues à une vasodilatation, les maladies de Reynauld, les fibroses, les maladies du collagène,
- les troubles de la fréquence et du rythme cardiaque, en particulier ceux qui sont occasionnés par la douleur ou le stress.

La présente invention inclut aussi une méthode pour traiter lesdites affections aux doses indiquées ci-dessus.

Dans les Préparations et dans les Exemples on utilise les abréviations suivantes :
Me, OMe : méthyle, méthoxy
Et, OEt : éthyle, éthoxy
EtOH : éthanol
MeOH : méthanol
Ether : éther diéthylique
Ether iso : éther diisopropylique
DMF : diméthylformamide
DMSO : diméthylsulfoxyde
DCM : dichlorométhane
THF : tétrahydrofurane
AcOEt : acétate d'éthyle
Na₂CO₃ : carbonate de sodium
NaHCO₃ : hydrogénocarbonate de sodium
NaCl : chlorure de sodium
Na₂SO₄ : sulfate de sodium
MgSO₄ : sulfate de magnésium
NaOH : soude
HCl : acide chlorhydrique
TFA : acide trifluoroacétique
KCN : cyanure de potassium
Na₂S₂O₅ : métabisulfite de sodium
DBU : 1,8-diazabicyclo[5.4.0]undec-7-ène
NH₄Cl : chlorure d'ammonium
F : point de fusion
TA : température ambiante
silice H : gel de silice 60H commercialisé par Merck (DARMSTADT)
RMN : résonnance magnétique nucléaire
δ : déplacement chimique
s : singulet
se : singulet élargi
d : doublet
t : triplet
qd : quadruplet
mt : multiplet
m : massif

### PREPARATIONS

### Préparation 1.1.

### 5-(3,4-Dichlorophényl)-5-[2-(tétrahydropyran-2-yloxy)éthyl]-tétrahydro-2H-1,3-oxazin-2-one.

### A) 2-(3,4-dichlorophényl)-4-(tétrahydropyran-2-yloxy)butanenitrile.

On refroidit au bain de glace une suspension de 17,75 g d'hydrure de sodium (à 80 % dans l'huile) dans 750 ml de THF, ajoute goutte à goutte une solution de 100 g de 3,4-dichlorophénylacétonitrile dans 250 ml de THF et laisse deux heures sous agitation à TA. On refroidit à -20°C le mélange réactionnel, ajoute goutte à goutte une solution de 112,36 g de 1-bromo-2-(tétrahydropyran-2-yloxy)éthane dans 120 ml de THF et laisse 2 heures sous agitation à TA. On concentre sous vide le mélange réactionnel, reprend le résidu à l'eau, extrait à l'éther, lave deux fois la phase organique par une solution tampon pH = 4, par une solution tampon pH = 7, deux fois par une solution saturée de NaCl, sèche sur Na₂SO₄ et évapore sous vide le solvant. On chromatographie le résidu sur silice en éluant au toluène puis par le mélange toluène/AcOEt (100/3 ; v/v). On obtient 113,5 g du produit attendu que l'on utilise tel quel.

### B) 2-(3,4-Dichlorophényl)-2-(hydroxyméthyl)-4-(tétrahydropyran-2-yloxy) butanenitrile.

On chauffe à reflux pendant 1 heure un mélange de 12,56 g du composé obtenu à l'étape précédente, 9,6 g d'une solution à 37 % de formaldéhyde dans l'eau, 0,3 g de DBU dans 25 ml de 1,2-diméthoxyéthane. On concentre sous vide le mélange réactionnel, extrait le résidu à l'éther, lave deux fois la phase organique à l'eau, deux fois par une solution tampon pH = 4, deux fois à l'eau, deux fois par une solution saturée de NaCl, sèche sur Na₂SO₄ et évapore sous vide le solvant. On obtient 17 g du produit attendu que l'on utilise tel quel.

### C) 2-(3,4-Dichlorophényl)-2-(hydroxyméthyl)-4-(tétrahydropyran-2-yloxy) butylamine.

On hydrogène pendant 5 heures à 40°C et à pression atmosphérique un mélange de 17 g du composé obtenu à l'étape précédente, 6 g de nickel de Raney® dans 300 ml d'EtOH et 40 ml d'une solution à 20% d'ammoniac dans l'eau. On filtre le catalyseur et concentre sous vide le filtrat. On reprend le résidu au DCM, lave la phase organique à l'eau, par une solution saturée de NaCl, sèche sur MgSO₄ et évapore sous vide le solvant. On obtient 16,5 g du produit attendu sous forme d'huile et que l'on utilise tel quel.

### D) 5-(3,4-Dichlorophényl)-5-[2-(tétrahydropyran-2-yloxy)éthyl]-tétrahydro-2H-1,3-oxazin-2-one.

On refroidit à -70°C 24,6 g d'une solution à 20% de phosgène dans le toluène, dilué dans 150 ml de DCM, ajoute goutte à goutte une solution de 16,5 g du composé obtenu à l'étape précédente, 5,7 g de triéthylamine dans 100 ml de DCM et laisse sous agitation en laissant remonter la température à TA. On concentre sous vide le mélange réactionnel, reprend le résidu par un mélange eau/AcOEt, essore le produit qui cristallise à l'interphase et obtient un premier jet du produit attendu. Après décantation du filtrat, on lave la phase organique à l'eau, par une solution tampon pH = 4, par une solution saturée de NaCl, sèche sur MgSO₄ et évapore sous vide le solvant. On reprend le résidu à l'AcOEt, essore le produit cristallisé formé et obtient le deuxième jet du produit. On obtient au total 4,5 g du produit attendu.

### Préparation 1.2

### 6-(3,4-Dichlorophényl)-6-[2-(tétrahydropyran-2-yloxy)éthyl]morpholin-3-one.

### A) 2-(3,4-dichlorophényl)-2-hydroxyacétonitrile.

On laisse une nuit sous agitation à TA un mélange de 70 g de 3,4-dichlorobenzaldéhyde, 90 g de Na₂S₂O₅ dans 300 ml d'eau. On refroidit à 0°C le mélange réactionnel, ajoute goutte à goutte une solution de 52 g de KCN dans 100 ml d'eau et laisse sous agitation en laissant remonter la température à TA. On extrait le mélange réactionnel à l'éther, lave la phase organique à l'eau, sèche sur Na₂SO₄ et évapore sous vide le solvant. On obtient 76 g du produit attendu que l'on utilise tel quel.

### B) 2-(3,4-dichlorophényl)-2-(tétrahydropyran-2-yloxy)acétonitrile.

On refroidit à 0°C une solution de 76 g du composé obtenu à l'étape précédente, 0,25 g d'acide *p*-toluènesulfonique monohydrate dans 300 ml de DCM, ajoute goutte à goutte une solution de 39 g de 3,4-dihydro-2*H*-pyrane dans 50 ml de DCM et laisse sous agitation en laissant remonter la température à TA. On lave le mélange réactionnel par une solution saturée de NaHCO₃, à l'eau, sèche la phase organique sur Na₂SO₄ et évapore sous vide le solvant. On obtient 33 g du produit attendu après cristallisation à 0°C dans le pentane, F = 61°C.

### C) 4-(Benzoyloxy)-2-(3,4-dichlorophényl)-2-(tétrahydropyran-2-yloxy)butanenitrile.

On refroidit à -60°C 56 ml d'une solution 2M de diisopropylamidure de lithium dans le THF, ajoute goutte à goutte une solution de 32 g du composé obtenu à l'étape précédente dans 50 ml de THF et laisse 1 heure sous agitation à -60°C. On ajoute ensuite à -60°C, goutte à goutte, une solution de 25,4 g de benzoate de 2-bromoéthyle dans 50 ml de THF et laisse sous agitation en laissant remonter la température à TA. On concentre sous vide le mélange réactionnel, extrait le résidu à l'éther, lave la phase organique à l'eau, par une solution tampon pH = 4, sèche sur Na₂SO₄ et évapore sous vide le solvant. On chromatographie le résidu sur silice en éluant par le mélange toluène/AcOEt (100/5 ; v/v). On obtient 34 g du produit attendu que l'on utilise tel quel.

### D) 4-(Benzoyloxy)-2-(3,4-dichlorophényl)-2-(tétrahydropyran-2-yloxy)butylamine.

On hydrogène à TA et à-pression atmosphérique un mélange de 34 g du composé obtenu à l'étape précédente, 10 g de nickel de Raney® dans 400 ml d'EtOH et 40 ml d'une solution concentrée d'ammoniaque. On filtre le catalyseur et concentre sous vide le filtrat. On reprend le résidu à l'eau, extrait à l'éther, lave la phase organique par une solution saturée de NaCl, sèche sur Na₂SO₄ et évapore sous vide le solvant. On chromatographie le résidu sur silice H en éluant par le gradient du mélange DCM/MeOH de (100/1 ; v/v) à (100/3 ; v/v). On obtient 16 g du produit attendu que l'on utilise tel quel.

### E) N-(2-bromoacétyl)-4-(benzoyloxy)-2-(3,4-dichlorophényl)-2-hydroxybutylamine.

On refroidit à -60°C une solution de 16 g du composé obtenu à l'étape précédente, 4,8 g de triéthylamine dans 100 ml de DCM, ajoute goutte à goutte une solution de 5,68 g de chlorure de bromoacétyle dans 20 ml de DCM et laisse 30 minutes sous agitation. On concentre sous vide le mélange réactionnel, extrait le résidu à l'éther, lave la phase organique à l'eau, par une solution tampon pH = 4, sèche sur Na₂SO₄ et évapore sous vide le solvant. On dissout le produit obtenu dans un minimum de MeOH, acidifie à pH = 1 par ajout d'une solution saturée d'HCl gaz dans l'éther et évapore sous vide les solvants. On reprend le résidu à l'eau, extrait à l'AcOEt, lave la phase organique par une solution saturée de NaHCO₃, à l'eau, sèche sur Na₂SO₄ et évapore sous vide le solvant. On obtient 16 g du produit attendu que l'on utilise tel quel.

### F) 6-(3,4-dichlorophényl)-6-(2-hydroxyéthyl)morpholin-3-one.

On laisse 4 heures sous agitation un mélange de 16 g du composé obtenu à l'étape précédente, 50 ml de propan-2-ol, 15 ml d'une solution de NaOH 10N et 10 ml de DMF. On concentre sous vide le mélange réactionnel, extrait le résidu à l'AcOEt, lave la phase organique à l'eau, sèche sur Na₂SO₄ et évapore sous vide le solvant. On chromatographie le résidu sur silice H en éluant par le gradient du mélange DCM/MeOH de (100/3 ; v/v) à (100/5 ; v/v). On obtient 6,1 g du produit attendu.

Spectre de RMN du proton à 200 MHz dans DMSO-d₆
δ : 2,05 ppm : mt : 2H
3,0 à 4,4 ppm : m : 6H
4,5 ppm : t : 1H
7,3 à 8,3 ppm : m : 4H

### G) 6-(3,4-Dichlorophényl)-6-[2-(tétrahydropyran-2-yloxy)éthyl]morpholin-3-one.

On refroidit à 0°C une solution de 1,7 g du composé obtenu à l'étape précédente, 0,003 g d'acide *p*-toluènesulfonique monohydrate dans 50 ml de DCM, ajoute goutte à goutte 0,588 g de 3,4-dihydro-2*H*-pyrane dans 10 ml de DCM et laisse 1 heure sous agitation à TA. On concentre sous vide le mélange réactionnel, extrait le résidu à l'éther, lave la phase organique par une solution saturée de NaHCO₃, à l'eau, sèche sur Na₂SO₄ et évapore sous vide le solvant. On chromatographie le résidu sur silice H en éluant par le mélange DCM/MeOH (100/2 ; v/v). On obtient 1,8 g du produit attendu que l'on utilise tel quel.

### Préparation 1.3

### 6-[2-(Benzoyloxy)éthyl]-6-(3,4-dichlorophényl)morpholin-3-one.

On chauffe à 130°C pendant une nuit un mélange de 4,8 g du composé obtenu à l'étape E de la Préparation 1.2, 1,4 g de K₂CO₃ dans 100 ml de xylène. Après refroidissement à TA, on filtre le mélange réactionnel et concentre sous vide le filtrat. On extrait le résidu à l'éther, lave la phase organique par une solution tampon pH = 2, à l'eau, sèche sur MgSO₄ et évapore sous vide le solvant. On chromatographie le résidu sur silice H en éluant par le mélange DCM/MeOH (100/1 ; v/v). On obtient 1,4 g du produit attendu.

Spectre de RMN du proton à 200 MHz dans DMSO-d₆
δ : 2,45 ppm : mt : 2H
3,75 ppm : système AB : 2H
3,9 à 4,5 ppm : m : 4H
7,4 à 7,9 ppm : m : 8H
8,25 ppm: se: 1H

On peut également obtenir ce composé en suivant les trois étapes du procédé décrit ci-après.

### A') Chlorhydrate de 4-(benzoyloxy)-2-(3,4-dichlorophényl)-2-hydroxybutylamine.

Ce composé est décrit à l'étape A de la Préparation 1.4.

### B') N-(2-Chloroacétyl)-4-(benzoyloxy)-2-(3,4-dichlorophényl)-2-hydroxybutylamine.

On refroidit à 0°C une solution de 20 g du composé obtenu à l'étape précédente, 10,3 g de triéthylamine dans 100 ml de DCM, ajoute goutte à goutte 5,8 g de chlorure de chloroacétyle et laisse 30 minutes sous agitation. On concentre sous vide le mélange réactionnel, extrait le résidu à l'AcOEt, lave la phase organique à l'eau, par une solution tampon pH = 2, à l'eau, sèche sur MgSO₄ et évapore sous vide le solvant. On obtient 22 g du produit attendu que l'on utilise tel quel.

### C') 6-[2-(Benzoyloxy)éthyl]-6-(3,4-dichlorophényl)morpholin-3-one.

On refroidit à -10°C une solution de 22 g du composé obtenu à l'étape précédente dans 600 ml de THF, ajoute 11,42 g de *tert*-butylate de potassium et laisse sous agitation jusqu'à dissolution complète. On concentre sous vide le mélange réactionnel, extrait le résidu à l'AcOEt, lave la phase organique par une solution tampon pH = 2, à l'eau, sèche sur MgSO₄ et évapore sous vide le solvant. On obtient 12,8 g du produit attendu après cristallisation dans l'éther.

### Préparation 1.4

### 5-[2-(Benzoyloxy)éthyl]-5-(3,4-dichlorophényl)oxazolidin-2-one.

### A) Chlorhydrate de 4-(benzoyloxy)-2-(3,4-dichlorophényl)-2-hydroxybutylamine.

A une solution de 12 g du composé obtenu à l'étape D de la Préparation 1.2 dans 50 ml de MeOH on ajoute à TA jusqu'à pH = 1 une solution saturée d'HCl gaz dans l'éther et laisse 1 heure sous agitation à TA. On concentre sous vide le mélange réactionnel, reprend le résidu au DCM, essore le précipité formé et le lave à l'éther. On obtient 3,4 g du produit attendu après recristallisation dans le propan-2-ol, F = 200-204°C.

### B) 5-[2-(Benzoyloxy)éthyl]-5-(3,4-dichlorophényl)oxazolidin-2-one.

A une solution de 3 g du composé obtenu à l'étape précédente, 0,85 g de triéthylamine dans 30 ml de 1,2-dichloroéthane, on ajoute à TA 1,4 g de 1,1'-carbonyldiimidazole, laisse 30 minutes sous agitation à TA puis chauffe à 50°C pendant 2 heures. On concentre sous vide le mélange réactionnel, reprend le résidu à l'eau, extrait au DCM, lave la phase organique par une solution tampon pH = 2, à l'eau, sèche sur Na₂SO₄ et évapore sous vide le solvant. On obtient 3 g du produit attendu.

Spectre de RMN du proton à 200 MHz dans DMSO-d₆
δ : 2,6 ppm : mt : 2H
3,75 ppm : système AB : 2H
4,35 ppm : mt : 2H
7,4 à 7,8 ppm : m : 8H
7,9 ppm : s : 1H

### Préparation 1.5

### 6-[2-(Benzoyloxy)éthyl]-6-(3,4-difluorophényl)morpholin-3-one.

### A) 2-(3,4-Difluorophényl)-2-hydroxyacétonitrile.

On chauffe à 50°C une solution de 80,2 g de Na₂S₂O₅ dans 250 ml d'eau, ajoute 50 g de 3,4-difluorobenzaldéhyde, laisse 1 heure sous agitation à 50°C et une nuit à TA. On refroidit à 0°C le mélange réactionnel, ajoute goutte à goutte une solution de 77,7 g du KCN dans 100 ml d'eau et laisse sous agitation en laissant remonter la température à TA puis poursuit l'agitation 1 heure à TA. On extrait le mélange réactionnel à l'éther, lave la phase organique à l'eau, sèche sur MgSO₄ et évapore sous vide le solvant. On obtient 48 g du produit attendu que l'on utilise tel quel.

### B) 2-(3,4-Difluorophényl)-2-(tétrahydropyran-2-yloxy)acétonitrile.

On refroidit à 0°C une solution de 48 g du composé obtenu à l'étape précédente, 0,2 g d'acide *p*-toluènesulfonique monohydrate dans 500 ml de DCM, ajoute goutte à goutte une solution de 28,6 g de 3,4-dihydro-2*H*-pyrane dans 50 ml de DCM et laisse sous agitation en laissant remonter la température à TA puis poursuit l'agitation une nuit à TA. On concentre sous vide le mélange réactionnel, extrait le résidu au DCM, lave la phase organique par une solution à 10 % de Na₂CO₃, à l'eau, sèche la phase organique sur Na₂SO₄ et évapore sous vide le solvant. On chromatographie le résidu sur silice en éluant par le mélange toluène/AcOEt (100/15 ; v/v). On obtient 43 g du produit attendu que l'on utilise tel quel.

### C) 4-(Benzoyloxy)-2-(3,4-difluorophényl)-2-(tétrahydropyran-2-yloxy) butanenitrile.

On refroidit à -60°C 133 ml d'une solution 1,5M de diisopropylamidure de lithium dans le THF, ajoute goutte à goutte une solution de 43 g du composé obtenu à l'étape précédente dans 250 ml de THF et laisse 30 minutes sous agitation à -60°C. On ajoute ensuite à -60°C, goutte à goutte, une solution de 45,8 g de benzoate de 2-bromoéthyle dans 100 ml de THF et laisse sous agitation en laissant remonter la température à TA. On concentre sous vide le mélange réactionnel, extrait le résidu à l'éther, lave la phase organique à l'eau, par une solution tampon pH = 4, sèche sur Na₂SO₄ et évapore sous vide le solvant. On chromatographie le résidu sur silice en éluant par le mélange toluène/AcOEt (100/5 ; v/v). On obtient 47 g du produit attendu que l'on utilise tel quel.

### D) 4-(Benzoyloxy)-2-(3,4-difluorophényl)-2-(tétrahydropyran-2-yloxy)butylamine.

On hydrogène à TA et à pression atmosphérique un mélange de 47 g du composé obtenu à l'étape précédente, 10 g de nickel de Raney® dans 400 ml d'EtOH. On filtre le catalyseur et concentre sous vide le filtrat. On reprend le résidu à l'eau, extrait à l'éther, lave la phase organique à l'eau, sèche sur Na₂SO₄ et évapore sous vide le solvant. On obtient 45 g du produit attendu que l'on utilise tel quel.

### E) Chlorhydrate de 4-(benzoyloxy)-2-(3,4-difluorophényl)-2-hydroxybutylamine.

A une solution de 45 g du composé obtenu à l'étape précédente dans 250 ml de MeOH on ajoute à TA jusqu'à pH = 1 une solution saturée d'HCl gaz dans l'éther et laisse 30 minutes sous agitation à TA. On concentre sous vide le mélange réactionnel, reprend le résidu à l'éther, essore le précipité formé et le lave à l'éther. On obtient 15 g du produit attendu après recristallisation dans le propan-2-ol, F = 202-204°C.

### F) N-(2-Chloroacétyl)-4-(benzoyloxy)-2-(3,4-difluorophényl)-2-hydroxybutylamine.

On refroidit à 0°C une solution de 12,2 g du composé obtenu à l'étape précédente, 7,88 g de triéthylamine dans 100 ml de DCM, ajoute goutte à goutte une solution de 3,85 g de chlorure de chloroacétyle dans 100 ml de DCM et laisse 30 minutes sous agitation. On concentre sous vide le mélange réactionnel, extrait le résidu par le mélange éther/AcOEt (50/50 ; v/v), lave la phase organique à l'eau, par une solution tampon pH = 4, à l'eau, sèche sur Na₂SO₄ et évapore sous vide le solvant. On obtient 13,5 g du produit attendu que l'on utilise tel quel.

### G) 6-[2-(Benzoyloxy)éthyl]-6-(3,4-difluorophényl)morpholin-3-one.

On chauffe à reflux pendant une nuit un mélange de 13,5 g du composé obtenu à l'étape précédente, 20,7 g de K₂CO₃ dans 100 ml de toluène. On concentre sous vide le mélange réactionnel, reprend le résidu à l'éther et filtre. On lave le filtrat à l'eau, par une solution tampon pH = 2, à l'eau, sèche sur MgSO₄ et évapore sous vide le solvant. On chromatographie le résidu sur silice H en éluant par le mélange DCM/MeOH (100/1 ; v/v). On obtient 4,9 g du produit attendu.

Spectre de RMN du proton à 200 MHz dans DMSO-d₆
δ : 2,35 ppm : mt : 2H
3,65 ppm : système AB : 2H
3,8 à 4,35 ppm : m : 4H
7,1 à 7,8 ppm : m : 8H
8,2 ppm : se : 1H

### Préparation 1.6

### 5-[2-(Benzoyloxy)éthyl]-5-(3,4-difluorophényl)oxazolidin-2-one.

A une solution de 2,1 g du composé obtenu à l'étape E de la Préparation 1.5, 0,63 g de triéthylamine dans 50 ml de 1,2-dichloroéthane, on ajoute à TA 1 g de 1,1'-carbonyldiimidazole, laisse ensuite 1 heure sous agitation à TA puis chauffe à 50°C pendant 2 heures. On concentre sous vide le mélange réactionnel, extrait le résidu au DCM, lave la phase organique à l'eau, par une solution tampon pH = 2, à l'eau, sèche sur MgSO₄ et évapore sous vide le solvant. On obtient 1,5 g du produit attendu.

### Préparation 2.1

### 4-Phényl-4-(pyrrolidin-1-ylcarbonyl)pipéridine, hémihydrate.

### A) 1-tert-Butoxycarbonyl-4-carboxy-4-phénylpipéridine.

A un mélange de 30 g de *p*-toluènesulfonate de 4-carboxy-4-phénylpipéridine dans 300 ml de dioxane, on ajoute 30 ml d'eau, 32,9 g de K₂CO₃, puis chauffe à 60°C et ajoute goutte à goutte 18,2 g de di-*tert*-butyldicarbonate. On chauffe ensuite 2 heures à 60°C puis 30 minutes à reflux. Après refroidissement à TA, on concentre sous vide le mélange réactionnel, extrait le résidu au DCM, lave la phase organique par une solution tampon pH = 2, acidifie à pH = 4 par ajout d'HCl 2N, lave par une solution tampon pH = 2, à l'eau, par une solution saturée de NaCl, sèche sur MgSO₄ et évapore sous vide le solvant. On obtient 23,7 g du produit attendu.

### B) 1-tert-Butoxycarbonyl-4-(pyrrolidin-1-ylcarbonyl)-4-phénylpipéridine.

A une solution de 14 g du composé obtenu à l'étape précédente dans 200 ml de DCM, on ajoute 9,29 g de triéthylamine puis 3,27 g de pyrrolidine. On refroidit au bain de glace, ajoute 22,4 g de BOP et laisse sous agitation en laissant remonter la température à TA. On concentre sous vide le mélange réactionnel, extrait le résidu au DCM, lave la phase organique à l'eau, trois fois par une solution de NaOH à 10 %, à l'eau, trois fois par une solution saturée de NaCl, sèche sur MgSO₄ et évapore sous vide le solvant. On obtient 16,4 g du produit attendu.

### C) 4-Phényl-4-(pyrrolidin-1-ylcarbonyl)pipéridine, hémihydrate.

A une solution de 16,4 g du composé obtenu à l'étape précédente dans 200 ml de MeOH, on ajoute jusqu'à pH = 1 une solution d'HCl concentrée et laisse 5 heures sous agitation à TA. On concentre sous vide le mélange réactionnel, reprend le résidu à l'acétone et évapore sous vide le solvant. On obtient un solide blanc que l'on recristallise dans le propan-2-ol. On reprend le produit obtenu par une solution de NaOH à 10 %, extrait au DCM, lave la phase organique par une solution de NaOH à 10 %, par une solution saturée de NaCl, sèche sur MgSO₄ et évapore sous vide le solvant. On obtient 7 g du produit attendu après cristallisation dans l'éther, F = 126°C.

### Préparation 2.2

### p-Toluènesulfonate de 4-(N',N'-diméthyluréido)-4-phénylpipéridine, hémihydrate

### A) 4-Acétamido-1-benzyl-4-phénylpipéridine.

Ce composé est préparé par action de l'acétonitrile sur le 1-benzyl-4-hydroxy-4-phénylpipéridine selon le procédé décrit dans EP-A-474561.

### B) Dichlorhydrate de 4-amino-1-benzyl-4-phénylpipéridine.

On chauffe à reflux pendant 48 heures un mélange de 30 g du composé obtenu à l'étape précédente, 58 ml d'une solution d'HCl concentrée dans 135 ml d'eau. On concentre sous vide le mélange réactionnel, reprend le résidu dans un mélange EtOH/toluène et évapore sous vide les solvants. On dissout le résidu dans 50 ml de MeOH et cristallise par addition de 250 ml d'acétone. On obtient 30,5 g du produit attendu après essorage et séchage.

### C) 1-Benzyl-4-(N',N'-diméthyluréido)-4-phénylpipéridine.

A une solution de 6 g du composé obtenu à l'étape précédente, 7,14 g de triéthylamine dans 50 ml de 1,2-dichloroéthane, on ajoute à TA, goutte à goutte, une solution de 1,9 g de chlorure de N,N-diméthylcarbamoyle dans 10 ml de 1,2-dichloroéthane et chauffe à reflux pendant 8 heures. On rajoute quelques gouttes de chlorure de N,N-diméthylcarbamoyle et poursuit le reflux pendant 3 heures. On concentre sous vide le mélange réactionnel, extrait le résidu au DCM, lave la phase organique à l'eau, par une solution de NaOH à 10 %, à l'eau, par une solution saturée de NaCl, sèche sur MgSO₄ et évapore sous vide le solvant. On chromatographie le résidu sur silice en éluant par le gradient du mélange DCM/MeOH de (99/1 ; v/v) à (96/4 ; v/v). On obtient 1,8 g du produit attendu.

### D) p-Toluènesulfonate de 4-(N',N'-diméthyluréido)-4-phénylpipéridine, hémihydrate.

On hydrogène à 40°C et à pression atmosphérique un mélange de 1,8 g du composé obtenu à l'étape précédente, 1,11 g d'acide *p*-toluènesulfonique monohydrate, 0,2 g de palladium sur charbon à 10 % dans 150 ml d'EtOH 95. On filtre le catalyseur sur Célite® et évapore sous vide le filtrat. On reprend le résidu à l'acétone et évapore sous vide le solvant. On dissout le produit obtenu dans 25 ml d'acétone, ajoute lentement cette solution dans 200 ml d'éther et essore le produit cristallisé formé. On obtient 1,86 g du produit attendu, F = 120-122°C.

### Préparation 2.3

### p-Toluènesulfonate de 4-(acétyl-N-méthylamino)-4-phénylpipéridine.

### A) 1-Benzyl-4-(formylamino)-4-phénylpipéridine.

A une solution de 48,9 g du composé obtenu à l'étape B de la Préparation 2.2, 25 g de formiate de sodium dans 340 ml d'acide formique, on ajoute goutte à goutte 110 ml d'anhydride acétique puis laisse une nuit sous agitation à TA. On concentre sous vide le mélange réactionnel, reprend le résidu à l'eau, alcalinise par ajout d'une solution de NaOH concentrée, extrait au DCM, sèche la phase organique sur MgSO₄ et évapore sous vide le solvant. On obtient 38,8 g du produit attendu après cristallisation dans le mélange éther iso/pentane, F = 140°C.

### B) 1-Benzyl-4-(méthylamino)-4-phénylpipéridine.

A une suspension de 12,5 g d'hydrure d'aluminium et de lithium dans 100 ml de THF, on ajoute lentement une solution de 38,8 g du composé obtenu à l'étape précédente dans 400 ml de THF et chauffe à reflux pendant 3 heures. Après refroidissement, on ajoute au mélange réactionnel une solution de 5 ml de NaOH concentrée dans 45 ml d'eau, filtre les sels minéraux et concentre sous vide le filtrat. On obtient 38 g du produit attendu.

### C) 4-(Acétyl-N-méthylamino)-1-benzyl-4-phénylpipéridine.

On refroidit à 0-5°C une solution de 30 g du composé obtenu à l'étape précédente, 16,5 ml de triéthylamine dans 300 ml de DCM, ajoute goutte à goutte 8 ml de chlorure d'acétyle et laisse 30 minutes sous agitation à TA. On lave deux fois le mélange réactionnel à l'eau, par une solution de NaOH 2N, sèche la phase organique sur MgSO₄ et évapore sous vide le solvant. On obtient 31,6 g du produit attendu après cristallisation dans le mélange éther iso/pentane, F = 104°C.

### D) p-Toluènesulfonate de 4-(acétyl-N-méthylamino)-4-phénylpipéridine.

On hydrogène pendant 3 heures à 25°C et à pression atmosphérique un mélange de 5 g du composé obtenu à l'étape précédente, 2,9 g d'acide *p*-toluènesulfonique monohydrate, 0,5 g de palladium sur charbon à 10 % et 80 ml d'EtOH. On filtre le catalyseur et concentre sous vide le filtrat. On obtient 5,7 g du produit attendu après cristallisation dans l'acétone, F = 165°C.

### Préparation 2.4

### Trifluoroacétate de 4-(éthoxycarbonylamino)-4-phénylpipéridine.

### A) 1-tert-Butoxycarbonyl-4-isocyanato-4-phénylpipéridine.

On refroidit à 0-5°C une solution de 25 g du composé obtenu à l'étape A de la Préparation 2.1, 10,35 g de triéthylamine dans 100 ml d'acétone, ajoute goutte à goutte à une température inférieure à 5°C une solution de 8,7 g de chloroformiate de méthyle dans 30 ml d'acétone et laisse 30 minutes sous agitation à 5°C. Puis on ajoute à une température inférieure à 5°C et goutte à goutte une solution de 10,66 g d'azidure de sodium dans 30 ml d'eau et laisse 30 minutes sous agitation à 5°C. On verse le mélange réactionnel sur 500 ml d'eau glacée, extrait quatre fois au toluène, lave deux fois la phase organique par une solution tampon pH = 2, par une solution saturée de NaCl, sèche sur MgSO₄ et filtre. On chauffe à 90°C le filtrat pendant 1 heure et évapore sous vide le solvant. On obtient 18,9 g du produit attendu sous forme d'huile.

### B) 1-tert-Butoxycarbonyl-4-(éthoxycarbonylamino)-4-phénylpipéridine.

On chauffe à reflux pendant 5 heures 30 minutes une solution de 6,28 g du composé obtenu à l'étape précédente dans 100 ml d'EtOH. On ajoute deux gouttes de triéthylamine puis laisse une nuit sous agitation à TA et concentre sous vide. On obtient 7,25 g du produit attendu.

### C) Trifluoroacétate de 4-(éthoxycarbonylamino)-4-phénylpipéridine.

On laisse 30 minutes sous agitation à TA une solution de 7,25 g du composé obtenu à l'étape précédente dans 20 ml de TFA puis concentre sous vide. On reprend le résidu dans l'acétone et évapore sous vide le solvant. On obtient 6,02 g du produit attendu après cristallisation dans le mélange acétone/éther, F = 173°C.

### Préparation 2.5

### Benzènesulfonate de 4-phényl-4-(pyrrolidin-1-ylcarbonylamino)pipéridine.

### A) 1-(Benzyloxycarbonyl)-4-carboxy-4-phénylpipéridine.

On refroidit à 5°C un mélange de 37,7 g de *p*-toluènesulfonate de 4-carboxy-4-phénylpipéridine, 53,3 g d'une solution aqueuse à 30 % de NaOH et 250 ml d'eau. On ajoute rapidement à 5°C une solution de 18 g de chloroformiate de benzyle dans 60 ml d'acétone et laisse une nuit sous agitation en laissant remonter la température à TA. On lave deux fois le mélange réactionnel à l'éther et acidifie, après décantation, la phase aqueuse à pH = 1 par ajout d'HCl concentré puis d'HCl 2N. On essore le précipité formé, le sèche, le reprend à l'éther et l'essore à nouveau. On obtient 30,6 g du produit attendu, F = 142-144°C.

### B) 1-(Benzyloxycarbonyl)-4-phényl-4-(pyrrolidin-1-ylcarbonylamino)pipéridine.

On chauffe à reflux pendant 1 heure un mélange de 33,9 g du composé obtenu à l'étape précédente et 47,6 g de chlorure de thionyle dans 200 ml de 1,2-dichloroéthane. On concentre sous vide le mélange réactionnel, reprend le résidu à l'acétone et évapore sous vide le solvant. On dissout le résidu dans 200 ml d'acétone, refroidit à 5°C, ajoute goutte à goutte une solution de 13 g d'azidure de sodium dans 50 ml d'eau et laisse 1 heure sous agitation. On évapore sous vide à TA 100 ml d'acétone, ajoute à la solution restante une solution saturée de NaHCO₃, extrait au toluène, lave la phase organique à l'eau, par une solution saturée de NaCl, sèche sur Na₂SO₄ et évapore 50 % en volume de solvant. On chauffe à reflux pendant 30 minutes la solution toluénique restante puis concentre sous vide. On reprend le résidu dans 200 ml d'éther, ajoute goutte à goutte une solution de 7,1 g de pyrrolidine dans 20 ml d'éther et laisse 5 minutes sous agitation. On concentre sous vide, extrait le résidu au DCM, lave la phase organique par une solution d'HCl 2N, par une solution à 5 % de NaHCO₃, par une solution saturée de NaCl, sèche sur Na₂SO₄ et évapore sous vide le solvant. On dissout le résidu dans 40 ml d'AcOEt chaud, ajoute 200 ml d'éther et essore le produit cristallisé formé. On obtient 31,4 g du produit attendu.

### C) Benzènesulfonate de 4-phényl-4-(pyrrolidin-1-ylcarbonylamino)pipéridine.

On hydrogène à 40°C et à pression atmosphérique un mélange de 29 g du composé obtenu à l'étape précédente, 11,27 g d'acide benzènesulfonique, 2 g de palladium sur charbon à 10 % et 250 ml d'EtOH. On filtre le catalyseur sur Célite® et concentre sous vide le filtrat. On cristallise le résidu dans un mélange EtOH/acétone. On obtient 29,4 g du produit attendu, F = 185°C.

### Préparation 2.6

### p-Toluènesulfonate de 4-[2-(diméthylamino)thiazol-4-yl]-4-phénylpipéridine.

### A) 1,1-Diméthylthiourée.

On refroidit au bain de glace une solution de 10,67 g de thiocyanate de potassium dans 100 ml d'acétone, ajoute goutte à goutte une solution de 12,06 g de chlorure de pivaloyle dans 30 ml d'acétone et laisse 30 minutes sous agitation en laissant remonter la température à TA. On refroidit à -10°C le mélange réactionnel, ajoute goutte à goutte 17,9 ml d'une solution 5,6N de diméthylamine dans le MeOH et laisse sous agitation en laissant remonter la température à TA. On concentre sous vide, extrait le résidu à l'éther, lave deux fois la phase organique à l'eau, par une solution saturée de NaCl, sèche sur MgSO₄ et évapore sous vide le solvant. On reprend le produit obtenu dans 50 ml d'une solution d'HCl concentrée et chauffe à reflux pendant 1 heure. Après refroidissement à TA, on lave deux fois le mélange réactionnel à l'éther, alcalinise la phase aqueuse à pH = 9 par ajout d'une solution à 30 % de NaOH, extrait au DCM, lave la phase organique par une solution à 5 % de NaOH, par une solution saturée de NaCl, sèche sur MgSO₄ et évapore sous vide le solvant. On reprend le résidu dans l'éther et essore le précipité formé, on obtient 6,7 g du produit attendu.

### B) Bromhydrate de 4-(2-bromoacétyl)-4-phénylpipéridine.

A une suspension de 14 g de bromhydrate de 4-acétyl-4-phénylpipéridine dans 200 ml de DCM on ajoute rapidement à TA 7,9 g de brome et laisse une nuit sous agitation à TA. On dilue le mélange réactionnel par ajout de 200 ml d'éther, essore le précipité formé et le lave à l'éther. On obtient 16,7 g du produit attendu après séchage sous vide.

### C) p-Toluènesulfonate de 4-[2-(diméthylamino)thiazol-4-yl]-4-phénylpipéridine.

On chauffe à reflux pendant 1 heure 30 minutes un mélange de 7,26 g du composé obtenu à l'étape B), 2,08 g du composé obtenu à l'étape A) dans 150 ml d'EtOH. Après refroidissement à TA, on concentre sous vide, reprend le résidu à l'eau, alcalinise à pH = 10 par ajout d'une solution à 10 % de NaOH, extrait au DCM, lave la phase organique par une solution à 10 % de NaOH, par une solution saturée de NaCl, sèche sur MgSO₄ et évapore sous vide le solvant. On dissout le produit obtenu dans l'acétone, ajoute goutte à goutte une solution de 3,31 g d'acide *p*-toluènesulfonique monohydrate dans 10 ml d'acétone et essore le produit cristallisé formé. On obtient 6,1 g du produit attendu, F = 164°C.

### Préparation 2.7

### p-Toluènesulfonate de 4-(morpholin-4-ylcarbonylamino)-4-phénylpipéridine, monohydrate.

### A) 1-tert-Butoxycarbonyl-4-(morpholin-4-ylcarbonylamino)-4-phénylpipéridine.

A une solution de 6 g du composé obtenu à l'étape A de la Préparation 2.4 dans 100 ml d'acétone, on ajoute à TA et goutte à goutte une solution de 1,74 g de morpholine dans 10 ml d'acétone. On concentre sous vide le mélange réactionnel, extrait le résidu à l'AcOEt, lave la phase organique par une solution tampon pH = 2, par une solution saturée de NaCl, sèche sur MgSO₄ et évapore sous vide le solvant. On obtient 7,1 g du produit attendu.

### B) p-Toluènesulfonate de 4-(morpholin-4-ylcarbonylamino)-4-phénylpipéridine, monohydrate.

A une solution de 7,1 g du composé obtenu à l'étape précédente dans 100 ml de MeOH, on ajoute 15 ml d'une solution d'HCl concentrée et laisse une nuit sous agitation à TA. On concentre sous vide le mélange réactionnel, reprend le résidu à l'eau, alcalinise à pH = 10 par ajout d'une solution de NaOH concentrée, extrait trois fois au DCM, lave les phases organiques jointes par une solution saturée de NaCl, sèche sur MgSO₄ et évapore sous vide le solvant. On dissout le produit obtenu dans l'acétone, ajoute goutte à goutte une solution de 3,46 g d'acide *p*-toluènesulfonique monohydrate dans 10 ml d'acétone, et concentre sous vide. On reprend le résidu dans l'éther et essore le précipité formé. On obtient 7 g du produit attendu, F = 93°C.

### Préparation 2.8

### Benzènesulfonate de 4-phényl-4-(pyrrolidin-1-ylaminocarbonyl)pipéridine.

### A) Benzènesulfonate de 1-(benzyloxycarbonyl)-4-phényl-4-(pyrrolidin-1-ylaminocarbonyl)pipéridine.

On chauffe à reflux un mélange de 11,54 g du composé obtenu à l'étape A de la Préparation 2.5 dans 100 ml de 1,2-dichloroéthane, puis ajoute 16,18 g de chlorure de thionyle, chauffe à reflux pendant 1 heure et laisse une nuit sous agitation à TA. On concentre sous vide le mélange réactionnel, dissout le résidu dans 100 ml de DCM, refroidit à 5°C et ajoute successivement 10,3 g de triéthylamine puis 5 g de chlorhydrate de 1-aminopyrrolidine. Après 1 heure sous agitation, on concentre sous vide le mélange réactionnel, extrait le résidu à l'AcOEt, lave la phase organique à l'eau, par une solution saturée de NaCl, sèche sur MgSO₄ et évapore sous vide le solvant. On dissout le produit obtenu dans l'acétone, ajoute de l'éther jusqu'à précipitation et essore le précipité formé. On dissout le précipité dans l'EtOH, ajoute 2,61 g d'acide benzènesulfonique et essore le précipité formé. On obtient 9,34 g du produit attendu.

### B) Benzènesulfonate de 4-phényl-4-(pyrrolidin-1-ylaminocarbonyl)pipéridine.

On hydrogène pendant 3 heures à 40°C et à pression atmosphérique un mélange de 9,34 g du composé obtenu à l'étape précédente, 1 g de palladium sur charbon à 5 % et 200 ml d'EtOH. On filtre le catalyseur et concentre sous vide le filtrat. On obtient 6,13 g du produit attendu après cristallisation dans le mélange EtOH/acétone.

### Préparation 2.9

### p-Toluènesulfonate de 4-benzyl-4-(pyrrolidin-1-ylcarbonylamino)pipéridine.

### A) 4-Cyanopipéridine.

On ajoute par petites portions 25 g d'isonipécotamide (ou pipéridine-4-carboxamide) à 70 ml de POCl₃ et chauffe à reflux pendant 4 heures. On concentre sous vide le mélange réactionnel, reprend le résidu dans de la glace, alcalinise à pH = 13 par ajout d'une solution de NaOH concentrée, extrait au DCM puis 4 fois à l'éther, sèche les phases organiques jointes sur MgSO₄ et évapore sous vide les solvants. On distille sous pression réduite l'huile obtenue. On obtient 6,4 g du produit attendu, Eb = 108-110°C sous 2400 Pa.

### B) 4-Cyano-1,4-dibenzylpipéridine.

On refroidit à -50°C une solution de 15 g du composé obtenu à l'étape précédente dans 250 ml de THF, ajoute goutte à goutte 190 ml d'une solution 1,5M de diisopropylamidure de lithium dans le cyclohexane et laisse 30 minutes sous agitation à -50°C. Puis on ajoute 34 ml de bromure de benzyle et laisse sous agitation en laissant remonter la température à TA. Après 3 heures à TA, on verse le mélange réactionnel sur un mélange de glace et d'HCl concentré, ajoute de l'éther, essore le précipité formé et le lave à l'eau. On reprend le précipité dans de l'eau, alcalinise à pH = 13 par ajout d'une solution de NaOH concentrée, extrait à l'éther, sèche la phase organique sur MgSO₄ et évapore sous vide le solvant. On obtient 31,7 g du produit attendu après cristallisation dans le pentane, F = 92°C.

### C) 1,4-Dibenzyl-4-carboxypipéridine.

A une solution de 25 ml d'eau, 25 ml d'H₂SO₄ concentré et 25 ml d'AcOH, on ajoute 6 g du composé obtenu à l'étape précédente et chauffe à 140°C pendant 5 heures. Après refroidissement, on verse le mélange réactionnel sur de la glace, on amène à pH = 6,5 par ajout d'une solution de NaOH concentrée-et laisse sous agitation jusqu'à cristallisation. On essore le produit cristallisé et le lave à l'eau. On reprend le produit dans du MeOH, l'essore et le lave à l'éther. On obtient 3 g du produit attendu, F = 262°C.

### D) 1,4-Dibenzyl-4-isocyanatopipéridine.

On chauffe à 60°C pendant 1 heure un mélange de 2 g du composé obtenu à l'étape précédente et 1,6 g de pentachlorure de phosphore dans 40 ml de chloroforme. On concentre sous vide le mélange réactionnel, reprend le résidu dans 40 ml d'acétone, ajoute une solution de 2 g d'azidure de sodium dans 5 ml d'eau et laisse 30 minutes sous agitation à TA. On concentre sous vide à TA, reprend le résidu à l'éther, lave la phase organique par une solution saturée de Na₂CO₃, à l'eau, sèche sur MgSO₄ et évapore sous vide le solvant. On reprend le résidu dans 40 ml de toluène et chauffe à reflux pendant 1 heure. On concentre sous vide et obtient 2 g du produit attendu sous forme d'huile.

### E) 1,4-Dibenzyl-4-(pyrrolidin-1-ylcarbonylamino)pipéridine.

A une solution de 5 g du composé obtenu à l'étape précédente dans 50 ml de DCM, on ajoute à TA 1,5 ml de pyrrolidine et laisse 2 heures sous agitation à TA. On concentre sous vide le mélange réactionnel, reprend le résidu au pentane, essore le précipité formé et le lave au pentane. On obtient 4,5 g du produit attendu après séchage, F = 126°C.

### F) p-Toluènesulfonate de 4-benzyl-4-(pyrrolidin-1-ylcarbonylamino)pipéridine.

On hydrogène pendant 48 heures à TA et à pression atmosphérique un mélange de 4,2 g du composé obtenu à l'étape précédente, 2,1 g d'acide *p*-toluènesulfonique monohydrate, 0,4 g de palladium sur charbon à 10 % et 50 ml d'EtOH. On filtre le catalyseur sur Célite® et concentre sous vide le filtrat. On reprend le résidu dans l'éther et essore le précipité formé. On obtient 4,99 g du produit attendu, F > 180°C.

### Préparation 2.10

### p-Toluènesulfonate de 4-(méthoxycarbonylamino)-4-phénylpipéridine, hémihydrate.

On chauffe à reflux pendant 5 heures une solution de 6,05 g du composé obtenu à l'étape A de la Préparation 2.4 dans 100 ml de MeOH. On ajoute 1 goutte de triéthylamine et laisse une nuit sous agitation à TA. Puis on ajoute jusqu'à pH = 1 une solution d'HCl concentrée et concentre sous vide le mélange réactionnel. On reprend le résidu par une solution de NaOH à 10 %, extrait au DCM, lave la phase organique par une solution de NaOH à 10 %, par une solution saturée de NaCl, sèche sur MgSO₄ et évapore sous vide le solvant. On dissout le produit obtenu dans l'acétone, ajoute goutte à goutte une solution de 3,57 g d'acide *p*-toluènesulfonique monohydrate dans 10 ml d'acétone et concentre sous vide. On reprend le produit obtenu dans l'éther et évapore sous vide le solvant. On obtient 7,17 g du produit attendu, F = 159°C.

### Préparation 2.11

### p-Toluènesulfonate de 4-(2-amino-1,3,4-oxadiazol-5-yl)-4-phénylpipéridine, hémihydrate.

### A) 1-(Benzyloxycarbonyl)-4-(chloroformyl)-4-phénylpipéridine.

On chauffe à reflux pendant 1 heure un mélange de 17,1 g du composé obtenu à l'étape A de la Préparation 2.5, 24 g de chlorure de thionyle dans 150 ml de 1,2-dichloroéthane. On concentre sous vide, reprend le résidu au chloroforme et évapore sous vide le solvant. On reprend le résidu dans un mélange éther/pentane et évapore à nouveau sous vide les solvants. On obtient 20 g du produit attendu sous forme de gomme que l'on utilise tel quel.

### B) 1-(Benzyloxycarbonyl)-4-carbazoyl-4-phénylpipéridine.

On refroidit à -50°C une solution de 16 g d'hydrazine monohydrate dans 40 ml d'EtOH, ajoute goutte à goutte une solution de 11,44 g du composé obtenu à l'étape précédente dans 20 ml de 1,2-diméthoxyéthane et laisse sous agitation en laissant remonter la température à TA. On concentre sous vide, reprend le résidu à l'eau, extrait au DCM, lave la phase organique à l'eau, par une solution saturée de NaCl, sèche sur MgSO₄ et évapore sous vide le solvant. On reprend le résidu par un mélange EtOH/benzène et évapore sous vide les solvants. On obtient 11,2 g du produit attendu sous forme de gomme que l'on utilise tel quel.

### C) 4-(2-Amino-1,3,4-oxadiazol-5-yl)-1-(benzyloxycarbonyl)-4-phénylpipéridine.

A une solution de 11,2 g du composé obtenu à l'étape précédente dans 60 ml d'EtOH, on ajoute à TA une solution de 3,39 g de bromure de cyanogène dans 10 ml d'EtOH et chauffe à reflux pendant 1 heure. On concentre le mélange réactionnel jusqu'à 50 ml d'EtOH, puis ajoute goutte à goutte de l'eau jusqu'à obtenir un volume de 400 ml de mélange réactionnel. On essore le produit cristallisé formé, le lave à l'eau puis au DCM, à l'AcOEt, et à l'éther. On obtient 8 g du produit attendu.

### D) p-Toluènesulfonate de 4-(2-amino-1,3,4-oxadiazol-5-yl)-4-phénylpipéridine hémihydrate.

On hydrogène à 50°C et à pression atmosphérique un mélange de 7,85 g du composé obtenu à l'étape précédente, 3,95 g d'acide *p*-toluènesulfonique monohydrate, 0,8 g de palladium sur charbon à 10 %, 350 ml d'EtOH 95 et 10 ml d'eau. Après 3 heures, on filtre le catalyseur sur Célite® et concentre sous vide le filtrat. On reprend le résidu à l'acétone, essore le produit cristallisé formé et le lave à l'acétone puis à l'éther. On obtient 7,65 g du produit attendu, F = 183-185°C.

### Préparation 2.12

### p-Toluènesulfonate de 4-[(acétyl-N-méthylamino)méthyl]-4-phénylpipéridine.

### A) 4-(aminométhyl)-1-benzyl-4-phénylpipéridine.

On refroidit à 0°C une suspension de 2,8 g d'hydrure d'aluminium et de lithium dans 50 ml de THF et ajoute, goutte à goutte, une solution de 20 g de 1-benzyl-4-cyano-4-phénylpipéridine dans 50 ml de THF. On laisse 1 heure sous agitation à TA puis chauffe à 40°C pendant 1 heure. On refroidit au bain de glace le mélange réactionnel, ajoute successivement 3 ml d'eau, 3 ml d'une solution de NaOH 4N et 12 ml d'eau. On filtre les sels minéraux et évapore sous vide le filtrat. On chromatographie le résidu sur silice H en éluant par le gradient du mélange DCM/MeOH de (100/3 ; v/v) à (100/10 ; v/v). On obtient 11 g du produit attendu.

### B) 1-Benzyl-4-[(N-formylamino)méthyl]-4-phénylpipéridine.

A un mélange de 11 g du composé obtenu à l'étape précédente dans 76 ml d'acide formique on ajoute, à TA et goutte à goutte, 25 ml d'anhydride acétique puis on laisse 5 heures sous agitation. On concentre sous vide le mélange réactionnel, reprend le résidu à l'eau, alcalinise à pH = 14 par ajout de NaOH concentré, extrait à l'éther, lave à l'eau, sèche sur Na₂SO₄ et évapore sous vide le solvant. On obtient 12 g du produit attendu.

### C) 1-Benzyl-4-[(N-méthylamino)méthyl]-4-phénylpipéridine.

On chauffe à 40°C une suspension de 3,9 g d'hydrure d'aluminium et de lithium dans 50 ml de THF, ajoute, goutte à goutte, une solution de 12 g du composé obtenu à l'étape précédente dans 50 ml de THF puis chauffe à reflux pendant 3 heures. Après refroidissement au bain de glace on ajoute successivement 4 ml d'eau, 4 ml d'une solution de NaOH 4N et 12 ml d'eau. On filtre les sels minéraux et concentre sous vide le filtrat. On extrait le résidu à l'éther, sèche sur Na₂SO₄ et évapore sous vide le solvant. On obtient 10 g du produit attendu.

### D) 4-[(Acétyl-N-méthylamino)méthyl]-1-benzyl-4-phénylpipéridine.

A une solution de 3,3 g du composé obtenu à l'étape précédente, 1,4 g de triéthylamine dans 50 ml de DCM on ajoute 0,863 g de chlorure d'acétyle et laisse 2 heures sous agitation à TA. On concentre sous vide le mélange réactionnel, reprend le résidu à l'eau, extrait à l'éther, lave à l'eau, sèche sur Na₂SO₄ et évapore le solvant. On chromatographie le résidu sur silice H en éluant par le mélange DCM/MeOH (100/3 ; v/v). On obtient 2,4 g du produit attendu.

### E) p-Toluènesulfonate de 4-[(acétyl-N-méthylamino)méthyl]-4-phénylpipéridine.

On hydrogène, à TA et à pression atmosphérique, un mélange de 2,3 g du composé obtenu à l'étape précédente, 1,2 g d'acide p-toluènesulfonique monohydrate, 0,23 g du palladium sur charbon à 10 % et 100 ml de MeOH. On filtre le catalyseur et évapore sous vide le filtrat. On obtient 2,7 g du produit attendu après trituration dans l'éther et essorage.

### Préparation 2.13

### p-Toluènesulfonate de 4-[(éthoxycarbonyl-N-méthylamino)méthyl]-4-phénylpipéridine.

### A) 1-Benzyl-4-[(éthoxycarbonyl-N-méthylamino)méthyl]-4-phénylpipéridine.

A une solution de 2,3 g du composé obtenu à l'étape C de la Préparation 2.12 et 1,03 g de triéthylamine dans 50 ml de DCM, on ajoute à TA et goutte à goutte une solution de 0,85 g de chloroformiate d'éthyle dans 10 ml de DCM et laisse 30 minutes sous agitation à TA. On concentre sous vide le mélange réactionnel, reprend le résidu à l'eau, extrait à l'éther, lave la phase organique à l'eau, par une solution saturée de NaCl, sèche sur Na₂SO₄ et évapore sous vide le solvant. On chromatographie le résidu sur silice H en éluant par le mélange DCM/MeOH (100/3 ; v/v). On obtient 2,3 g du produit attendu.

### B) p-Toluènesulfonate de 4-[(éthoxycarbonyl-N-méthylamino)méthyl]-4-phénylpipéridine.

On hydrogène à 40°C et à pression atmosphérique, un mélange de 2,3 g du composé obtenu à l'étape précédente, 1,19 g d'acide *p*-toluènesulfonique monohydrate, 0,7 g de palladium sur charbon à 5 % et 50 ml de DCM. On filtre le catalyseur sur Célite® et concentre sous vide le filtrat. On obtient 2,7 g du produit attendu.

### Préparation 2.14

### p-Toluènesulfonate de 4-[(N',N'-diméthyl-N-méthyluréido)méthyl]-4-phénylpipéridine.

### A) 1-Benzyl-4-[(N',N'-diméthyl-N-méthyluréido)méthyl]-4-phénylpipéridine.

A une solution de 2,5 g du composé obtenu à l'étape C de la Préparation 2.13 et 1,11 g de triéthylamine dans 50 ml de DCM, on ajoute à TA et goutte à goutte une solution de 0,92 g de chlorure de N,N-diméthylcarbamoyle dans 20 ml de DCM puis chauffe à reflux pendant 3 heures. On concentre sous vide le mélange réactionnel, reprend le résidu à l'eau, extrait à l'éther, lave la phase organique à l'eau, par une solution saturée de NaCl, sèche sur Na₂SO₄ et évapore sous vide le solvant. On chromatographie le résidu sur silice H en éluant par le mélange DCM/MeOH (100/3 ; v/v). On obtient 2,92 g du produit attendu.

### B) p-Toluènesulfonate de 4-[(N',N'-diméthyl-N-méthyluréido)méthyl]-4-phénylpipéridine.

On hydrogène à 40°C et à pression atmosphérique, un mélange de 2,92 g du composé obtenu à l'étape précédente, 1,52 g d'acide *p*-toluènesulfonique monohydrate, 0,3 g de palladium sur charbon à 10 % et 50 ml de MeOH. On filtre le catalyseur sur Célite® et concentre sous vide le filtrat. On obtient 2,6 g du produit attendu après trituration du résidu dans le mélange pentane/éther iso puis essorage.

### Préparation 2.15

### Benzènesulfonate de 4-phényl-4-uréidopipéridine.

### A) 1-(Benzyloxycarbonyl)-4-isocyanato-4-phénylpipéridine.

On chauffe à reflux pendant 1 heure un mélange de 50,89 g du composé obtenu à l'étape A) de la Préparation 2.5 et 71,4 g de chlorure de thionyle dans 400 ml de 1,2-dichloroéthane. On concentre sous vide le mélange réactionnel, reprend le résidu à l'acétone et évapore sous vide le solvant. On dissout le résidu dans 200 ml d'acétone, refroidit à 5°C, ajoute goutte à goutte une solution de 19,5 g d'azidure de sodium dans 50 ml d'eau et laisse 2 heures sous agitation à TA. On évapore sous vide à TA l'acétone, ajoute à la solution restante une solution saturée de NaHCO₃, extrait au toluène, lave la phase organique à l'eau, par une solution saturée de NaCl, sèche sur Na₂SO₄ et évapore 50 % en volume de solvant. On chauffe à reflux pendant 1 heure la solution toluènique restante puis concentre sous vide. On obtient 54 g du produit attendu sous forme d'huile orange qui cristallise.

### B) 1-(Benzyloxycarbonyl)-4-phényl-4-uréidopipéridine.

Dans une solution de 29 g du composé obtenu à l'étape précédente dans 300 ml d'éther et 300 ml de DCM, on fait barboter à TA et en excès de l'ammoniac gaz puis laisse une nuit sous agitation à TA. On concentre sous vide le mélange réactionnel, reprend le résidu dans de l'acétone chaud et laisse redescendre la température à TA. Dès que le produit cristallise on ajoute un mélange d'éther/AcOEt puis essore les cristaux formés. On obtient 26,4 g du produit attendu.

### C) Benzènesulfonate de 4-phényl-4-uréidopipéridine.

On hydrogène à 40°C et à pression atmosphérique un mélange de 25 g du composé obtenu à l'étape précédente, 11,2 g d'acide benzènesulfonique, 3 g de palladium sur charbon à 5 % et 300 ml d'EtOH. On dilue le mélange réactionnel par ajout d'eau et de MeOH, filtre le catalyseur sur Célite® et concentre sous vide le filtrat. On obtient 26,45 g du produit attendu après cristallisation dans l'acétone, F = 235°C.

### Préparation 2.16

### Benzènesulfonate de 4-(N'-méthyluréido)-4-phénylpipéridine.

### A) 1-(Benzyloxycarbonyl)-4-(N'-méthyluréido)-4-phénylpipéridine.

On refroidit à 5°C une solution de 25 g du composé obtenu à l'étape A de la Préparation 2.15 dans 300 ml d'éther et fait barboter en excès de la méthylamine gaz. On dilue le mélange réactionnel par ajout de 150 ml de DCM et laisse une nuit sous agitation à TA. On concentre sous vide, reprend le résidu dans de l'AcOEt chaud et laisse revenir à TA. On ajoute de l'éther jusqu'à précipitation et essore le précipité formé. On obtient 24 g du produit attendu.

### B) Benzènesulfonate de 4-(N'-méthyluréido)-4-phénylpipéridine.

On hydrogène à 40°C et à pression atmosphérique un mélange de 23 g du composé obtenu à l'étape précédente, 9,9 g d'acide benzènesulfonique, 3 g de palladium sur charbon à 5 % et 300 ml d'EtOH 95. On filtre le catalyseur sur Célite® et concentre sous vide le filtrat. On reprend le résidu à l'acétone et essore le précipité formé. On obtient 22,36 g du produit attendu, F = 227°C.

### Préparation 2.17

### p-Toluènesulfonate de 4-(3,3-diméthylcarbazoyl)-4-phénylpipéridine.

### A) p-Toluènesulfonate de 1-(benzyloxycarbonyl)-4-(3,3-diméthylcarbazoyl)-4-phénylpipéridine.

On refroidit à 5°C une solution de 7,15 g du composé obtenu à l'étape A de la Préparation 2.11 dans 60 ml de DCM, ajoute goutte à goutte une solution de 1,44 g de 1,1-diméthylhydrazine, 4,04 g de triéthylamine dans 20 ml de DCM et laisse une nuit sous agitation à TA. On concentre sous vide le mélange réactionnel, reprend le résidu à l'eau, extrait à l'AcOEt, lave la phase organique par une solution saturée de NaCl, sèche sur Na₂SO₄ et évapore sous vide le solvant. On dissout le produit obtenu dans 100 ml d'acétone, ajoute rapidement une solution de 3,59 g d'acide *p*-toluènesulfonique monohydrate dans 10 ml d'acétone et concentre sous vide. On reprend le résidu dans un mélange éther/DCM et essore le précipité formé. On obtient 9,65 g du produit attendu.

### B) p-Toluènesulfonate de 4-(3,3-diméthylcarbazoyl)-4-phénylpipéridine.

On hydrogène à 30°C et à pression atmosphérique un mélange de 9,5 g du composé obtenu à l'étape précédente, 0,9 g de palladium sur charbon à 10 % dans 100 ml d'EtOH 95. On filtre le catalyseur sur Célite ® et concentre sous vide le filtrat. On reprend le résidu dans 100 ml d'acétone, essore le produit cristallisé formé et le lave à l'éther. On obtient 7 g du produit attendu, F = 210-212°C.

### Préparation 2.18

### Chlorhydrate de 4-[(éthylaminocarbonyloxy)méthyl]-4-phénylpipéridine.

### A) p-Toluènesulfonate de 4-méthoxycarbonyl-4-phénylpipéridine.

A une solution de 10 g de *p*-toluènesulfonate de 4-carboxy-4-phénylpipéridine dans 300 ml de MeOH, on ajoute 1 g d'acide *p*-toluènesulfonique monohydrate et chauffe à reflux pendant 3 jours. On concentre sous vide le mélange réactionnel, reprend le résidu dans l'acétone et ajoute de l'éther jusqu'à précipitation. Après essorage du précipité formé, on obtient 9,34 g du produit attendu.

### B) 4-Hydroxyméthyl-4-phénylpipéridine.

On refroidit à -20°C une suspension de 1,16 g d'hydrure d'aluminium et de lithium dans 50 ml de THF, ajoute 4 g du composé obtenu à l'étape précédente et laisse sous agitation une nuit en laissant remonter la température à TA. On hydrolyse par ajout de 1,2 ml d'eau, puis 2,5 ml d'une solution à 10 % de NaOH et 2,5 ml d'eau. On dilue à l'éther, filtre les sels minéraux et évapore sous vide le filtrat. On obtient 1,8 g du produit attendu.

### C) 1-tert-Butoxycarbonyl-4-(hydroxyméthyl)-4-phénylpipéridine.

A une solution de 22,8 g du composé obtenu à l'étape précédente dans 250 ml de 1,2-diméthoxyéthane on ajoute 26,05 g de di-*tert*-butyldicarbonate et chauffe à reflux pendant 2 heures. On concentre sous vide le mélange réactionnel, reprend le résidu au DCM, lave la phase organique par une solution tampon pH = 2, par une solution saturée de NaCl, sèche sur MgSO₄ et évapore sous vide le solvant. On obtient 17,86 g du produit attendu après cristallisation dans l'éther, F = 134°C.

### D) 1-tert-Butoxycarbonyl-4-[(éthylaminocarbonyloxy)méthyl]-4-phénylpipéridine.

On laisse une nuit sous agitation à TA un mélange de 2,91 g du composé obtenu à l'étape précédente, 2,4 g d'isocyanate d'éthyle, 2 gouttes de triéthylamine dans 30 ml de toluène. Puis on chauffe à 100°C pendant 24 heures et concentre sous vide le mélange réactionnel. On reprend le résidu à l'éther, lave la phase organique par une solution tampon pH = 2, par une solution saturée de NaCl, sèche sur MgSO₄ et évapore sous vide le solvant. On obtient 3,85 g du produit attendu sous forme d'huile.

### E) Chlorhydrate de 4-[(éthylaminocarbonyloxy)méthyl]-4-phénylpipéridine.

A une solution de 3,85 g du composé obtenu à l'étape précédente dans 50 ml de MeOH, on ajoute 10 ml d'HCl concentré et chauffe à 60°C pendant 2 heures. On concentre sous vide, reprend le résidu à l'acétone et évapore sous vide le solvant. On obtient 2,6 g du produit attendu après cristallisation dans le mélange AcOEt/éther, F = 240-242°C.

### Préparation 2.19

### 4-(Acétyloxyméthyl)-4-phénylpipéridine.

A une solution de 2,91 g du composé obtenu à l'étape C de la Préparation 2.20 et 1,31 g de triéthylamine dans 50 ml de DCM, on ajoute à TA 0,785 g de chlorure d'acétyle et laisse 30 minutes sous agitation à TA. On concentre sous vide, reprend le résidu à l'eau, extrait à l'AcOEt, lave deux fois la phase organique à l'eau, par une solution saturée de NaCl, sèche sur MgSO₄ et évapore sous vide le solvant. On reprend le résidu huileux dans 20 ml d'acide trifluoroacétique et laisse 10 minutes sous agitation à TA. On concentre sous vide, reprend le résidu à l'eau, alcalinise la phase aqueuse à pH = 11 par ajout d'une solution concentrée de NaOH, extrait au DCM, lave 5 fois la phase organique à l'eau, par une solution saturée de NaCl, sèche sur MgSO₄ et évapore sous vide le solvant. On obtient 1,6 g du produit attendu.

### Préparation 2.20

### Benzènesulfonate de 4-benzyl-4-[(éthoxycarbonyl-N-méthylamino)méthyl] pipéridine.

### A) 1-Benzyl-4-carbamoylpipéridine.

A un mélange de 58,2 g d'isonipécotamide et 69 g de K₂CO₃ dans 275 ml de DMF, on ajoute à TA et goutte à goutte 85,5 g de bromure de benzyle et laisse 2 heures sous agitation à 50°C. On concentre sous vide le mélange réactionnel, reprend le résidu à l'eau, extrait au DCM, lave la phase organique à l'eau, sèche sur Na₂SO₄ et évapore sous vide le solvant. On obtient 62 g du produit attendu après cristallisation dans 300 ml d'eau et sèchage sous vide.

### B) 1-Benzyl-4-cyanopipéridine.

On chauffe à reflux pendant 2 heures un mélange de 62 g du composé obtenu à l'étape précédente et 200 ml de POCl₃. On concentre sous vide, reprend le résidu à l'eau, alcalinise à pH = 13 par ajout d'une solution concentrée de NaOH, extrait au DCM, lave la phase organique à l'eau, sèche sur Na₂SO₄ et évapore sous vide le solvant. On distille sous pression réduite l'huile obtenue. On obtient 52 g du produit attendu, Eb = 120°C sous 10 Pa.

### C) 1,4-Dibenzyl-4-cyanopipéridine.

On refroidit à -50°C 200 ml d'une solution 1,5M de diisopropylamidure de lithium dans le cyclohexane diluée dans 100 ml de THF, ajoute goutte à goutte une solution de 52 g du composé obtenu à l'étape précédente dans 100 ml de THF et laisse 30 minutes sous agitation à -50°C. Puis on ajoute goutte à goutte et à température comprise entre -30°C et -25°C une solution de 51,3 g de bromure de benzyle dans 100 ml de THF et laisse sous agitation en laissant remonter la température à TA. On concentre sous vide, reprend le résidu à l'eau, extrait à l'éther, lave la phase organique à l'eau, sèche sur Na₂SO₄ et évapore sous vide le solvant. On obtient 64 g du produit attendu après cristallisation dans le pentane.

### D) 4-(Aminométhyl)-1,4-dibenzylpipéridine.

A une suspension de 1,95 g d'hydrure d'aluminium et de lithium dans 50 ml de THF, on ajoute à TA et goutte à goutte une solution de 14,5 g du composé obtenu à l'étape précédente dans 50 ml de THF puis chauffe à reflux pendant 6 heures. Après refroidissement à TA on ajoute 2 ml d'eau, 2 ml d'une solution de NaOH 4N et 6 ml d'eau. On filtre les sels minéraux sur Célite®, décante le filtrat, sèche la phase organique sur Na₂SO₄ et évapore sous vide le solvant. On obtient 14,7 g du produit attendu.

### E) 1,4-Dibenzyl-4-[(formylamino)méthyl]pipéridine.

A une solution de 14,7 g du composé obtenu à l'étape précédente dans 126 ml d'acide formique on ajoute à TA et goutte à goutte 42 ml d'anhydride acétique et laisse 3 heures sous agitation à TA. On concentre sous vide, reprend le résidu à l'eau, alcalinise à pH = 13 par ajout d'une solution concentrée de NaOH, extrait à l'AcOEt, lave la phase organique à l'eau, sèche sur Na₂SO₄ et évapore sous vide le solvant. On obtient 14,5 g du produit attendu.

### F) 1,4-Dibenzyl-4-[(méthylamino)méthyl]pipéridine.

A une suspension de 5 g d'hydrure d'aluminium et de lithium dans 100 ml de THF, on ajoute à 40°C et goutte à goutte une solution de 14,5 g du composé obtenu à l'étape précédente dans 100 ml de THF puis chauffe à reflux pendant 4 heures. Après refroidissement à TA on ajoute 5 ml d'eau, 5 ml d'une solution de NaOH 4N et 15 ml d'eau. On filtre les sels minéraux sur Célite®, décante le filtrat, sèche la phase organique sur Na₂SO₄ et évapore sous vide le solvant. on obtient 12,8 g du produit attendu.

### G) 1,4-Dibenzyl-4-[(éthoxycarbonyl-N-méthylamino)méthyl]pipéridine.

A une solution de 3 g du composé obtenu à l'étape précédente et 1,18 g de triéthylamine dans 50 ml de 1,2-dichloroéthane, on ajoute à TA et goutte à goutte 1,26 g de chloroformiate d'éthyle et laisse 1 heure sous agitation à TA. On concentre sous vide, reprend le résidu à l'eau, extrait à l'éther, sèche la phase organique sur Na₂SO₄ et évapore sous vide le solvant. On chromatographie le résidu sur silice H en éluant par le mélange DCM/MeOH (100/3 ; v/v). On obtient 1,6 g du produit attendu.

### H) Benzènesulfonate de 4-benzyl-4-[(éthoxycarbonyl-N-méthylamino)méthyl] pipéridine.

On hydrogène à 27°C et à pression atmosphérique un mélange de 1,6 g du composé obtenu à l'étape précédente, 0,66 g d'acide benzènesulfonique, 0,2 g de palladium sur charbon à 10 % et 30 ml de MeOH. On filtre le catalyseur sur Célite® et concentre sous vide le filtrat. On obtient 1,44 g du produit attendu.

### Préparation 2.21

### Benzènesulfonate de 4-benzyl-4-[(méthanesulfonyl-N-méthylamino)méthyl] pipéridine.

### A) 1,4-Dibenzyl-4-[(méthanesulfonyl-N-méthylamino)méthyl]pipéridine.

A une solution de 3,2 g du composé obtenu à l'étape F de la Préparation 2.20 et 1,26 g de triéthylamine dans 50 ml de 1,2-dichloroéthane, on ajoute à TA et goutte à goutte 1,26 g de chlorure de méthanesulfonyle et laisse 1 heure sous agitation à TA. On concentre sous vide, reprend le résidu à l'eau, extrait à l'AcOEt, sèche la phase organique sur Na₂SO₄ et évapore sous vide le solvant. On obtient 3,8 g du produit attendu.

### B) Benzènesulfonate de 4-benzyl-4-[(méthanesulfonyl-N-méthylamino)méthyl] pipéridine.

On hydrogène à TA et à pression atmosphérique un mélange de 3,8 g du composé obtenu à l'étape précédente, 1,58 g d'acide benzènesulfonique, 0,8 g de palladium sur charbon à 10 % et 30 ml de MeOH. On filtre le catalyseur sur Célite® et concentre sous vide le filtrat. On obtient 4,6 g du produit attendu, après cristallisation dans le mélange DCM/éther.

### Préparation 2.22

### Chlorhydrate de 4-benzyl-4-[(N',N'-diméthyl-N-méthyluréido)méthyl]pipéridine.

### A) 1,4-Dibenzyl-4-[(N',N'-diméthyl-N-méthyluréido)méthyl]pipéridine.

A une solution de 3,2 g du composé obtenu à l'étape F de la Préparation 2.20 et 1,2 g de triéthylamine dans 40 ml de 1,2-dichloroéthane, on ajoute à TA et goutte à goutte 1,12 g de chlorure de N,N-diméthylcarbamoyle et chauffe à reflux pendant 4 heures. On concentre sous vide, reprend le résidu à l'eau, extrait à l'éther, sèche la phase organique sur Na₂SO₄ et évapore sous vide le solvant. On obtient 3,6 g du produit attendu.

### B) Chlorhydrate de 4-benzyl-4-[(N',N'-diméthyl-N-méthyluréido)méthyl]pipéridine.

On laisse 1 heure 30 minutes sous agitation un mélange de 3,6 g du composé obtenu à l'étape précédente, 3,1 g de formiate d'ammonium et 0,8 g de palladium sur charbon à 5 % dans 50 ml de MeOH. On filtre le catalyseur et évapore sous vide le solvant. On dissout le résidu dans du DCM, ajoute une solution saturée d'acide chlorhydrique dans l'éther jusqu'à pH = 1 et évapore sous vide les solvants. On obtient 3,2 g du produit attendu.

### EXEMPLE 1

### Chlorhydrate de 3-benzyl-5-[2-(4-benzylpipérid-1-yl)éthyl]-5-(3,4-dichlorophényl)-tétrahydro-2H-1,3-oxazin-2-one, monohydrate.

### A) 3-Benzyl-5-(3,4-dichlorophényl)-5-[2-(tétrahydropyran-2-yloxy)éthyl]tétrahydro-2H-1,3-oxazin-2-one.

On refroidit à +5°C une solution de 4 g du composé obtenu à la Préparation 1.1 dans 60 ml de THF, ajoute 1,37 g de tert-butylate de potassium puis, goutte à goutte, une solution de 1,92 g de bromure de benzyle dans 15 ml de THF. On laisse sous agitation en laissant remonter la température à TA et concentre sous vide le mélange réactionnel. On extrait le résidu à l'AcOEt, lave trois fois la phase organique à l'eau, deux fois par une solution saturée de NaCl, sèche sur MgSO₄ et évapore sous vide le solvant. On obtient 4,2 g du produit attendu que l'on utilise tel quel.

### B) 3-Benzyl-5-(3,4-dichlorophényl)-5-(2-hydroxyéthyl)-tétrahydro-2H-1,3-oxazin-2-one, 0,25 hydrate.

A une solution de 4,2 g du composé obtenu à l'étape précédente dans 40 ml de MeOH, on ajoute jusqu'à pH = 1 une solution saturée d'HCl gaz dans l'éther et laisse 48 heures sous agitation à TA. On concentre sous vide le mélange réactionnel, reprend le résidu par une solution saturée d'HCl gaz dans le MeOH et évapore sous vide le solvant. On reprend le résidu à l'éther et essore le précipité formé. On obtient 3,44 g du produit attendu, F = 143°C.

### C) 3-Benzyl-5-(3,4-dichlorophényl)-5-[2-(méthanesulfonyloxy)éthyl]tétrahydro-2H-1,3-oxazin-2-one.

On refroidit à +5°C une solution de 1,4 g du composé obtenu à l'étape précédente, 0,45 g de triéthylamine dans 50 ml de DCM, ajoute goutte à goutte une solution de 0,46 g de chlorure de méthanesulfonyle dans 5 ml de DCM. Après l'addition, on concentre sous vide le mélange réactionnel, reprend le résidu à l'eau, extrait à l'AcOEt, lave deux fois la phase organique à l'eau, par une solution saturée de NaCl, sèche sur MgSO₄ et évapore sous vide le solvant. On obtient 1,7 g du produit attendu.

Spectre de RMN du proton à 200 MHz dans DMSO-d₆
δ : 2,03 ppm : t : 2H
2,98 ppm : s : 3H
3,78 ppm : système AB : 2H
3,83 ppm : t : 2H
4,1 à 4,8 ppm : m : 4H
7,0 à 7,6 ppm : m : 8H

### D) Chlorhydrate de 3-benzyl-5-[2-(4-benzylpipérid-1-yl)éthyl]-5-(3,4-dichloro phényl)-tétrahydro-2H-1,3-oxazin-2-one, monohydrate.

On chauffe à 60°C pendant 2 heures 30 minutes un mélange de 1,6 g de 4-benzylpipéridine, 1,7 g du composé obtenu à l'étape précédente, 0,6 g d'iodure de potassium dans 10 ml de DMF. Après refroidissement à TA, on verse le mélange réactionnel dans l'eau, extrait à l'AcOEt, lave deux fois la phase organique à l'eau, par une solution saturée de NaCl, sèche sur MgSO₄ et évapore sous vide le solvant. On chromatographie le résidu sur silice H en éluant au DCM. On reprend le produit obtenu par une solution saturée d'HCl gaz dans l'éther et essore le précipité formé. On obtient 0,9 g du produit attendu, F = 122°C.

### EXEMPLE 2

### Chlorhydrate de 3-benzyl-5-(3,4-dichlorophényl)-5-[2-(4-hydroxy-4-phényl pipérid-1-yl)éthyl]-tétrahydro-2H-1,3-oxazin-2-one.

On prépare ce composé selon le mode opératoire décrit à l'étape D de l'EXEMPLE 1 à partir de 1,32 g de 4-hydroxy-4-phénylpipéridine, 1,55 g du composé obtenu à l'étape C de l'EXEMPLE 1, 0,56 g d'iodure de potassium dans 10 ml de DMF. On chromatographie sur silice H en éluant au DCM puis par le mélange DCM/MeOH (96/4 ; v/v). On dissout le produit obtenu dans du DCM, acidifie à pH = 1 par ajout d'une solution saturée d'HCl gaz dans l'éther et évapore sous vide les solvants. On obtient 0,72 g du composé attendu après cristallisation dans l'éther, F = 172°C.

### EXEMPLE 3

### Chlorhydrate de 5-[2-(4-acétamido-4-phénylpipérid-1-yl)éthyl]-3-benzyl-5-(3,4-dichlorophényl)-tétrahydro-2H-1,3-oxazin-2-one, 1,5 hydrate.

On prépare ce composé selon le mode opératoire décrit à l'étape D de l'EXEMPLE 1 à partir de 2,77 g de 4-acétamido-4-phénylpipéridine, 1,7 g du composé obtenu à l'étape C de l'EXEMPLE 1, 0,61 g d'iodure de potassium dans 10 ml de DMF. On chromatographie sur silice H en éluant au DCM puis par le mélange DCM/MeOH (97/3 ; v/v). On reprend le résidu par une solution saturée d'HCl gaz dans l'éther et évapore sous vide le solvant. On obtient 0,6 g du produit attendu après cristallisation dans l'AcOEt, F = 168°C.

### EXEMPLE 4

### Chlorhydrate de 3-[3,5-bis(trifluorométhyl)benzyl]-5-(3,4-dichlorophényl)-5-[2-(4-phénylpipérid-1-yl)éthyl]-tétrahydro-2H-1,3-oxazin-2-one, hémihydrate.

### A) 3-[3,5-Bis(trifluorométhyl)benzyl]-5-(3,4-dichlorophényl)-5-[2-(tétrahydropyran-2-yloxy)éthyl]-tétrahydro-2H-1,3-oxazin-2-one.

On refroidit à +5°C une solution de 3,7 g du composé obtenu à la Préparation 1.1 dans 60 ml de THF, ajoute 1,27 g de *tert*-butylate de potassium puis goutte à goutte une solution de 2,72 g de chlorure de 3,5-bis(trifluorométhyl)benzyle dans 15 ml de THF. On laisse sous agitation en laissant remonter la température à TA et concentre sous vide le mélange réactionnel. On reprend le résidu à l'eau, extrait à l'AcOEt, lave la phase organique à l'eau, par une solution saturée de NaCl, sèche sur MgSO₄ et évapore sous vide le solvant. On obtient 5,9 g du produit attendu que l'on utilise tel quel.

### B) 3-[3,5-Bis(trifluorométhyl)benzyl]-5-(3,4-dichlorophényl)-5-(2-hydroxyéthyl)-tétrahydro-2H-1,3-oxazin-2-one.

A une solution de 5,9 g du composé obtenu à l'étape précédente dans 50 ml de MeOH, on ajoute jusqu'à pH = 1 une solution saturée d'HCl gaz dans l'éther et laisse 15 minutes sous agitation à TA. On concentre sous vide le mélange réactionnel, reprend deux fois le résidu par une solution saturée d'HCl gaz dans le MeOH et évapore sous vide le solvant. On reprend le résidu à l'hexane et essore le précipité formé. On obtient 3,9 g du produit attendu.

Spectre de RMN du proton à 200 MHz dans DMSO-d₆
δ : 1,9 ppm : t : 2H
2,9 à 3,6 ppm : m : 4H
3,8 ppm : système AB : 2H
4,35 ppm : s : 2H
5,45 ppm : se : 1H
7,1 à 7,8 ppm : m : 3H
8,0 à 8,5 ppm : m : 3H
8,9 ppm : se : 1H

### C) 3-[3,5-Bis(trifluorométhyl)benzyl]-5-(3,4-dichlorophényl)-5-[2-(méthanesulfonyl oxy)éthyl]-tétrahydro-2H-1,3-oxazin-2-one.

On refroidit à +5°C une solution de 3 g du composé obtenu à l'étape précédente, 0,7 g de triéthylamine dans 50 ml de DCM, ajoute goutte à goutte une solution de 0,66 g de chlorure de méthanesulfonyle dans 6 ml de DCM et laisse sous agitation en laissant remonter la température à TA. On concentre sous vide le mélange réactionnel, reprend le résidu à l'eau, extrait à l'AcOEt, lave la phase organique à l'eau, par une solution d'HCl 2N, à l'eau, par une solution à 5 % de NaHCO₃, à l'eau, par une solution saturée de NaCl, sèche sur MgSO₄ et évapore sous vide le solvant. On obtient 3,4 g du produit attendu.

### D) Chlorhydrate de 3-[3,5-bis(trifluorométhyl)benzyl]-5-(3,4-dichlorophényl)-5-[2-(4-phénylpipérid-1-yl)éthyl]-tétrahydro-2H-1,3-oxazin-2-one, hémihydrate.

On chauffe à 60°C pendant 3 heures un mélange de 0,32 g de 4-phénylpipéridine, 1 g du composé obtenu à l'étape précédente, 0,28 g d'iodure de potassium dans 5 ml de DMF. Après refroidissement on verse le mélange réactionnel dans l'eau, extrait à l'AcOEt, lave la phase organique à l'eau, par une solution saturée de NaCl, sèche sur MgSO₄ et évapore sous vide le solvant. On chromatographie le résidu sur silice H en éluant au DCM puis par le mélange DCM/MeOH (98/2 ; v/v). On reprend le produit obtenu par une solution saturée d'HCl gaz dans l'éther et essore le précipité formé. On obtient 0,22 g du produit attendu, F = 174°C.

### EXEMPLE 5

### Chlorhydrate de 5-[2-(4-benzylpipérid-1-yl)éthyl]-3-[3,5-bis(trifluorométhyl) benzyl]-5-(3,4-dichlorophényl)-tétrahydro-2H-1,3-oxazin-2-one, 1,5 hydrate.

On prépare ce composé selon le mode opératoire décrit à l'étape D de l'EXEMPLE 4 à partir de 0,3 g de 4-benzylpipéridine, 0,8 g du composé obtenu à l'étape C de l'EXEMPLE 4, 0,24 g d'iodure de potassium et 5 ml de DMF. On chromatographie sur silice H en éluant au DCM puis par le mélange DCM/MeOH (96/4 ; v/v). On reprend le produit obtenu par une solution saturée d'HCl gaz dans l'éther et essore le précipité formé. On obtient 0,13 g du produit attendu, F = 124°C.

### EXEMPLE 6

### Chlorhydrate de 4-benzyl-6-(3,4-dichlorophényl)-6-[2-(4-hydroxy-4-phényl pipérid-1-yl)éthyl]morpholin-3-one, hémihydrate.

### A) 4-Benzyl-6-(3,4-dichlorophényl)-6-(2-hydroxyéthyl)morpholin-3-one.

A une solution de 1,7 g du composé obtenu à la Préparation 1.2 dans 30 ml de THF on ajoute à TA 0,51 g de *tert*-butylate de potassium puis lentement 0,77 g de bromure de benzyle et chauffe 2 heures à 50°C. On concentre sous vide le mélange réactionnel, reprend le résidu à l'eau, extrait à l'éther, lave la phase organique à l'eau, sèche sur Na₂SO₄ et évapore sous vide le solvant. On chromatographie le résidu sur silice H en éluant par le mélange DCM/MeOH (100/0,5 ; v/v). On dissout le produit obtenu (1 g) dans 10 ml de MeOH, acidifie à pH = 1 par ajout d'une solution saturée d'HCl gaz dans l'éther et évapore sous vide les solvants. On extrait le résidu à l'éther, lave la phase organique à l'eau, sèche sur Na₂SO₄ et évapore sous vide le solvant. On obtient 0,65 g du produit attendu.

Spectre de RMN du proton à 200 MHz dans DMSO-d₆
δ : 1,9 ppm : mt : 2H
2,7 à 3,3 ppm : mt : 2H
3,65 ppm : système AB : 2H
4,0 ppm : système AB : 2H
4,3 ppm : t : 1H
4,45 ppm : système AB : 2H
7,0 à 7,6 : m : 11H

On peut également obtenir ce composé en utilisant le procédé décrit ci-après.

### A') 4-Benzyl-6-(3,4-dichlorophényl)-6-(2-hydroxyéthyl)morpholin-3-one.

On laisse deux heures sous agitation à TA un mélange de 6 g du composé obtenu à la Préparation 1.3, 1,87 g de *tert*-butylate de potassium dans 100 ml de THF, puis ajoute lentement 2,85 g de bromure de benzyle et chauffe à reflux pendant 3 heures. On concentre sous, vide le mélange réactionnel, reprend le résidu par une solution tampon pH = 2, extrait à l'éther, lave la phase organique à l'eau, sèche sur Na₂SO₄ et évapore sous vide le solvant. On dissout le produit obtenu dans du MeOH, ajoute 1,3 g d'hydroxyde de lithium monohydrate et laisse 30 minutes sous agitation à TA. On concentre sous vide le mélange réactionnel, extrait le résidu à l'éther, lave la phase organique à l'eau, sèche sur Na₂SO₄ et évapore sous vide le solvant. On chromatographie le résidu sur silice H en éluant par le mélange DCM/MeOH (100/1 ; v/v). On obtient 4,6 g du produit attendu.

### B) 4-Benzyl-6-(3,4-dichlorophényl)-6-[2-(méthanesulfonyloxy)éthyl]morpholin-3-one.

On refroidit à 0°C une solution de 0,65 g du composé obtenu à l'étape précédente, 0,34 g de triéthylamine dans 30 ml de DCM et ajoute 0,25 g de chlorure de méthanesulfonyle. Après l'addition, on concentre sous vide le mélange réactionnel, extrait le résidu à l'AcOEt, lave la phase organique à l'eau, sèche sur Na₂SO₄ et évapore sous vide le solvant. On obtient 0,71 g du produit attendu que l'on utilise tel quel.

### C) Chlorhydrate de 4-benzyl-6-(3,4-dichlorophényl)-6-[2-(4-hydroxy-4-phényl pipérid-1-yl)éthyl]morpholin-3-one, hémihydrate.

On chauffe à reflux pendant 4 heures un mélange de 0,7 g du composé obtenu à l'étape précédente, 0,378 g de 4-hydroxy-4-phénylpipéridine, 0,8 g de K₂CO₃ dans 5 ml d'acétonitrile. Après refroidissement à TA, on verse le mélange réactionnel dans l'eau, extrait à l'AcOEt, lave la phase organique à l'eau, sèche sur Na₂SO₄ et évapore sous vide le solvant. On chromatographie le résidu sur silice H en éluant par le mélange DCM/MeOH (100/3 ; v/v). On dissout le produit obtenu dans un minimum de DCM, acidifie à pH = 1 par ajout d'une solution saturée d'HCl gaz dans l'éther et essore le précipité formé. On obtient 0,43 g du produit attendu, F = 160-162°C.

### EXEMPLE 7

Chlorhydrate de 3-benzyl-5-(3,4-dichlorophényl)-5-[2-(4-hydroxy-4-phényl pipérid-1-yl)éthyl]oxazolidin-2-one.

### A) 5-[2-(Benzoyloxy)éthyl]-3-benzyl-5-(3,4-dichlorophényl)oxazolidin-2-one.

A une solution de 1,5 g du composé obtenu à la Préparation 1.4 dans 30 ml de THF on ajoute à TA 0,44 g de *tert*-butylate de potassium et laisse 1 heure sous agitation à TA. On ajoute ensuite goutte à goutte 0,667 g de bromure de benzyle et chauffe à reflux pendant 3 heures. On concentre sous vide le mélange réactionnel, extrait le résidu à l'éther, lave la phase organique par une solution tampon pH = 2, à l'eau, sèche sur Na₂SO₄ et évapore sous vide le solvant. On chromatographie le résidu sur silice H en éluant au DCM puis par le mélange DCM/MeOH (99,5/0,5 ; v/v). On obtient 0,9 g du produit attendu.

Spectre de RMN du proton à 200 MHz dans DMSO-d₆
δ : 2,6 ppm : mt : 2H
3,5 à 4,0 ppm : système AB : 2H
4,2 à 4,7 ppm : m : 4H
7,2 à 8,0 ppm : m : 13H

### B) 3-Benzyl-5-(3,4-dichlorophényl)-5-(2-hydroxyéthyl)oxazolidin-2-one.

On laisse 30 minutes sous agitation à TA un mélange de 0,9 g du composé obtenu à l'étape précédente, 0,5 ml d'une solution de NaOH concentrée dans 30 ml de MeOH et 15 ml de DCM. On concentre sous vide le mélange réactionnel, extrait le résidu à l'éther, lave la phase organique à l'eau, sèche sur Na₂SO₄ et évapore sous vide le solvant. On obtient 0,7 g du produit attendu que l'on utilise tel quel.

### C) 3-Benzyl-5-(3,4-dichlorophényl)-5-[2-(méthanesulfonyloxy)éthyl]oxazolidin-2-one.

A une solution de 0,7 g du composé obtenu à l'étape précédente, 0,21 g de triéthylamine dans 20 ml de DCM, on ajoute à TA 0,24 g de chlorure de méthane sulfonyle et laisse 1 heure sous agitation à TA. On concentre sous vide le mélange réactionnel, extrait le résidu à l'AcOEt, lave la phase organique à l'eau, sèche sur Na₂SO₄ et évapore sous vide le solvant. On obtient 0,76 g du produit attendu que l'on utilise tel quel.

### D) Chlorhydrate de 3-benzyl-5-(3,4-dichlorophényl)-5-[2-(4-hydroxy-4-phényl pipérid-1-yl)éthyl]oxazolidin-2-one.

On chauffe à 50-60°C pendant 3 heures un mélange de 0,76 g de 4-hydroxy-4-phénylpipéridine, 0,282 g d'iodure de potassium dans 2 ml de DMF. Après refroidissement à TA, on verse le mélange réactionnel dans l'eau, extrait à l'AcOEt, lave la phase organique à l'eau, sèche sur Na₂SO₄ et évapore sous vide le solvant. On chromatographie le résidu sur silice H en éluant par le gradient du mélange DCM/MeOH de (100/2 ; v/v) à (100/3 ; v/v). On dissout le produit obtenu dans un minimum de DCM, acidifie à pH = 1 par ajout d'une solution saturée d'HCl gaz dans l'éther et essore le précipité formé. On obtient 0,36 g du produit attendu, F = 223-225°C.

### EXEMPLE 8 (ne concerne pas un composé de l'invention)

### Chlorure de 5-(3,4-dichlorophényl)-5-[2-[4-phényl-1-azoniabicyclo[2.2.2] oct-1-yl]éthyl]-3-[3,5-bis(trifluorométhyl)benzyl]oxazolidin-2-one, dihydrate.

### A) 5-(3,4-dichlorophényl)-5-(2-hydroxyéthyl)-3-[3,5-bis(trifluorométhyl)benzyl] oxazolidin-2-one.

On laisse 30 minutes sous agitation à TA un mélange de 1,4 g du composé obtenu à la Préparation 1.4, 0,45 g de *tert*-butylate de potassium dans 30 ml de THF, ajoute 1,05 g de chlorure de 3,5-bis(trifluorométhyl)benzyle et chauffe à reflux pendant 5 heures. On concentre sous vide, extrait le résidu à l'éther, lave la phase organique à l'eau, sèche sur MgSO₄ et évapore sous vide le solvant. On reprend le produit obtenu dans 30 ml de MeOH, ajoute 0,18 g d'hydroxyde de lithium monohydrate et 1 ml d'eau et laisse 2 heures sous agitation à TA. On concentre sous vide, extrait le résidu à l'éther, lave la phase organique à l'eau, sèche sur MgSO₄ et évapore sous vide le solvant. On chromatographie le résidu sur silice H en éluant par le mélange DCM/MeOH (100/0,5 ; v/v). On obtient 0,96 g du produit attendu.

Spectre de RMN du proton à 200 MHz dans DMSO-d₆
δ : 2,2 ppm : mt : 2H
3,0 à 3,55 ppm : mt : 2H
3,57 à 3,9 ppm : système AB : 2H
4,35 à 4,7 ppm : m : 3H
7,2 à 8,1 ppm : m : 6H

### B) 5-(3,4-dichlorophényl)-5-[2-(méthanesulfonyloxy)éthyl]-3-[3,5-bis(trifluoro méthyl)benzyl]oxazolidin-2-one.

A une solution de 0,95 g du composé obtenu à l'étape précédente, 0,21 g de triéthylamine dans 20 ml de DCM, on ajoute à TA 0,24 g de chlorure de méthane sulfonyle et laisse 1 heure sous agitation à TA. On concentre sous vide le mélange réactionnel, extrait le résidu à l'AcOEt, lave la phase organique à l'eau, sèche sur Na₂SO₄ et évapore sous vide le solvant. On obtient 1,1 g du produit attendu que l'on utilise tel quel.

### C) Chlorure de 5-(3,4-dichlorophényl)-5-[2-[4-phényl-1-azoniabicyclo[2.2.2] oct-1-yl]éthyl]-3-[3,5-bis(trifluorométhyl)benzyl]oxazolidin-2-one, dihydrate.

On chauffe à 60°C pendant 5 heures un mélange de 0,46 g de 4-phényl-1-azabicyclo[2.2.2]octane, 1,1 g du composé obtenu à l'étape précédente dans 2 ml de DMF. Après refroidissement à TA, on verse le mélange réactionnel dans l'eau, extrait au DCM, lave la phase organique à l'eau, par une solution saturée de NaCl, par une solution d'HCl 2N, à l'eau, sèche sur MgSO₄ et évapore sous vide le solvant. On obtient 0,6 g du produit attendu après cristallisation dans le mélange DCM/pentane, F = 127-130°C (déc).

### EXEMPLE 9

### Chlorhydrate de 6-[2-(4-acétamido-4-phénylpipérid-1-yl)éthyl]-4-benzyl-6-(3,4-difluorophényl)morpholin-3-one, hémihydrate.

### A) 6-[2-(Benzoyloxy)éthyl]-4-benzyl-6-(3,4-difluorophényl)morpholin-3-one.

On laisse 30 minutes sous agitation à TA un mélange de 1,1 g du composé obtenu à la Préparation 1.5, 0,38 g de *tert*-butylate de potassium dans 50 ml de THF, ajoute 0,51 g de bromure de benzyle et chauffe à reflux pendant 2 heures. On concentre sous vide le mélange réactionnel, extrait le résidu à l'éther, lave la phase organique à l'eau, par une solution tampon pH = 2, sèche sur Na₂SO₄ et évapore sous vide le solvant. On chromatographie le résidu sur silice H en éluant au DCM puis par le mélange DCM/MeOH (100/2 ; v/v). On obtient 0,6 g du produit attendu.

Spectre de RMN du proton à 200 MHz dans DMSO-d₆
δ : 2,3 ppm : mt : 2H
3,75 ppm : système AB : 2H
3,9 à 4,4 ppm : m : 4H
4,5 ppm : s : 2H
7,0 à 8,0 ppm : m : 13H

### B) 4-Benzyl-6-(3,4-difluorophényl)-6-(2-hydroxyéthyl)morpholin-3-one.

On laisse 30 minutes sous agitation à TA un mélange de 0,6 g du composé obtenu à l'étape précédente, 0,5 ml d'une solution concentrée de NaOH dans 10 ml de MeOH. On concentre sous vide le mélange réactionnel, extrait le résidu à l'AcOEt, lave la phase organique à l'eau, sèche sur Na₂SO₄ et évapore sous vide le solvant. On obtient 0,42 g du produit attendu que l'on utilise tel quel.

### C) 4-Benzyl-6-(3,4-difluorophényl)-6-[2-(méthanesulfonyloxy)éthyl]morpholin-3-one.

A une solution de 0,42 g du composé obtenu à l'étape précédente, 0,146 g de triéthylamine dans 20 ml de DCM, on ajoute à TA 0,137 g de chlorure de méthanesulfonyle et laisse 30 minutes sous agitation à TA. On concentre sous vide, extrait le résidu à l'éther, lave la phase organique à l'eau, sèche sur Na₂SO₄ et évapore sous vide le solvant. On obtient 0,49 g du produit attendu que l'on utilise tel quel.

### D) Chlorhydrate de 6-[2-(4-acétamido-4-phénylpipérid-1-yl)éthyl]-4-benzyl-6-(3,4-difluorophényl)morpholin-3-one, hémihydrate.

On chauffe à 80°C pendant 2 heures un mélange de 0,49 g du composé obtenu à l'étape précédente, 0,94 g de *p*-toluènesulfonate de 4-acétamido-4-phénylpipéridine, 0,48 g de K₂CO₃ dans 1 ml de DMF. Après refroidissement à TA, on verse le mélange réactionnel dans l'eau, extrait à l'AcOEt, lave la phase organique à l'eau, sèche sur Na₂SO₄ et évapore sous vide le solvant. On chromatographie le résidu sur silice H en éluant par le mélange DCM/MeOH (100/3 ; v/v). On dissout le produit obtenu dans un mininum de DCM, acidifie à pH = 1 par ajout d'une solution saturée d'HCl gaz dans l'éther et essore le précipité formé. On obtient 0,5 g du produit attendu, F = 273-275°C.

### EXEMPLE 10

### Chlorhydrate de 4-benzyl-6-(3,4-dichlorophényl)-6-[2-[4-phényl-4-(pyrrolidin-1-ylcarbonyl)pipérid-1-yl]éthyl]morpholin-3-one, monohydrate.

On chauffe à 80°C pendant 2 heures un mélange de 0,98 g du composé obtenu à l'étape B de l'EXEMPLE 6, 0,645 g de 4-phényl-4-(pyrrolidin-1-ylcarbonyl)pipéridine, 0,69 g de K₂CO₃ dans 3 ml de DMF. Après refroidissement à TA, on verse le mélange réactionnel dans l'eau, extrait par le mélange AcOEt/éther, lave la phase organique à l'eau, sèche sur MgSO₄ et évapore sous vide le solvant. On chromatographie le résidu sur silice H en éluant par le mélange DCM/MeOH (100/2 ; v/v). On dissout le produit obtenu dans un minimum de DCM, acidifie à pH = 1 par ajout d'une solution saturée d'HCl gaz dans l'éther et essore le précipité formé. On obtient 0,42 g du produit attendu, F = 155-178°C.

### EXEMPLE 11

### Chlorhydrate de 6-[2-[4-(acétyl-N-méthylamino)-4-phénylpipérid-1-yl]éthyl] -4-benzyl-6-(3,4-dichlorophényl)morpholin-3-one, monohydrate.

On chauffe à 80-100°C pendant 2 heures un mélange de 1,1 g du composé obtenu à l'étape B de l'EXEMPLE 6, 1,85 g de *p*-toluènesulfonate de 4-(acétyl-N-méthylamino)-4-phénylpipéridine, 1,3 g de K₂CO₃ dans 3 ml de DMF. Après refroidissement à TA, on verse le mélange réactionnel dans l'eau, extrait à l'éther, lave la phase organique à l'eau, sèche sur MgSO₄ et évapore sous vide le solvant. On chromatographie le résidu sur silice H en éluant par le mélange DCM/MeOH de (100/1; v/v) à (100/4 ; v/v). On dissout le produit obtenu dans du DCM, ajoute jusqu'à pH = 1 une solution saturée d'acide chlorhydrique dans l'éther et essore le précipité formé. On obtient 0,62 g du produit attendu, F = 148-150°C.

### EXEMPLE 12

### Chlorhydrate de 4-benzyl-6-[2-[4-(éthoxycarbonylamino)-4-phénylpipérid-1-yl] éthyl]-6-(3,4-dichlorophényl)morpholin-3-one, monohydrate.

On chauffe à 80-100°C pendant 2 heures un mélange de 1,1 g du composé obtenu à l'étape B de l'EXEMPLE 6, 1 g de trifluoroacétate de 4-(éthoxycarbonylamino)-4-phénylpipéridine, 0,7 g de K₂CO₃ dans 3 ml de DMF. Après refroidissement à TA, on verse le mélange réactionnel dans l'eau, laisse 30 minutes sous agitation, essore le précipité formé, le lave à l'eau, et le sèche sous vide à 60°C. On chromatographie le précipité sur silice H en éluant par le mélange DCM/MeOH de (100/1 ; v/v) à (100/4,5 ; v/v). On dissout le produit obtenu dans du DCM, ajoute jusqu'à pH = 1 une solution saturée d'acide chlorhydrique dans l'éther et essore le précipité formé. On obtient 0,52 g du produit attendu, F = 148-150°C.

### EXEMPLE 13

### Chlorhydrate de 5-(3,4-dichlorophényl)-5-[2-(4-phénylpipérid-1-yl)éthyl]-3-[3,5-bis(trifluorométhyl)benzyl]oxazolidin-2-one.

On chauffe à 80°C pendant 3 heures un mélange de 1,5 g du composé obtenu à l'étape B de l'EXEMPLE 8, 1,03 g de 4-phénylpipéridine dans 3 ml de DMF. Après refroidissement, on verse le mélange réactionnel dans l'eau, extrait à l'AcOEt, lave la phase organique à l'eau, sèche sur MgSO₄ et évapore sous vide le solvant. On chromatographie le résidu sur silice H en éluant par le mélange DCM/MeOH (100/1 ; v/v). On dissout le produit obtenu dans du DCM, ajoute jusqu'à pH = 1 une solution saturée d'HCl gaz dans l'éther et évapore sous vide. On obtient 0,7 g du produit attendu après cristallisation dans le mélange DCM/pentane, F = 112-114°C.

### EXEMPLE 14

### Chlorhydrate de 5-[2-(4-acétamido-4-phénylpipérid-1-yl)éthyl]-5-(3,4-dichlorophényl)-3-[3,5-bis(trifluorométhyl)benzyl]oxazolidin-2-one, monohydrate.

On chauffe à 80°C pendant 3 heures un mélange de 1,5 g du composé obtenu à l'étape B de l'EXEMPLE 8, 1,5 g de *p*-toluènesulfonate de 4-acétamido-4-phénylpipéridine, 1,6 g de K₂CO₃ dans 4 ml de DMF. Après refroidissement, on verse le mélange réactionnel dans l'eau, extrait par le mélange AcOEt/éther (50/50 ; v/v), lave la phase organique à l'eau, sèche sur MgSO₄ et évapore sous vide le solvant. On chromatographie le résidu sur silice H en éluant par le mélange DCM/MeOH (100/3 ; v/v). On dissout le produit obtenu dans du DCM, ajoute jusqu'à pH = 1 une solution saturée d'HCl gaz dans l'éther et évapore sous vide. On obtient 0,66 g du produit attendu après cristallisation dans le mélange DCM/pentane, F = 165-170°C.

### EXEMPLE 15

### Chlorhydrate de 4-benzyl-6-(3,4-dichlorophényl)-6-[2-[4-phényl-4-(pyrrolidin-1-ylcarbonylamino)pipérid-1-yl]éthyl]morpholin-3-one, monohydrate.

On chauffe à 80-100°C pendant 3 heures un mélange de 1,1 g du composé obtenu à l'étape B de l'EXEMPLE 6, 1,2 g de benzènesulfonate de 4-phényl-4-(pyrrolidin-1-ylcarbonylamino)pipéridine, 0,95 g de K₂CO₃ dans 3 ml de DMF. Après refroidissement à TA, on verse le mélange réactionnel dans l'eau, essore le précipité formé, le lave à l'eau et le sèche sous vide. On chromatographie le précipité sur silice H en éluant par le mélange DCM/MeOH de (100/2 ; v/v) à (100/3 ; v/v). On dissout le produit obtenu dans un minimum de DCM, acidifie à pH = 1 par ajout d'une solution saturée d'HCl gaz dans l'éther et essore le précipité formé. On obtient 0,54 g du produit attendu, F =158-160°C.

### EXEMPLE 16

### Dichlorhydrate de 4-benzyl-6-(3,4-dichlorophényl)-6-[2-[4-(2-diméthylamino thiazol-4-yl)-4-phénylpipérid-1-yl]éthyl]morpholin-3-one.

On prépare ce composé selon le mode opératoire décrit à l'EXEMPLE 15 à partir de 1,1 g du composé obtenu à l'étape B de l'EXEMPLE 6, 1,3 g de *p*-toluènesulfonate de 4-[2-(diméthylamino)thiazol-4-yl)-4-phénylpipéridine, 0,97 g de K₂CO₃ et 4 ml de DMF. On obtient 0,76 g du produit attendu, F = 155-160°C.

En procédant selon le mode opératoire décrit à l'EXEMPLE 11 à partir du composé obtenu à l'étape B de l'EXEMPLE 6 et des pipéridines décrites dans les Préparations, on prépare les composés selon l'invention rassemblés dans le TABLEAU I ci-après.

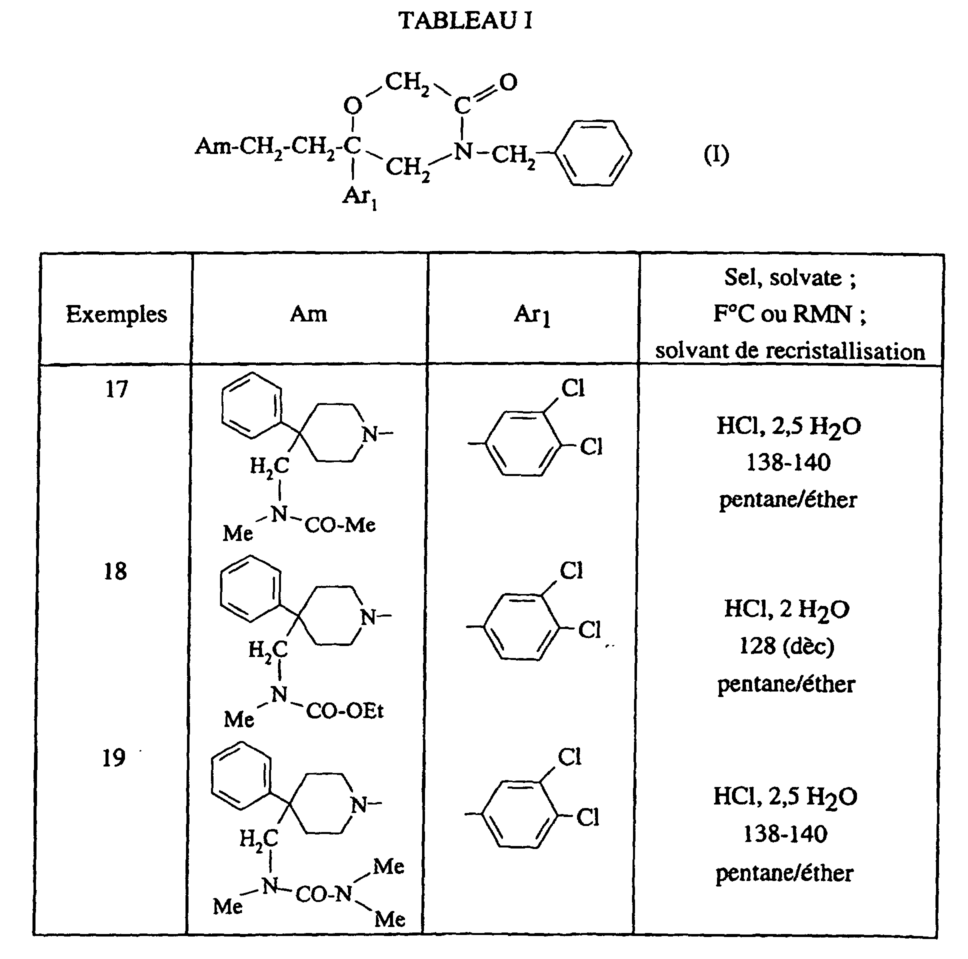

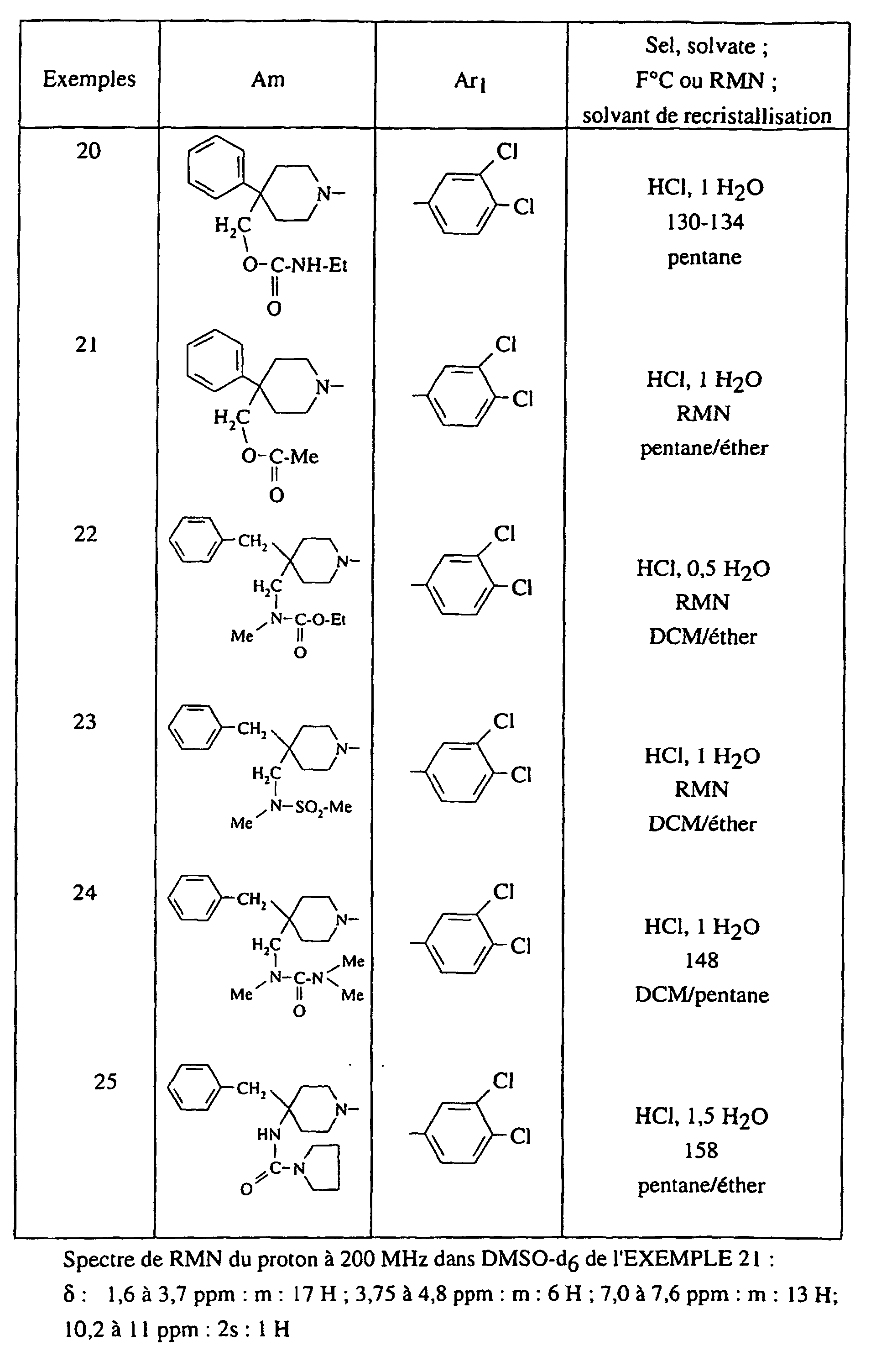

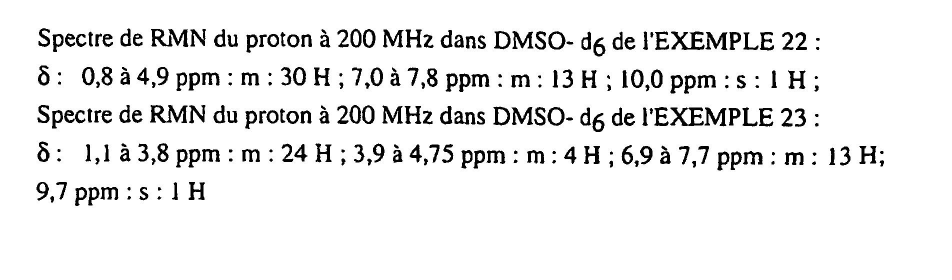

## Revendications

1. Composé de formule : 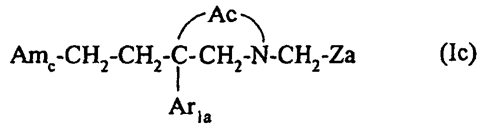 dans laquelle :
- Ac représente un radical bivalent choisi parmi : -O-CH₂-CO- ; -CH₂-O-CO- ou -O-CO- ;
- Am_{c} représente un groupe Am₁ₐ de formule : 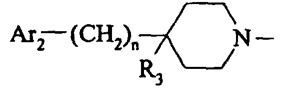
- n est 0 ou 1 ;
- Ar₂ représente un pyridyle ; un phényle non substitué ou substitué une ou plusieurs fois par un substituant choisi parmi : un atome d'halogène, un hydroxy, un (C₁-C₄)alcoxy, un (C₁-C₄)alkyle, un trifluorométhyle, un nitro, un méthylènedioxy, lesdits substituants étant identiques ou différents ; un thiényle ; un pyrimidyle ; un imidazolyle non substitué ou substitué par un (C₁-C₄)alkyle ;
- R₃ représente un groupe choisi parmi :
(1) hydrogène ;
(2) (C₁-C₇)alkyle ;
(3) formyle ;
(4) (C₁-C₇)alkylcarbonyle ;
(5) cyano ;
(6) -(CH₂)_{q}-OH ;
(7) -(CH₂)_{q}-O-(C₁-C₇)alkyle ;
(8) -(CH₂)_{q}-OCHO ;
(9) -(CH₂)_{q}-OCOR₁₇ ;
(10) -(CH₂)_{q}-OCONH-(C₁-C₇)alkyle ;
(11) -NR₄R₅ ;
(12) -(CH₂)_{q}-NR₆C(=W₁)R₇ ;
(13) -(CH₂)_{q}-NR₆COOR₈ ;
(14) -(CH₂)_{q}-NR₆SO₂R₉ ;
(15) -(CH₂)_{q}-NR₆C(=W₁)NR₁₀R₁₁ ;
(16) -CH₂-NR₁₂R₁₃ ;
(17) -CH₂-CH₂-NR₁₂R₁₃ ;
(18) -COOH ;
(19) (C₁-C₇)alcoxycarbonyle ;
(20) -C(=W₁)NR₁₀R₁₁ ;
(21) -CH₂-COOH ;
(22) (C₁-C₇)alcoxycarbonylméthyle ;
(23) -CH₂-C(=W₁)NR₁₀R₁₁ ;
(24) -O-CH₂CH₂-OR₁₈ ;
(25) -NR₆COCOR₁₉ ;
(26) -CO-NR₂₀-NR₂₁R₂₂ ;
(27) 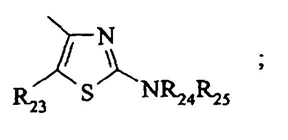
(28) 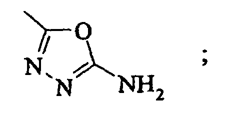
- ou R₃ constitue une double liaison entre l'atome de carbone auquel il est lié et l'atome de carbone voisin du cycle pipéridine ;
- q est 0, 1 ou 2 ;
- W représente un atome d'oxygène ou un atome de soufre ;
- R₄ et R₅ représentent chacun indépendamment un hydrogène ou un (C₁-C₇)alkyle ; R₅ peut de plus représenter un (C₃-C₇)cycloalkylméthyle, un benzyle ou un phényle ; ou R₄ et R₅ ensemble avec l'atome d'azote auquel ils sont liés constituent un hétérocycle choisi parmi l'azétidine, la pyrrolidine, la pipéridine, la morpholine, la thiomorpholine, la perhydroazépine ou la pipérazine non substituée ou substituée en position 4 par un (C₁-C₄)alkyle ;
- R₆ représente un hydrogène ou un (C₁-C₇)alkyle ;
- R₇ représente un hydrogène ; un (C₁-C₇)alkyle ; un vinyle ; un phényle ; un benzyle ; un pyridyle ; un (C₃-C₇)cycloalkyle non substitué ou substitué par un ou plusieurs méthyles ; un furyle ; un thiényle ; un pyrrolyle ; un imidazolyle ;
- ou R₆ et R₇ ensemble représentent un groupe -(CH₂)ₚ- ;
- p est 3 ou 4 ;
- R₈ représente un (C₁-C₇)alkyle ou un phényle ;
- R₉ représente un (C₁-C₇)alkyle ; un amino libre ou substitué par un ou deux (C₁-C₇)alkyles ; un phényle non substitué ou substitué une ou plusieurs fois par un substituant choisi parmi : un atome d'halogène, un (C₁-C₇)alkyle, un trifluorométhyle, un hydroxy, un (C₁-C₇)alcoxy, un carboxy, un (C₁-C₇)alcoxycarbonyle, un (C₁-C₇)alkylcarbonyloxy, un cyano, un nitro, un amino libre ou substitué par un ou deux (C₁-C₇)alkyles, lesdits substituants étant identiques ou différents ;
- R₁₀ et R₁₁ représentent chacun indépendamment un hydrogène ou un (C₁-C₇)alkyle ; R₁₁ peut de plus représenter un (C₃-C₇)cycloalkyle, un (C₃-C₇)cycloalkylméthyle, un hydroxy, un (C₁-C₄)alcoxy, un benzyle ou un phényle; ou R₁₀ et R₁₁ ensemble avec l'atome d'azote auquel ils sont liés constituent un hétérocycle choisi parmi l'azétidine, la pyrrolidine, la pipéridine, la morpholine, la thiomorpholine ou la perhydroazépine ;
- R₁₂ et R₁₃ représentent chacun indépendamment un hydrogène ou un (C₁-C₇)alkyle ; R₁₃ peut de plus représenter un (C₃-C₇)cycloalkylméthyle ou un benzyle ;
- R₁₇ représente un (C₁-C₇)alkyle ; un (C₃-C₇)cycloalkyle non substitué ou substitué par un ou plusieurs méthyles ; un phényle ; un pyridyle ;
- R₁₈ représente un hydrogène ; un (C₁-C₇)alkyle ; un formyle ; un (C₁-C₇)alkylcarbonyle ;
- R₁₉ représente un (C₁-C₄)alcoxy ;
- R₂₀ représente un hydrogène ou un (C₁-C₇)alkyle ;
- R₂₁ et R₂₂ représentent chacun indépendamment un hydrogène ou un (C₁-C₇)alkyle ;
- ou bien R₂₁ et R₂₂ ensemble avec l'atome d'azote auquel ils sont liés constituent un hétérocycle choisi parmi la pyrrolidine, la pipéridine ou la morpholine ;
- R₂₃ représente un hydrogène ou un (C₁-C₇)alkyle ;
- R₂₄ et R₂₅ représentent chacun indépendamment un hydrogène ou un (C₁-C₇)alkyle ; R₂₅ peut de plus représenter un formyle ou un (C₁-C₇)alkylcarbonyle ;
- Ar₁ₐ représente un phényle non substitué ou substitué une ou plusieurs fois par un substituant choisi parmi : un atome d'halogène, un hydroxy, un (C₁-C₄)alcoxy, un (C₁-C₄)alkyle, un trifluorométhyle, lesdits substituants étant identiques ou différents ;
- Za représente un phényle non substitué ou substitué une ou plusieurs fois par un substituant choisi parmi : un atome d'halogène, un trifluorométhyle, un (C₁-C₁₀)alkyle, un (C₁-C₁₀)alcoxy, un hydroxy, lesdits substituants étant identiques ou différents ;
et ses sels avec des acides minéraux ou organiques.

2. Composé optiquement pur selon la revendication 1 de formule : 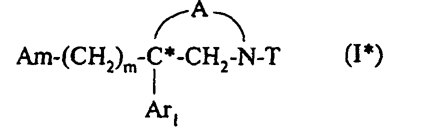 dans laquelle :
- "*" signifie que l'atome de carbone ainsi marqué a la configuration absolue (+) ou (-) déterminée ;
- Am est Amc tel que défini dans la revendication 1 ;
- m est 2 ;
- Ar₁ est Ar₁ₐ tel que défini dans la revendication 1 ;
- A est Ac tel que défini dans la revendication 1 ;
- T est -CH₂-Za, Za étant tel que défini dans la revendication 1 ;
ainsi que ses sels avec des acides minéraux ou organiques.

3. Composé selon l'une des revendications 1 ou 2 de formule : 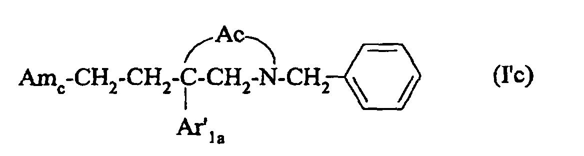 dans laquelle :
- Ac représente un radical bivalent choisi parmi : -O-CH₂-CO- ; -CH₂-O-CO- ou -O-CO-;
- Amc est tel que défini dans la revendication 1 ;
- Ar'₁ₐ représente un 3,4-dichlorophényle ou un 3,4-difluorophényle ; et leurs sels avec des acides minéraux ou organiques.

4. Composé de formule (Ic) ou (I'c) selon l'une quelconque des revendications 1 à 3, dans laquelle :
- Ar₂ représente un pyridyle ; un phényle non substitué ou substitué une ou plusieurs fois par un substituant choisi parmi : un atome d'halogène, un hydroxy, un (C₁-C₄)alcoxy, un (C₁-C₄)alkyle, un trifluorométhyle, lesdits substituants étant identiques ou différents ;
- R₃ représente un hydrogène, un (C₁-C₇)alkyle ; un formyle ; un (C₁-C₇)alkylcarbonyle ; un cyano ; un groupe -(CH₂)_{q}-OH ; un groupe (C₁-C₇)alkyl-O-(CH₂)_{q}- ; un groupe HCOO-(CH₂)_{q}- ; un groupe (C₁-C₇)alkyl-COO-(CH₂)_{q}-; un groupe (C₁-C₇)alkyl-NHCOO-(CH₂)_{q}- ; un groupe -NR₄R₅ ; un groupe -(CH₂)_{q}-NR₆COR₇ ; un groupe -(CH₂)_{q}-NR₆COOR₈ ; un groupe -(CH₂)_{q}-NR₆SO₂R₉ ; un groupe -(CH₂)_{q}-NR₆CONR₁₀R₁₁ ; un groupe -CH₂-NR₁₂R₁₃ ; un groupe -CH₂-CH₂-NR₁₂R₁₃ ; un carboxy ; un (C₁-C₇)alcoxycarbonyle ; un groupe -CONR₁₀R₁₁ ; un carboxyméthyle ; un (C₁-C₇)alcoxycarbonylméthyle ; un groupe -CH₂-CONR₁₀R₁₁ ; un 2-aminothiazol-4-yle dans lequel l'amino est libre ou substitué par un ou deux (C₁-C₇)alkyles ;
- ou R₃ constitue une double liaison entre l'atome de carbone auquel il est lié et l'atome de carbone voisin du cycle pipéridine ;
- q est 0, 1 ou 2 ;
et leurs sels avec des acides minéraux ou organiques.

5. Procédé pour la préparation des composés de formule (Ic) selon la revendication 1 et de leurs sels, **caractérisé en ce que** :
1) on traite un composé de formule : 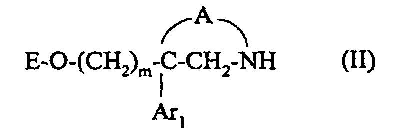 dans laquelle Ar₁ est Ar₁ₐ et A est Ac, tels que définis pour un composé de formule (Ic) dans la revendication 1, m est 2 et E représente l'hydrogène ou un groupe O-protecteur,
avec un dérivé halogéné de formule :
Hal-CH₂-Z (IV)
dans laquelle Z est Za est tel que défini dans la revendication 1, et Hal représente un halogène,
pour obtenir un composé de formule : 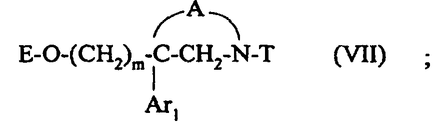 dans laquelle E, m, Ar₁ et A sont tels que définis ci-dessus et T est -CH₂-Za, Za étant tel que défini ci-dessus ;
2) on élimine éventuellement le groupe O-protecteur par action d'un acide ou d'une base, pour obtenir l'alcool de formule : 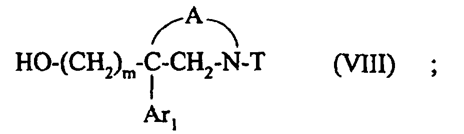 dans laquelle m, Ar₁, A et T sont tels que définis ci-dessus ;
3) on traite l'alcool (VIII) avec un composé de formule :
Y-SO₂-Cl (IX)
dans laquelle Y représente un groupe méthyle, phényle, tolyle, trifluorométhyle, pour obtenir un composé de formule : 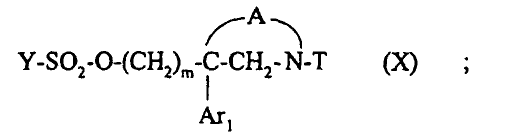 dans laquelle Y, m, Ar₁, A et T sont tels que définis ci-dessus ;
4) on fait réagir le composé (X) avec une amine secondaire cyclique de formule : 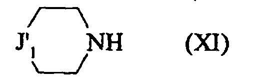 dans laquelle J'₁ représente un groupe : 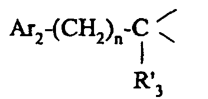 dans lequel Ar₂ et n sont tels que définis pour (Ic) dans la revendication 1 et R'₃ représente soit R₃ tel que défini pour (Ic) dans la revendication 1, soit un précurseur de R₃, étant entendu que lorsque R'₃ représente un hydroxyle ou un amino, ces groupes peuvent être protégés ; et
5) après déprotection éventuelle du groupe hydroxy ou du groupe amino représenté par R'₃ ou transformation éventuelle de R'₃ en R₃, on transforme éventuellement le produit ainsi obtenu en un de ses sels.

6. Procédé pour la préparation des composés de formule (Ic) selon la revendication 1, et de ses sels, **caractérisé en ce que** :
1') on oxyde un composé de formule (VIII) tel que défini dans la revendication 5 pour obtenir un composé de formule : 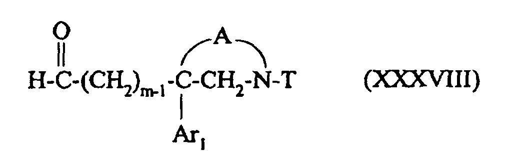 dans laquelle m, Ar₁, A et T sont tels que définis dans la revendication 5,
2') on fait réagir le composé de formule (XXXVIII) avec un composé de formule (XI) tel que défini dans la revendication 5 en présence d'un acide, puis on réduit le sel d'imminium formé intermédiairement au moyen d'un agent réducteur ; et
3') après déprotection éventuelle des groupes hydroxyles ou des groupes aminés ou transformation éventuelle de R'₃ en R₃, on transforme éventuellement le produit ainsi obtenu en l'un de ses sels.

7. Composé de formule : 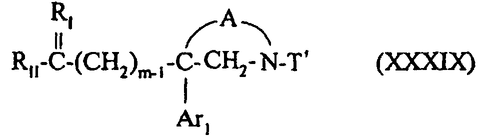 dans laquelle A est Ac et Ar, est Ar₁ₐ, Ac et Ar₁ₐ étant tels que définis pour (Ic) dans la revendication 1, m est 2 ;
- R_{I} représente deux atomes d'hydrogène et R_{II} représente :
- soit un groupe -O-E dans lequel E représente un atome d'hydrogène ou un groupe O-protecteur, choisi parmi le tétrahydropyran-2-yle, le benzoyle et le (C₁-C₄)alkylcarbonyle ;
- soit un groupe -O-SO₂-Y dans lequel Y représente un groupe méthyle, phényle, tolyle ou trifluorométhyle ;
- ou bien R_{I} représente un atome d'oxygène et R_{II} représente un atome d'hydrogène ;
- T' représente -CH₂-Za, Za étant tel que défini pour un composé de formule (Ic), T' peut de plus représenter l'hydrogène lorsque à la fois R_{I} représente 2 atomes d'hydrogène et R_{II} représente un groupe -O-E ;
sous forme énantiomériquement pure ou sous forme de racémique.

8. Composé selon la revendication 7, répondant à la formule : 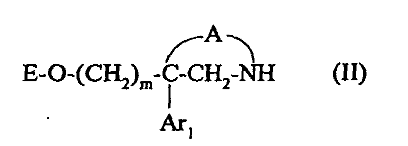 dans laquelle A est Ac et Ar, est Ar₁ₐ, Ac et Ar₁ₐ étant tels que définis pour (Ic) dans la revendication 1, m est 2 et E représente l'hydrogène ou un groupe O-protecteur, choisi parmi le tétrahydropyran-2-yle, le benzoyle et le (C₁-C₄)alkylcarbonyle, sous forme énantiomériquement pure ou sous forme racémique.

9. Composé selon la revendication 7, répondant à la formule : 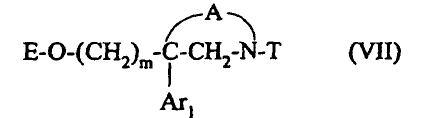 dans laquelle A est Ac et Ar₁ est Ar₁ₐ, Ac et Ar₁ₐ étant tels que définis pour (Ic) dans la revendication 1, m est 2, T est -CH₂-Za, Za étant tel que défini dans la revendication 1 et E représente l'hydrogène ou un groupe O-protecteur choisi parmi le tétrahydropyran-2-yle, le benzoyle et le (C₁-C₄)alkylcarbonyle, sous forme énantiomériquement pure ou sous forme racémique.

10. Composé selon la revendication 7, répondant à la formule : 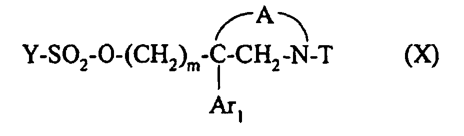 dans laquelle A est Ac et Ar₁ est Ar₁ₐ, Ac et Ar₁ₐ étant tels que définis pour (Ic) dans la revendication 1, m est 2, T est -CH₂-Za, Za étant tel que défini dans la revendication 1 et Y représente un groupe méthyle, phényle, tolyle ou trifluorométhyle, sous forme énantiomériquement pure ou sous forme racémique.

11. Composé selon la revendication 7, répondant à la formule : 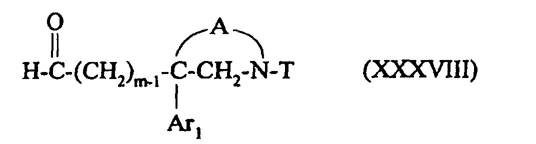 dans laquelle A est Ac et Ar, est Ar₁ₐ, Ac et Ar₁ₐ étant tels que définis pour (Ic) dans la revendication 1, m est 2, T est -CH₂-Za, Za étant tel que défini dans la revendication 1, sous forme énantiomériquement pure ou sous forme racémique.

12. Composé de formule : 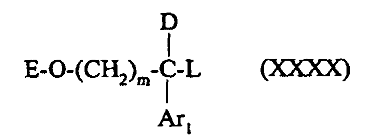 dans laquelle :
- m est 2 et Ar₁ est Ar₁ₐ tel que défini pour un composé de formule (Ic) dans la revendication 1,
- E représente un hydrogène ou un groupe O-protecteur, choisi parmi le tétrahydropyran-2-yle, le benzoyle et le (C₁-C₄)alkylcarbonyle ;
- L représente un groupe cyano ou un groupe amino méthyle ;
- D représente le groupe -CH₂-OH ;
sous forme énantiomériquement pure ou sous forme racémique lorsque L est un groupe aminométhyle.

13. Composition pharmaceutique contenant à titre de principe actif, un composé selon l'une quelconque des revendications 1 à 4, ou un de ses sels pharmaceutiquement acceptables.

14. Composition pharmaceutique selon la revendication 13, sous forme d'unité de dosage, dans laquelle le principe actif est mélangé à au moins un excipient pharmaceutique.

15. Composition pharmaceutique selon la revendication 14, contenant 0,5 à 1000 mg de principe actif.

16. Composition pharmaceutique selon la revendication 15, contenant 2,5 à 250 mg de principe actif.

## Patentansprüche

1. Verbindungen der Formel 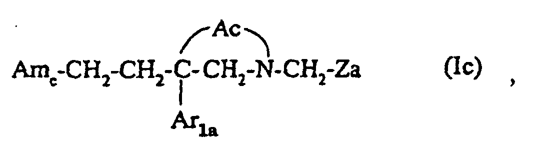 in der bedeuten:
- Ac eine zweiwertige Gruppe, die unter -O-CH₂-CO-, -CH₂-O-CO- und -O-CO- ausgewählt ist;
- Amc eine Gruppe Am₁ₐ der Formel 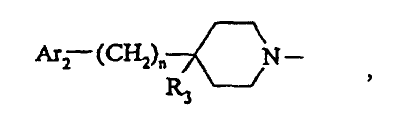 worin bedeuten:
- n 0 oder 1;
- Ar₂ Pyridyl; Phenyl, das unsubstituiert oder einfach oder mehrfach mit einem Substituenten substituiert ist, der ausgewählt ist unter Halogenatomen, Hydroxy, C₁₋₄-Alkoxy, C₁₋₄-Alkyl, Trifluormethyl, Nitro und Methylendioxy, wobei diese Substituenten gleich oder verschieden sind; Thienyl; Pyrimidyl; Imidazolyl, das unsubstituiert oder mit einer C₁₋₄-Alkylgruppe substituiert ist;
- R₃ eine Gruppe, die ausgewählt ist unter
(1) Wasserstoff;
(2) C₁₋₇-Alkyl;
(3) Formyl;
(4) C₁₋₇-Alkylcarbonyl;
(5) Cyano;
(6) -(CH₂)_{q}-OH;
(7) -(CH₂)_{q}-O-C₁₋₇-Alkyl;
(8) -(CH₂)_{q}-OCHO;
(9) -(CH₂)_{q}-OCOR₁₇;
(10) -(CH₂)_{q}-OCONH-C₁₋₇-Alkyl;
(11) -NR₄R₅;
(12) -(CH₂)_{q}-NR₆C(=W₁)R₇;
(13) -(CH₂)_{q}-NR₆COOR₈;
(14) -(CH₂)_{q}-NR₆SO₂R₉;
(15) -(CH₂)_{q}-NR₆C(=W₁)NR₁₀R₁₁;
(16) -CH₂-NR₁₂R₁₃;
(17) -CH₂-CH₂-NR₁₂R₁₃;
(18) -COOH;
(19) C₁₋₇-Alkoxycarbonyl;
(20) -C(=W₁)NR₁₀R₁₁;
(21) -CH₂-COOH;
(22) C₁₋₇-Alkoxycarbonylmethyl;
(23) -CH₂-C(=W₁)NR₁₀R₁₁;
(24) -O-CH₂CH₂-OR₁₈;
(25) NR₆COCOR₁₉;
(26) -CO-NR₂₀-NR₂₁R₂₂;
(27) 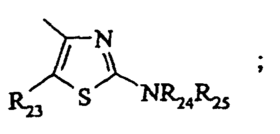
(28) 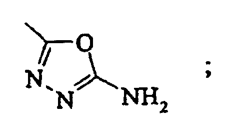
- oder R₃ eine Doppelbindung zwischen dem Kohlenstoffatom, an dem R₃ gebunden ist, und dem benachbarten Kohlenstoffatom des Piperidinrings;
wobei darstellen:
- q 0, 1 oder 2;
- W₁ ein Sauerstoffatom oder ein Schwefelatom;
- R₄ und R₅ unabhängig voneinander Wasserstoff oder C₁₋₇-Alkyl, wobei R₅ ferner C₃₋₇-Cycloalkylmethyl, Benzyl oder Phenyl bedeuten kann; oder R₄ und R₅ zusammen mit dem Stickstoffatom, an dem sie gebunden sind, einen Heterocyclus, der ausgewählt ist unter Azetidin, Pyrrolidin, Piperidin, Morpholin, Thiomorpholin, Perhydroazetin oder Piperazin, das unsubstituiert oder in Position 4 mit einer C₁₋₄-Alkylgruppe substituiert ist;
- R₆ Wasserstoff oder C₁₋₇-Alkyl;
- R₇ Wasserstoff; C₁₋₇-Alkyl; Vinyl; Phenyl; Benzyl; Pyridyl; C₃₋₇-Cycloalkyl, das unsubstituiert oder mit einer oder mehreren Methylgruppen substituiert ist; Furyl; Thienyl; Pyrrolyl; Imidazolyl;
- oder R₆ und R₇ zusammen eine Gruppe -(CH₂)ₚ-,
worin p 3 oder 4 bedeutet;
- R₈ C₁₋₇-Alkyl oder Phenyl;
- R₉ C₁₋₇-Alkyl; eine Aminogruppe, die frei oder mit einer oder zwei C₁₋₇-Alkylgruppen substituiert ist; Phenyl, das unsubstituiert oder einfach oder mehrfach mit einem Substituenten substituiert ist, der ausgewählt ist unter Halogenatomen, C₁₋₇-Alkyl, Trifluormethyl, Hydroxy, C₁₋₇-Alkoxy, Carboxy, C₁₋₇-Alkoxycarbonyl, C₁₋₇-Alkylcarbonyloxy, Cyano, Nitro, Amino, das frei oder mit einer oder zwei C₁₋₇-Alkylgruppen substituiert ist, wobei die Substituenten gleich oder verschieden sind;
- R₁₀ und R₁₁ unabhängig voneinander Wasserstoff oder C₁₋₇-Alkyl, wobei R₁₁ ferner C₃₋₇-Cycloalkyl, C₃₋₇-Cycloalkylmethyl, Hydroxy, C₁₋₄-Alkoxy, Benzyl oder Phenyl bedeuten kann; oder R₁₀ und R₁₁ zusammen mit dem Stickstoffatom, an dem sie gebunden sind, einen Heterocyclus, der ausgewählt ist unter Azetidin, Pyrrolidin, Piperidin, Morpholin, Thiomorpholin und Perhydroazetin;
- R₁₂ und R₁₃ unabhängig voneinander Wasserstoff oder C₁₋₇-Alkyl, wobei R₁₃ ferner C₃₋₇-Cycloalkylmethyl oder Benzyl bedeuten kann;
- R₁₇ C₁₋₇-Alkyl; C₃₋₇-Cycloalkyl, das unsubstituiert oder mit einer oder mehreren Methylgruppen substituiert ist; Phenyl; Pyridyl;
- R₁₈ Wasserstoff; C₁₋₇-Alkyl; Formyl; C₁₋₇-Alkylcarbonyl;
- R₁₉ C₁₋₄-Alkoxy;
- R₂₀ Wasserstoff oder C₁₋₇-Alkyl;
- R₂₁ und R₂₂ unabhängig voneinander Wasserstoff oder C₁₋₇-Alkyl, oder R₂₁ und R₂₂ zusammen mit dem Stickstoffatom, an dem sie gebunden sind, einen Heterocyclus, der ausgewählt ist unter Pyrrolidin, Piperidin und Morpholin;
- R₂₃ Wasserstoff oder C₁₋₇-Alkyl;
- R₂₄ und R₂₅ unabhängig voneinander Wasserstoff oder C₁₋₇-Alkyl, wobei R₂₅ ferner Formyl oder C₁₋₇-Alkylcarbonyl bedeuten kann;
- Ar₁ₐ Phenyl, das unsubstituiert oder einfach oder mehrfach mit einem Substituenten substituiert ist, der ausgewählt ist unter Halogenatomen, Hydroxy, C₁₋₄-Alkoxy, C₁₋₄-Alkyl, Trifluormethyl, wobei diese Substituenten gleich oder verschieden sind;
- Za Phenyl, das unsubstituiert oder einfach oder mehrfach mit einem Substituenten substituiert ist, der ausgewählt ist unter Halogenatomen, Trifluormethyl, C₁₋₁₀-Alkyl, C₁₋₁₀-Alkoxy, Hydroxy, wobei diese Substituenten gleich oder verschieden sind;
sowie die Salze dieser Verbindungen mit anorganischen und organischen Säuren.

2. Optisch reine Verbindungen nach Anspruch 1 der Formel 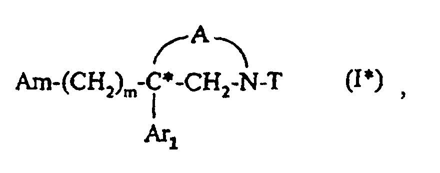 in der bedeuten:
- "*", dass das damit markierte Kohlenstoffatom die ermittelte absolute (+)-Konfiguration oder (-)-Konfiguration besitzt;
- Am Amc wie in Anspruch 1 definiert;
- m 2;
- Ar₁ Ar₁ₐ wie in Anspruch 1 definiert;
- A Ac wie in Anspruch 1 definiert;
und
- T -CH₂-Za, wobei Za wie in Anspruch 1 definiert ist,
sowie die Salze dieser Verbindungen mit anorganischen und organischen Säuren.

3. Verbindungen nach Anspruch 1 oder 2 der Formel 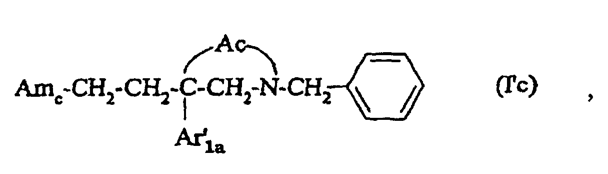 in der bedeuten:
- Ac eine zweiwertige Gruppe, die unter -O-CH₂-CO-, -CH₂-O-CO- und -O-CO- ausgewählt ist;
- Amc dasselbe wie in Anspruch 1 definiert;
- Ar'₁ₐ 3,4-Dichlorphenyl oder 3,4-Difluorphenyl;
sowie die Salze dieser Verbindungen mit anorganischen und organischen Säuren.

4. Verbindungen der Formel (Ic) oder (I'c) nach einem der Ansprüche 1 bis 3, worin bedeuten:
- Ar₂ Pyridyl; Phenyl, das unsubstituiert oder einfach oder mehrfach mit einem Substituenten substituiert ist, der ausgewählt ist unter Halogenatomen, Hydroxy, C₁₋₄-Alkoxy, C₁₋₄-Alkyl, Trifluormethyl, wobei diese Substituenten gleich oder verschieden sind;
- R₃ Wasserstoff; C₁₋₇-Alkyl; Formyl; C₁₋₇-Alkylcarbonyl; Cyano; -(CH₂)_{q}-OH; C₁₋₇-Alkyl-O-(CH₂)_{q}-; HCOO-(CH₂)_{q}-; C₁₋₇-Alkyl-COO-(CH₂)_{q}-; C₁₋₇-Alkyl-NHCOO-(CH₂)_{q}-; -NR₄R₅; -(CH₂)_{q}-NR₆COR₇; -(CH₂)_{q}-NR₆COOR₈; -(CH₂)_{q}-NR₆SO₂R₉; -(CH₂)_{q}-NR₆CONR₁₀R₁₁; -(CH₂)_{q}-NR₁₂R₁₃; -CH₂-CH₂-NR₁₂R₁₃; Carboxy; C₁₋₇-Alkoxycarbonyl; -CONR₁₀R₁₁; Carboxymethyl; C₁₋₇-Alkoxycarbonylmethyl; -CH₂-CONR₁₀R₁₁;
2-Aminotiazol-4-yl, worin die Aminogruppe frei oder mit einer oder zwei C₁₋₇-Alkylgruppen substituiert ist;
- oder R₃ eine Doppelbindung zwischen dem Kohlenstoffatom, an dem R₃ gebunden ist, und dem benachbarten Kohlenstoffatom des Piperidinrings,
und
- q 0, 1 oder 2,
sowie die Salze dieser Verbindungen mit anorganischen und organischen Säuren.

5. Verfahren zur Herstellung der Verbindungen der Formel (Ic) nach Anspruch 1 und ihrer Salze, **gekennzeichnet durch**
1) Behandlung einer Verbindung der Formel 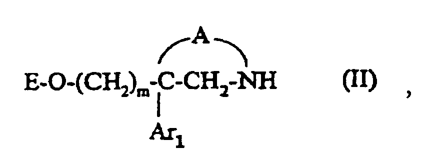 in der Ar₁ Ar₁ₐ und A Ac bedeuten, wie diese in Anspruch 1 für eine Verbindung der Formel (Ic) definiert sind,
m gleich 2 ist und
E Wasserstoff oder eine O-Schutzgruppe bedeutet,
mit einem halogenierten Derivat der Formel
**Hal-CH**_{**2**}**-Z (IV) ,**
in der Z Za wie in Anspruch 1 definiert bedeutet und Hal ein Halogen darstellt,
zu einer Verbindung der Formel 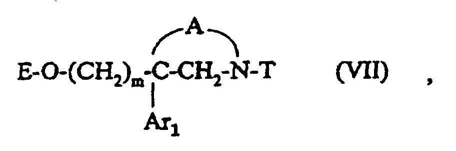 in der E, m, Ar₁ und A wie oben definiert sind und T -CH₂-Za bedeutet, wobei Za wie oben definiert ist;
2) gegebenenfalls Abspaltung der O-Schutzgruppe **durch** Einwirkung einer Säure oder einer Base unter Erhalt des Alkohols der Formel 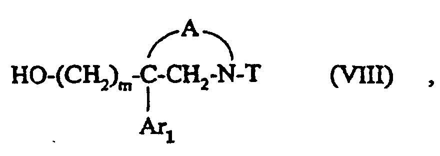 in der m, Ar₁, A und T wie oben definiert sind;
3) Behandlung des Alkohols (VIII) mit einer Verbindung der Formel
**Y-SO**_{**2**}**-Cl (IX) ,**
in der Y Methyl, Phenyl, Tolyl oder Trifluormethyl bedeutet,
zu einer Verbindung der Formel 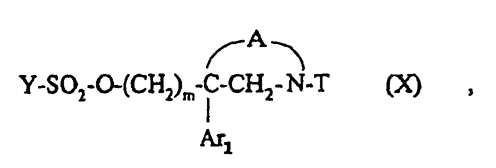 in der Y, m, Ar₁, A und T wie oben definiert sind;
4) Umsetzung der Verbindung (X) mit einem sekundären cyclischen Amin der Formel 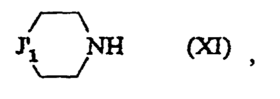 in der J'₁ eine Gruppe 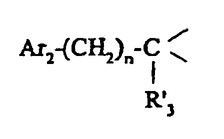 bedeutet, in der Ar₂ und n wie für (Ic) in Anspruch 1 definiert sind und R'₃ entweder R₃ wie für (Ic) in Anspruch 1 definiert oder einen Vorläufer von R₃ bedeutet, mit der Maßgabe, dass, wenn R'₃ Hydroxy oder Amino bedeutet, diese Gruppen geschützt sein können,
und
5) nach gegebenenfalls vorgenommener Schutzgruppenabspaltung von der Hydroxy- oder Aminogruppe, die **durch** R'₃ dargestellt wird, oder gegebenenfalls vorgenommener Umwandlung von R'₃ in R₃
gegebenenfalls Umwandlung des so erhaltenen Produkts in eines seiner Salze.

6. Verfahren zur Herstellung der Verbindungen der Formel (Ic) nach Anspruch 1 und ihrer Salze, **gekennzeichnet durch**
1') Oxidation einer Verbindung der Formel (VIII) wie in Anspruch 5 definiert unter Erhalt einer Verbindung der Formel 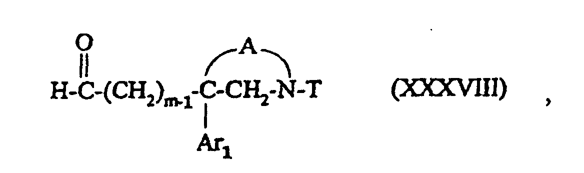 in der m, Ar₁, A und T wie in Anspruch 5 definiert sind;
2') Umsetzung der Verbindung der Formel (XXXVIII) mit einer Verbindung der Formel (XI) wie in Anspruch 5 definiert in Gegenwart einer Säure und anschließende Reduktion des als Zwischenprodukt gebildeten Iminiumsalzes mit einem Reduktionsmittel
und
3') nach gegebenenfalls vorgenommener Schutzgruppenabspaltung von den Hydroxylgruppen oder Aminogruppen oder gegebenenfalls vorgenommener Umwandlung von R'₃ in R₃ gegebenenfalls Umwandlung des so erhaltenen Produkts in eines seiner Salze.

7. Verbindungen der Formel 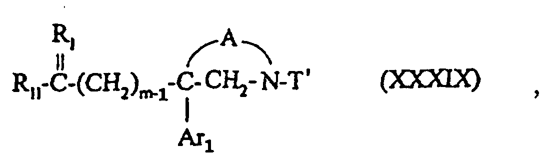 in der bedeuten:
- A Ac und Ar₁ Ar₁ₐ, wobei Ac und Ar₁ₐ wie für (Ic) in Anspruch 1 definiert sind;
- m 2;
- R_{I} zwei Wasserstoffatome und R_{II}
- entweder eine Gruppe -O-E, in der E ein Wasserstoffatom oder eine O-Schutzgruppe bedeutet, die unter Tetrahydropyran-2-yl, Benzoyl und C₁₋₄-Alkylcarbonyl ausgewählt ist,
- oder eine Gruppe -O-SO₂-Y, in der Y Methyl, Phenyl, Tolyl oder Trifluormethyl bedeutet,
- oder R_{I} ein Sauerstoffatom und R_{II} ein Wasserstoffatom;
- T' -CH₂-Za, wobei Za wie für eine Verbindung der Formel (Ic) definiert ist, wobei T' ferner ein Wasserstoffatom bedeuten kann, wenn gleichzeitig R_{I} zwei Wasserstoffatome und R_{II} eine Gruppe -O-E bedeuten,
in enantiomerenreiner Form oder in Racematform.

8. Verbindungen nach Anspruch 7 der Formel 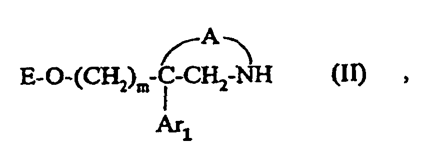 worin bedeuten:
A Ac und Ar₁ Ar₁ₐ, wobei Ac und Ar₁ₐ wie für (Ic) in Anspruch 1 definiert sind,
m 2 und
E Wasserstoff oder eine O-Schutzgruppe, die unter Tetrahydropyran-2-yl, Benzoyl und C₁₋₄-Alkylcarbonyl ausgewählt ist,
in enantiomerenreiner Form oder in Racematform.

9. Verbindungen nach Anspruch 7 der Formel 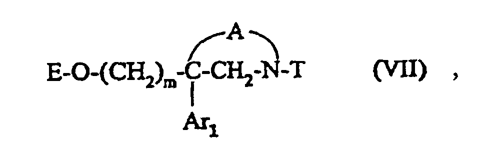 worin bedeuten:
A Ac und Ar₁ Ar₁ₐ, wobei Ac und Ar₁ₐ wie für (Ic) in Anspruch 1 definiert sind,
m 2,
T -CH₂-Za, wobei Za wie in Anspruch 1 definiert ist, und
E Wasserstoff oder eine O-Schutzgruppe, die unter Tetrahydropyran-2-yl, Benzoyl und C₁₋₄-Alkylcarbonyl ausgewählt ist,
in enantiomerenreiner Form oder in Racematform.

10. Verbindungen nach Anspruch 7 der Formel 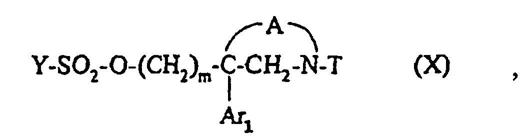 worin bedeuten:
A Ac und Ar₁ Ar₁ₐ, wobei Ac und Ar₁ₐ wie für (Ic) in Anspruch 1, definiert sind,
m 2,
T -CH₂-Za, wobei Za wie in Anspruch 1 definiert ist, und
Y Methyl, Phenyl, Tolyl oder Trifluormethyl,
in enantiomerenreiner Form oder in Racematform.

11. Verbindungen nach Anspruch 7 der Formel 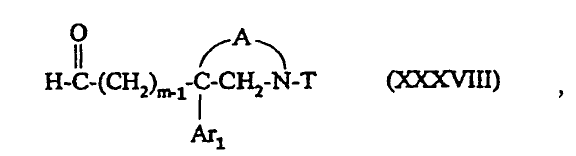 worin bedeuten:
A Ac und Ar₁ Ar₁ₐ, wobei Ac und Ar₁ₐ wie für (Ic) in Anspruch 1 definiert sind,
m 2 und
T -CH₂-Za, wobei Za wie in Anspruch 1 definiert ist,
in enantiomerenreiner Form oder in Racematform.

12. Verbindungen der Formel 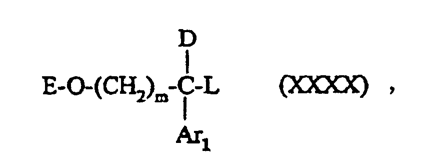 worin bedeuten:
- m 2,
- Ar₁ Ar₁ₐ wie für eine Verbindung der Formel (Ic) in Anspruch 1 definiert,
- E Wasserstoff oder eine O-Schutzgruppe, die unter Tetrahydropyran-2-yl, Benzoyl und C₁₋₄-Alkylcarbonyl ausgewählt ist,
- L Cyano oder Aminomethyl und
- D die Gruppe -CH₂-OH,
in enantiomerenreiner Form oder in Racematform, wenn L eine Aminomethylgruppe ist.

13. Pharmazeutische Zusammensetzung, die als Wirkstoff eine Verbindung nach einem der Ansprüche 1 bis 4 oder eines ihrer pharmazeutisch akzeptablen Salze enthält.

14. Pharmazeutische Zusammensetzung nach Anspruch 13 in Form einer Dosiereinheit, in welcher der Wirkstoff mit mindestens einem pharmazeutischen Excipiens vermischt ist.

15. Pharmazeutische Zusammensetzung nach Anspruch 14, die 0,5 bis 1000 mg Wirkstoff enthält.

16. Pharmazeutische Zusammensetzung nach Anspruch 15, die 2,5 bis 250 mg Wirkstoff enthält.

## Claims

1. Compound of the formula: 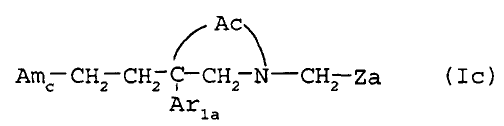 in which:
- Ac is a divalent radical selected from -O-CH₂-CO-, -CH₂-O-CO- and -O-CO-;
- Am_{c} is a group Am₁ₐ of the formula: 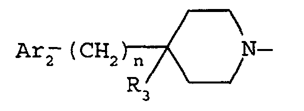
- n is 0 or 1;
- Ar₂ is a pyridyl; a phenyl which is unsubstituted or monosubstituted or polysubstituted by a substituent selected from a halogen atom, a hydroxyl, a (C₁-C₄)-alkoxy, a (C₁-C₄)-alkyl, a trifluoromethyl, a nitro and a methylenedioxy, said substituents being identical or different; a thienyl; a pyrimidyl; or an imidazolyl which is unsubsituted or substituted by a (C₁-C₄)-alkyl;
- R₃ is a group selected from:
(1) hydrogen;
(2) (C₁-C₇)-alkyl;
(3) formyl;
(4) (C₁-C₇)-alkylcarbonyl;
(5) cyano;
(6) -(CH₂)_{q}-OH;
(7) -(CH₂)_{q}-O-(C₁-C₇)-alkyl;
(8) -(CH₂)_{q}-OCHO;
(9) -(CH₂)_{q}-OCOR₁₇;
(10) -(CH₂)_{q}-OCONH-(C₁-C₇)-alkyl;
(11) -NR₄R₅;
(12) -(CH₂)_{q}-NR₆C(=W₁)R₇;
(13) -(CH₂)_{q}-NR₆COOR₈;
(14) -(CH₂)_{q}-NR₆SO₂R₉;
(15) -(CH₂)_{q}-NR₆C(=W₁)NR₁₀R₁₁;
(16) -CH₂-NR₁₂R₁₃;
(17) -CH₂-CH₂-NR₁₂R₁₃;
(18) -COOH;
(19) (C₁-C₇)-alkoxycarbonyl;
(20) -C(=W₁)NR₁₀R₁₁;
(21) -CH₂-COOH;
(22) (C₁-C₇)-alkoxycarbonylmethyl;
(23) -CH₂-C(=W₁)NR₁₀R₁₁;
(24) -O-CH₂CH₂-OR₁₈;
(25) -NR₆COCOR₁₉;
(26) -CO-NR₂₀-NR₂₁R₂₂;
(27) 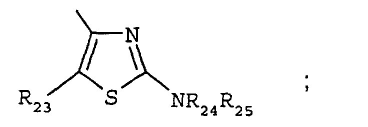
(28) 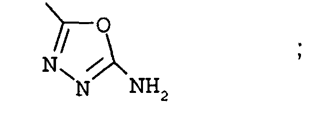
- or R₃ constitutes a double bond between the carbon atom to which it is bonded and the adjacent carbon atom of the piperidine ring;
- q is 0, 1 or 2;
- W₁ is an oxygen atom or a sulfur atom;
- R₄ and R₅ are each independently a hydrogen or a (C₁-C₇)-alkyl; R₅ can also be a (C₃-C₇)-cycloalkylmethyl, a benzyl or a phenyl; or R₄ and R₅, together with the nitrogen atom to which they are bonded, form a heterocycle selected from azetidine, pyrrolidine, piperidine, morpholine, thiomorpholine, perhydroazepine and piperazine which is unsubstituted or substituted in the 4-position by a (C₁-C₄)-alkyl;
- R₆ is a hydrogen or a (C₁-C₇)-alkyl;
- R₇ is a hydrogen; a (C₁-C₇)-alkyl; a vinyl; a phenyl; a benzyl; a pyridyl; a (C₃-C₇)-cycloalkyl which is unsubstituted or substituted by one or more methyls; a furyl; a thienyl; a pyrrolyl; or an imidazolyl;
- or R₆ and R₇ together are a group - (CH₂)ₚ-;
- p is 3 or 4;
- R₈ is a (C₁-C₇)-alkyl or a phenyl;
- R₉ is a (C₁-C₇)-alkyl; an amino which is free or substituted by one or two (C₁-C₇)-alkyls; or a phenyl which is unsubstituted or monosubstituted or polysubstituted by a substituent selected from a halogen atom, a (C₁-C₇)-alkyl, a trifluoromethyl, a hydroxyl, a (C₁-C₇)-alkoxy, a carboxyl, a (C₁-C₇)-alkoxycarbonyl, a (C₁-C₇)-alkylcarbonyloxy, a cyano, a nitro and an amino which is free or substituted by one or two (C₁-C₇)-alkyls, said substituents being identical or different;
- R₁₀ and R₁₁ are each independently a hydrogen or a (C₁-C₇)-alkyl; R₁₁ can also be a (C₃-C₇)-cycloalkyl, a (C₃-C₇)-cycloalkylmethyl, a hydroxyl, a (C₁-C₄)-alkoxy, a benzyl or a phenyl; or R₁₀ and R₁₁, together with the nitrogen atom to which they are bonded, form a heterocycle selected from azetidine, pyrrolidine, piperidine, morpholine, thiomorpholine and perhydroazepine;
- R₁₂ and R₁₃ are each independently a hydrogen or a (C₁-C₇)-alkyl; R₁₃ can also be a (C₃-C₇)cycloalkylmethyl or a benzyl;
- R₁₇ is a (C₁-C₇)-alkyl; a (C₃-C₇)-cycloalkyl which is unsubstituted or substituted by one or more methyls; a phenyl; or a pyridyl;
- R₁₈ is a hydrogen; a (C₁-C₇)-alkyl; a formyl; or a (C₁-C₇)-alkylcarbonyl;
- R₁₉ is a (C₁-C₄)-alkoxy;
- R₂₀ is a hydrogen or a (C₁-C₇)-alkyl;
- R₂₁ and R₂₂ are each independently a hydrogen or a (C₁-C₇)-alkyl ;
- or alternatively R₂₁ and R₂₂, together with the nitrogen atom to which they are bonded, form a heterocycle selected from pyrrolidine, piperidine and morpholine ;
- R₂₃ is a hydrogen or a (C₁-C₇)-alkyl ; and
- R₂₄ and R₂₅ are each independently a hydrogen or a (C₁-C₇)-alkyl; R₂₅ can also be a formyl or a (C₁-C₇)-alkylcarbonyl;
- Ar₁ₐ is a phenyl which is unsubstituted or monosubstituted or polysubstituted by a substituent selected from a halogen atom, a hydroxyl, a (C₁-C₄)-alkoxy, a (C₁-C₄)-alkyl and a trifluoromethyl, said substituents being identical or different; and
- Za is a phenyl which is unsubstituted or monosubstituted or polysubstituted by a substituent selected from a halogen atom, a trifluoromethyl, a (C₁-C₁₀)-alkyl, a (C₁-C₁₀)-alkoxy and a hydroxyl, said substituents being identical or different;
and the salts thereof with mineral or organic acids.

2. Optically pure compound according to claim 1 of the formula: 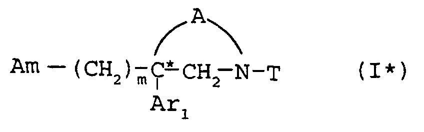 in which:
- "*" denotes that the carbon atom carrying this label has the determined (+) or (-) absolute configuration ; and
- Am is Am_{c} as defined in claim 1;
- m is 2;
- Ar₁ is Ar₁ₐ as defined in claim 1;
- A is Ac as defined in claim 1;
- T is -CH₂-Za, Za being as defined in claim 1;
and the salts thereof with mineral or organic acids.

3. Compound according to any one of claims 1 or 2, of formula: 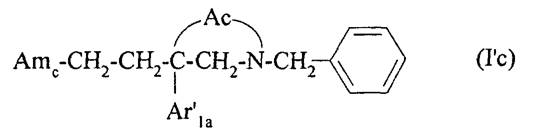 in which:
- Ac is a radical selected from -O-CH₂-CO-; -CH₂-O-CO- and -O-CO-;
- Am_{c} is as defined in claim 1;
- Ar'₁ₐ is a 3,4-dichlorophenyl or a 3,4-difluorophenyl;
and the salts thereof with mineral or organic acids.

4. A compound of formula (Ic) or (I'c) according to any one of claims 1 to 3, in which:
- Ar₂ is a pyridyl; a phenyl which is unsubstituted or monosubstituted or polysubstituted by a substituent selected from a halogen atom, a hydroxyl, a (C₁-C₄)-alkoxy, a (C₁-C₄)-alkyl, a trifluoromethyl, said substituents being identical or different;
- R₃ is a hydrogen; a (C₁-C₇)-alkyl; a formyl; a C₁-C₇)-alkylcarbonyl; a cyano; a -(CH₂)_{q}-OH group; a (C₁-C₇)alkyl-O-(CH₂)_{q}- group; a HCOO-(CH₂)_{q}- group; a (C₁-C₇)alkyl-COO-(CH₂)_{q}- group; a (C₁-C₇)alkyl-NHCOO-(CH₂)_{q}-group; a -NR₄R₅ group; a -(CH₂)_{q}-NR₆COR₇ group; a -(CH₂)_{q}-NR₆COOR₈ group; a -(CH₂)_{q}-NR₆SO₂R₉ group; a -(CH₂)_{q}-NR₆CONR₁₀R₁₁ group; a -CH₂-NR₁₂R₁₃ group; a -CH₂-CH₂-NR₁₂R₁₃ group; a carboxy; a (C₁-C₇)-alkoxycarbonyl; a -CONR₁₀R₁₁ group; a carboxymethyl; a (C₁-C₇)-alkoxycarbonylmethyl; a -CH₂-CONR₁₀R₁₁ group; a 2-aminothiazol-4-yl, in which the amino is free or substituted by one or two (C₁-C₇)alkyl;
- or R₃ constitutes a double bond between the carbon atom to which it is bonded and the adjacent carbon atom of the piperidine ring;
- q is 0, 1 or 2;
and the salts thereof with mineral or organic acid.

5. Method of preparing the compounds of formula (Ic) according to claim 1 and the salts thereof, **characterized in that**:
1) a compound of the formula: 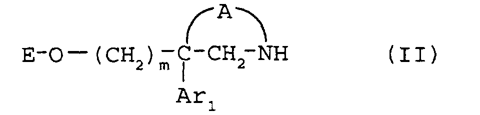 in which Ar₁ is Ar₁ₐ and A is Ac as defined for a compound of formula (Ic) in claim 1, m is 2 and E is hydrogen or an O-protecting group,
is treated with a halogenated derivative of the formula:
Hal-CH₂-Z (IV)
in which Z is Za as defined in claim 1 and Hal is a halogen,
to give a compound of the formula: 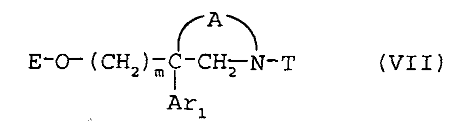 in which E, m, Ar₁ and A are as defined above and T is CH₂Za, Za being as defined above;
2) the O-protecting group is optionally removed by reaction with an acid or a base to give the alcohol of the formula: 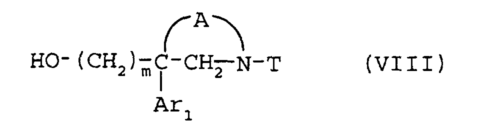 in which m, Ar₁, A and T are as defined above;
3) the alcohol (VIII) is treated with a compound of the formula:
Y-SO₂-Cl (IX)
in which Y is a methyl, phenyl, tolyl or trifluoromethyl group, to give a compound of the formula: 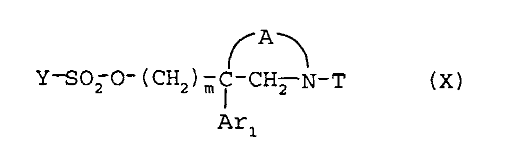 in which Y, m, Ar₁, A and T are as defined above;
4) the compound (X)is reacted with a cyclic secondary amine of the formula: 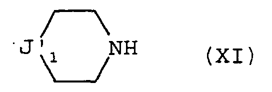 in which J'₁ is a group 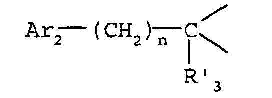 in which Ar₂ and n are as defined for (Ic) in claim 1 and R'₃ is either R₃ as defined for (Ic) in claim 1 or a precursor of R₃, it being understood that if R'₃ is a hydroxyl or an amino, these groups can be protected;
5) after optional deprotection of the hydroxyl group or the amino group represented by R'₃, or optional conversion of R'₃ to R₃, the resulting product is optionally converted to a salt thereof.

6. Method of preparing the compounds of formula (Ic) according to claim 1 and the salts thereof, **characterized in that**:
1') a compound of formula (VIII) as defined in claim 5 is oxidized to give a compound of the formula 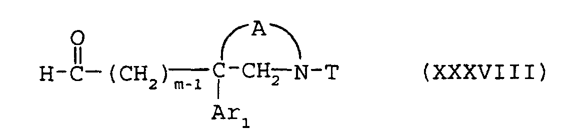 in which m, Ar₁, A and T are as defined in claim 5;
2') the compound of formula (XXXVIII) is reacted with a compound of formula (XI) as defined in claim 5, in the presence of an acid, and the iminium salt formed as an intermediate is then reduced by means of a reducing agent; and
3') after optional deprotection of the hydroxyl groups or amino groups, or optional conversion of R'₃ to R₃, the resulting product is optionally converted to a salt thereof.

7. Compound of the formula: 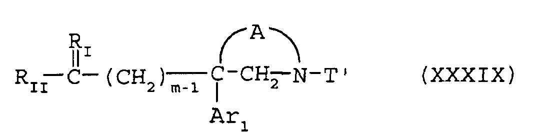 in which:
- A is Ac and Ar is Ar₁ₐ, Ac and Ar₁ₐ being as defined for (Ic) in claim 1 and m is 2;
- R_{I} is two hydrogen atoms and R_{II} is:
- either a group -O-E, in which E is a hydrogen atom or an O-protecting group selected from tetrahydropyran-2-yl; benzoyl and (C₁-C₄) alkylcarbonyl;
- or a group -O-SO₂-Y, in which Y is a methyl, phenyl, tolyl or trifluoromethyl group;
- or alternatively R_{I} is an oxygen atom and R_{II} is a hydrogen atom; and
- T' is -CH₂-Za, Za being as defined for a compound of formula (Ic); T' can also be hydrogen if R_{I} is 2 hydrogen atoms and R_{II} is simultaneously a group -O-E,
in enantiomerically pure form or in racemic form.

8. Compound according to claim 7, of the formula: 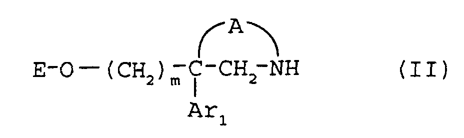 in which A is Ac and Ar₁ is Ar₁ₐ, Ac and Ar₁ₐ being as defined for (Ic) in claim 1, m is 2 and E is hydrogen or an O-protecting group selected from tetrahydropyran-2-yl; benzoyl and (C₁-C₄) alkylcarbonyl, in enantiomerically pure form or in racemic form.

9. Compound according to claim 7, of the formula: 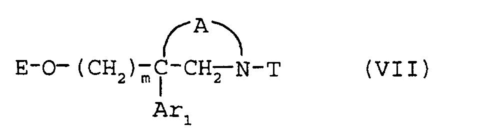 in which A is Ac and Ar₁ is Ar₁ₐ, Ac and Ar₁ₐ being as defined for (Ic) in claim 1, m is 2, T is -CH₂-Za, Za being as defined in claim 1, and E is hydrogen or an O-protecting group selected from tetrahydropyral-2-yl; benzoyl and (C₁-C₄)alkylcarbonyl, in enantiomerically pure form or in racemic form.

10. Compound according to claim 7, of the formula: 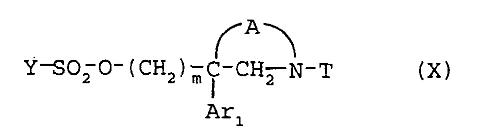 in which A is Ac and Ar₁ is Ar₁ₐ, Ac and Ar₁ₐ being as defined for (Ic) in claim 1, m is 2; T is -CH₂-Za, Za being as defined in claim 1 and Y is a methyl, phenyl, tolyl or trifluoromethyl group, in enantiomerically pure form or in racemic form.

11. Compound according to claim 7, of the formula: 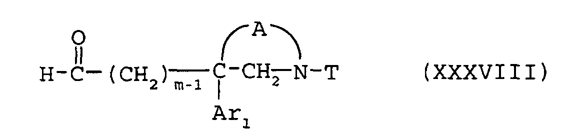 in which A is Ac and Ar₁ is Ar₁ₐ, Ac and Aria being as defined for (Ic) in claim 1, m is 2; T is -CH₂-Za, Za being as defined in claim 1, in enantiomerically pure form or in racemic form.

12. Compound of the formula: 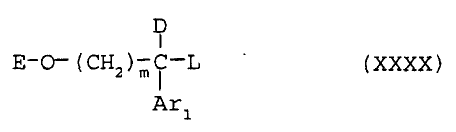 in which:
- m is 2 and Ar₁ is Ar₁ₐ as defined for a compound of formula (Ic) in claim 1;
- E is a hydrogen or an O-protecting group selected from tetrahydropyral-2-yl; benzoyl and (C₁₋C₄)alkylcarbonyl;
- L is a cyano or an aminomethyl group;
- D is the group -CH₂-OH;
in enantiomerically pure form or in racemic form if L is an aminomethyl group.

13. Pharmaceutical composition containing as the active principle a compound according to any one of claims 1 to 4, or a pharmaceutically salt thereof.

14. Pharmaceutical composition according to claim 13 in the form of a dosage unit in which the active principle is mixed with at least one pharmaceutical excipient.

15. Pharmaceutical composition according to claim 14, containing 0.5 to 1000 mg of the active principle.

16. Pharmaceutical composition according to claim 15, containing 2.5 to 250 mg of the active principle.
